# EUROPÄISCHE PATENTSCHRIFT

(11) **EP 2 132 206 B1**
(45) Veröffentlichungstag und Bekanntmachung des Hinweises auf die Patenterteilung: **01.04.2015**
(21) Anmeldenummer: 08715849.9
(22) Anmeldetag: 19.02.2008
(51) Int. Cl.: C07D 471/04, A61K 31/437, A61P 9/10

(54) **SUBSTITUIERTE 4-ARYL-1,4-DIHYDRO-1,6-NAPHTHYRIDINAMIDE UND IHRE VERWENDUNG**
SUBSTITUTED 4-ARYL-1,4-DIHYDRO-1,6-NAPHTHYRIDINAMIDES AND USE THEREOF
AMIDES DE 4-ARYL-1,4-DIHYDRO-1,6-NAPHTHYRIDINE SUBSTITUÉS ET UTILISATION DE CEUX-CI

(30) Priorität: 27.02.2007 DE 102007009494
(43) Veröffentlichungstag der Anmeldung: 16.12.2009
(73) Patentinhaber: Bayer Intellectual Property GmbH, 40789 Monheim (DE)
(72) Erfinder: BÄRFACKER, Lars, 46047 Oberhausen (DE); KOLKHOF, Peter, 42113 Wuppertal (DE); SCHLEMMER, Karl-Heinz, 42113 Wuppertal (DE); GROSSER, Rolf, 51373 Leverkusen (DE); NITSCHE, Adam, 50259 Pulheim (DE); KLEIN, Martina, 42579 Heiligenhaus (DE); MÜNTER, Klaus, 42489 Wülfrath (DE); ALBRECHT-KÜPPER, Barbara, 42289 Wülfrath (DE); HARTMANN, Elke, 42111 Wuppertal (DE)
(74) Vertreter: BIP Patents
(86) Internationale Anmeldenummer: PCT/EP2008/001257
(87) Internationale Veröffentlichungsnummer: WO 2008/104306

(56) Entgegenhaltungen:
- EP-A- 0 234 516
- WO-A-2005/087740
- WO-A-2007/009670
- WO-A-2007/140894
- WO-A-2007/140934

## Beschreibung

Die vorliegende Anmeldung betrifft neue, substituierte 4-Aryl-1,4-dihydro-1,6-naphthyridin-3-carboxamide, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von kardiovaskulären Erkrankungen.

Aldosteron spielt eine Schlüsselrolle in der Aufrechterhaltung der Flüssigkeits- und Elektrolythomöostase, indem es im Epithel des distalen Nephrons die Natriumretention und Kaliumsekretion fördert, was zur Konstanthaltung des Extrazellulärvolumens und damit zur Blutdruckregulation beiträgt. Daneben entfaltet Aldosteron direkte Effekte auf die Struktur und Funktion des Herz- und Gefäßsystems, wobei die dafür zugrunde liegenden Mechanismen noch nicht erschöpfend geklärt sind [R.E. Booth, J.P. Johnson, J.D. Stockand, Adv. Physiol. Educ. 26 (1), 8-20 (2002)].

Aldosteron ist ein Steroidhormon, das in der Nebennierenrinde gebildet wird. Seine Produktion wird indirekt ganz wesentlich in Abhängigkeit von der Nierendurchblutung reguliert. Jede Abnahme der Nierendurchblutung führt in der Niere zu einer Ausschüttung des Enzyms Renin in den Blutkreislauf. Dieses wiederum aktiviert die Bildung von Angiotensin II, das einerseits verengend auf die arteriellen Blutgefäße wirkt, andererseits aber auch die Bildung von Aldosteron in der Nebennierenrinde stimuliert. Somit fungiert die Niere als Blutdruck- und damit indirekter Volumen-Sensor im Blutkreislauf und wirkt über das Renin-Angiotensin-Aldosteron-System kritischen Volumenverlusten entgegen, indem einerseits der Blutdruck gesteigert wird (Angiotensin II-Wirkung), andererseits durch verstärkte Rückresorption von Natrium und Wasser in der Niere der Füllungszustand des Gefäßsystems wieder ausgeglichen wird (Aldosteron-Wirkung).

Dieses Regelsystem kann in vielfältiger Weise krankhaft gestört sein. So führt eine chronische Minderdurchblutung der Nieren (z.B. infolge einer Herzinsuffizienz und des hierdurch verursachten Blutrückstaus im Venensystem) zu einer chronisch überhöhten Ausschüttung von Aldosteron. Diese wiederum zieht eine Expansion des Blutvolumens nach sich und verstärkt hiermit die Herzschwäche durch ein Volumenüberangebot an das Herz. Eine Stauung von Blut in den Lungen mit Atemnot und Ödembildung in den Extremitäten sowie Aszites und Pleuraergüsse können die Folge sein; die Nierendurchblutung sinkt weiter ab. Überdies führt die übersteigerte Aldosteron-Wirkung zu einer Minderung der Kalium-Konzentration im Blut und in der Extrazellulärflüssigkeit. In ohnehin vorgeschädigten Herzmuskeln kann die Unterschreitung eines kritischen Mindestwertes tödlich endende Herzrhythmusstörungen auslösen. Hierin dürfte eine der Hauptursachen des häufig bei Patienten mit Herzinsuffizienz eintretenden *plötzlichen Herztodes* zu suchen sein.

Zusätzlich wird Aldosteron auch für eine Reihe der typischerweise bei Herzinsuffizienten zu beobachtenden Umbauprozesse des Herzmuskels verantwortlich gemacht. Somit ist der *Hyperaldosteronismus* eine entscheidende Komponente in der Pathogenese und Prognose der Herzinsuffizienz, die ursprünglich durch unterschiedliche Schädigungen, wie z.B. einen Herzinfarkt, eine Herzmuskelentzündung oder Bluthochdruck, ausgelöst werden kann. Diese Annahme wird durch die Tatsache erhärtet, dass in umfangreichen klinischen Studien in Patientenkollektiven mit chronischer Herzinsuffizienz bzw. nach akutem Myokardinfarkt durch Anwendung von Aldosteron-Antagonisten die Gesamtmortalität deutlich gesenkt wurde [B. Pitt, F. Zannad, W.J. Remme et al., N. Engl. J. Med. 341, 709-717 (1999); B. Pitt, W. Remme, F. Zannad et al., N. Engl. J. Med. 348, 1309-1321 (2003)]. Dies konnte unter anderem durch eine Senkung der Inzidenz des plötzlichen Herztodes erreicht werden.

Neueren Studien zufolge findet man auch bei einem nicht unerheblichen Teil der Patienten, die unter einer essentiellen Hypertonie leiden, eine sogenannte normokaliämische Variante des primären Hyperaldosteronismus [Prävalenz bis 11% aller Hypertoniker: L. Seiler und M. Reincke, Der Aldosteron-Renin-Quotient bei sekundärer Hypertonie, Herz 28, 686-691 (2003)]. Als beste Diagnostikmethode dient beim normokaliämischen Hyperaldosteronismus der Aldosteron/Renin-Quotient der entsprechenden Plasmakonzentrationen, so dass auch relative Aldosteron-Erhöhungen in Bezug auf die Renin-Plasmakonzentrationen diagnostizierbar und letztlich therapierbar werden. Deshalb ist ein im Zusammenhang mit einer essentiellen Hypertonie diagnostizierter Hyperaldosteronismus ein Ansatzpunkt für eine kausale und prophylaktisch sinnvolle Therapie.

Weitaus seltener als die oben aufgeführten Formen des Hyperaldosteronismus sind solche Krankheitsbilder, bei denen die Störung entweder in den Hormon-produzierenden Zellen der Nebenniere selbst zu finden ist oder deren Anzahl oder Masse durch eine Hyperplasie oder Wucherung vermehrt ist. Adenome oder diffuse Hyperplasien der Nebennierenrinde sind die häufigste Ursache des auch als *Conn-Syndrom* bezeichneten primären Hyperaldosteronismus, dessen Leitsymptome Hypertonie und hypokaliämische Alkalose sind. Auch hier steht neben der chirurgischen Entfernung des krankhaften Gewebes die medikamentöse Therapie mit Aldosteron-Antagonisten im Vordergrund [H.A. Kühn und J. Schirmeister (Hrsg.), Innere Medizin, 4. Aufl., Springer Verlag, Berlin, 1982].

Ein anderes typischerweise mit Erhöhung der Aldosteron-Konzentration im Plasma einhergehendes Krankheitsbild ist die fortgeschrittene Leberzirrhose. Die Ursache der Aldosteronerhöhung liegt hier vorwiegend im infolge der Leberfunktionsstörung eingeschränkten Abbau des Aldosterons. Volumenüberlastung, Ödeme und Hypokaliämie sind die typischen Folgen, die in der klinischen Praxis durch Aldosteron-Antagonisten erfolgreich gelindert werden können.

Die Wirkungen von Aldosteron werden über den in den Zielzellen intrazellulär lokalisierten Mineralokorticoid-Rezeptor vermittelt. Die bislang verfügbaren Aldosteron-Antagonisten besitzen wie Aldosteron selbst eine Steroid-Grundstruktur. Begrenzt wird die Anwendbarkeit derartiger steroidaler Antagonisten durch ihre Wechselwirkungen mit den Rezeptoren anderer Steroidhormone, die zum Teil zu erheblichen Nebenwirkungen wie Gynäkomastie und Impotenz und zum Abbrechen der Therapie führen [M.A. Zaman, S. Oparil, D.A. Calhoun, Nature Rev. Drug Disc. 1, 621-636 (2002)].

Die Anwendung wirkstarker, nicht-steroidaler und für den Mineralokorticoid-Rezeptor selektiver Antagonisten bietet die Möglichkeit, dieses Nebenwirkungsprofil zu umgehen und dadurch einen deutlichen Therapievorteil zu erzielen.

Aufgabe der vorliegenden Erfindung ist die Bereitstellung neuer Verbindungen, die als selektive Mineralokorticoid-Rezeptor-Antagonisten für die Behandlung von Erkrankungen, insbesondere von kardiovaskulären Erkrankungen, eingesetzt werden können.

In EP 0 133 530-A, EP 0 173 933-A, EP 0 189 898-A und EP 0 234 516-A werden 4-Aryl-substituierte 1,4-Dihydro-1,6-naphthyridine bzw. -naphthyridinone mit Calcium-antagonistischer Wirkung zur Behandlung von Gefäßerkrankungen offenbart. Über das pharmakologische Profil dieser Verbindungen wird unter anderem in G. Werner et al., Naunyn-Schmiedeberg's Arch. Pharmacol. 344 (3), 337-344 (1991) berichtet. Weiterhin werden 1,4-Dihydro-1,6-naphthyridin-Derivate in WO 02/10164 als Kaliumkanal-Öffner zur Behandlung unterschiedlicher, vor allem urologischer Erkrankungen beansprucht. 4-Fluorenonyl- bzw. 4-Chromenonyl-1,4-dihydropyridin-Derivate als Mineralokorticoid-Rezeptor-Antagonisten werden in WO 2005/087740 und WO 2007/009670 beschrieben. In WO 2006/066011 werden 4-Aryl-3-cyano-1,4-dihydropyridin-5-carbonsäureester und -amide als zum Teil duale Modulatoren von Steroidhormon-Rezeptoren und des L-Typ-Calciumkanals offenbart, und in WO 2005/097118 werden Verbindungen mit 4-Aryl-1,4-dihydropyridin-Kernstruktur als Aldosteron-Rezeptor-Antagonisten beansprucht.

Offenbart sind Verbindungen der allgemeinen Formel (I) 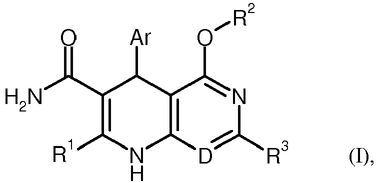 in welcher
- D: für N oder C-R⁴ steht, worin
R⁴ Wasserstoff, Fluor, Trifluormethyl oder (C₁-C₄)-Alkyl bedeutet,
- Ar: für eine Gruppe der Formel 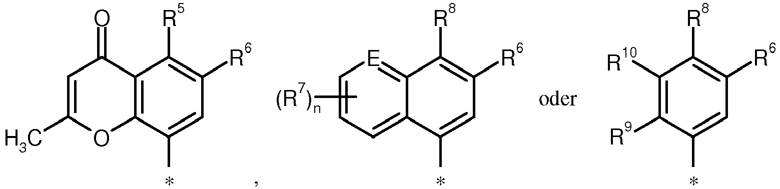 steht, worin
* die Verknüpfungsstelle bedeutet,
R⁵ Wasserstoff, Fluor, Chlor, Cyano, Nitro, Trifluormethyl oder (C₁-C₄)-Alkyl bedeutet,
R⁶ Wasserstoff oder Fluor bedeutet,
R⁷ Halogen, (C₁-C₄)-Alkyl, Trifluormethyl, (C₁-C₄)-Alkoxy oder Trifluormethoxy bedeutet,
R⁸ Cyano oder Nitro bedeutet,
R⁹ Wasserstoff, Halogen, (C₁-C₄)-Alkyl, (C₁-C₄)-Alkoxy, (C₁-C₄)-Alkylthio oder Di-(C₁-C₄)-alkylamino, wobei die Alkylgruppe in den genannten (C₁-C₄)-Alkyl-, (C₁-C₄)-Alkoxy- und (C₁-C₄)-Alkylthio-Resten jeweils bis zu dreifach mit Fluor substituiert sein kann,
oder
Phenyl, welches mit Halogen, (C₁-C₄)-Alkyl oder Trifluormethyl substituiert sein kann, bedeutet,
R¹⁰ Wasserstoff, Halogen oder (C₁-C₄)-Alkyl bedeutet,
E CH, C-R⁷ oder N bedeutet
und
n die Zahl 0, 1 oder 2 bedeutet,
wobei für den Fall, dass der Substituent R⁷ mehrfach auftritt, seine Bedeutungen gleich oder verschieden sein können,
- R¹: für (C₁-C₄)-Alkyl, welches bis zu dreifach mit Fluor substituiert sein kann, steht,
- R²: für (C₁-C₆)-Alkyl, welches mit (C₃-C₇)-Cycloalkyl oder bis zu dreifach mit Fluor substituiert sein kann, oder für eine Gruppe der Formel -SO2-R¹¹ steht, worin
R¹¹ (C₁-C₆)-Alkyl, Trifluormethyl, (C₃-C₇)-Cycloalkyl, Phenyl oder 5- oder 6-gliedriges Heteroaryl mit bis zu zwei Heteroatomen aus der Reihe N, O und/oder S bedeutet,
wobei Phenyl und Heteroaryl ihrerseits jeweils ein- oder zweifach, gleich oder verschieden, mit Halogen, Cyano, Nitro, (C₁-C₄)-Alkyl, Trifluormethyl, (C₁-C₄)-Alkoxy und/oder Trifluormethoxy substituiert sein können, und
- R³: für Wasserstoff, Fluor, Trifluormethyl oder (C₁-C₄)-Alkyl steht,
sowie ihre Salze, Solvate und Solvate der Salze.

Alle nachfolgenden Gegenstände und Aspekte, die nicht unter die Ansprüche fallen, sind lediglich offenbart aber nicht Teil der Erfindung, auch wenn sie als erfindungsgemäss bezeichnet sind.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die vorliegende Erfindung umfasst daher die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methyl-morpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl, iso-Pentyl und n-Hexyl.
(C₃-C₇)-cycloalkyl steht im Rahmen der Erfindung für eine monocyclische, gesättigte Cycloalkylgruppe mit 3 bis 7 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.
(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, n-Propoxy, Isopropoxy, n-Butoxy und tert.-Butoxy.
(C₁-C₄)-Alkylthio steht im Rahmen der Erfindung für einen geradkettigen oder verzweigten Alkylthiorest mit 1 bis 4 Kohlenstoffatomen. Beispielhaft und vorzugsweise seien genannt: Methylthio, Ethylthio, n-Propylthio, Isopropylthio, n-Butylthio und tert.-Butylthio.
Di-(C₁-C₄)-alkylamino steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N,N*-Dimethylamino, *N,N-*Diethylamino, *N-*Ethyl-*N*-methylamino, *N*-Methyl-*N*-n-propylamino, *N,N*-Diisopropylamino, *N-*Isopropyl-*N*-n-propylamino, *N*-n-Butyl*-N*-methylamino und *N*-tert.-Butyl-*N*-methylamino.
5- oder 6-gliedriges Heteroaryl steht im Rahmen der Erfindung für einen aromatischen Heterocyclus (Heteroaromaten) mit 5 oder 6 Ringatomen, der ein oder zwei Ring-Heteroatome aus der Reihe N, O und/oder S enthält und über ein Ring-Kohlenstoffatom verknüpft ist. Beispielhaft und vorzugsweise seien genannt: Furyl, Pyrrolyl, Thienyl, Pyrazolyl, Imidazolyl, Thiazolyl, Oxazolyl, Isoxazolyl, Isothiazolyl, Pyridyl, Pyrimidinyl, Pyridazinyl und Pyrazinyl.
Halogen schließt im Rahmen der Erfindung Fluor, Chlor, Brom und Iod ein. Bevorzugt sind Fluor oder Chlor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Offenbart sind Verbindungen der Formel (I), in welcher
- D: für C-R⁴ steht, worin
R⁴ Wasserstoff, Methyl oder Trifluormethyl bedeutet,
- Ar: für eine Gruppe der Formel 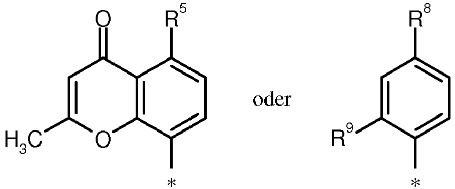 steht, worin
* die Verknüpfungsstelle bedeutet,
R⁵ Wasserstoff, Fluor, Chlor oder Cyano bedeutet,
R⁸ Cyano oder Nitro bedeutet
und
R⁹ Chlor, Brom, (C₁-C₄)-Alkyl, Trifluormethyl, (C₁-C₄)-Alkoxy, Trifluormethoxy, (C₁-C₄)-Alkylthio oder Trifluormethylthio bedeutet,
- R¹: für Methyl oder Trifluormethyl steht,
- R²: für (C₁-C₄)-Alkyl, Trifluormethyl oder eine Gruppe der Formel -SO₂-R¹¹ steht, worin
R¹¹ (C₁-C₄)-Alkyl oder Trifluormethyl bedeutet, und
- R³: für Wasserstoff, Methyl oder Trifluormethyl steht,
sowie ihre Salze, Solvate und Solvate der Salze.

Besonders bevorzugt im Rahmen der vorliegenden Erfindung sind Verbindungen der Formel (I), in welcher
- D: für C-R⁴ steht, worin
R⁴ Wasserstoff oder Methyl bedeutet,
- Ar: für eine Gruppe der Formel 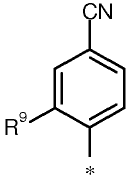 steht, worin
* die Verknüpfungsstelle
und
R⁹ Ethyl, Methoxy oder Trifluormethoxy bedeutet,
- R¹: für Methyl oder Trifluormethyl steht,
- R²: für Methyl, Ethyl, n-Propyl oder Isopropyl steht
und
- R³: für Wasserstoff oder Methyl steht,
sowie ihre Salze, Solvate und Solvate der Salze.

Ganz besonders bevorzugt sind Verbindungen gemäß Formel (I) mit den folgenden Strukturen: 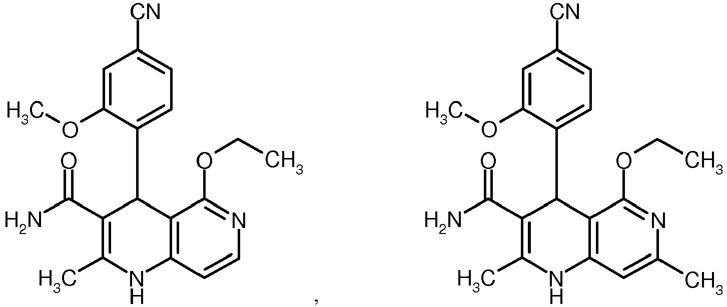 und 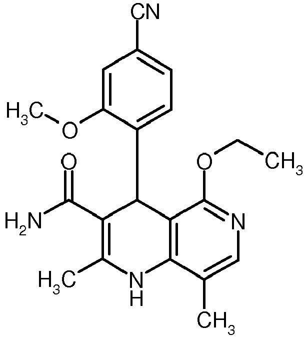 sowie ihre Salze, Solvate und Solvate der Salze.

Insbesondere bevorzugt sind hierbei die enantiomeren Verbindungen mit den folgenden Strukturen: 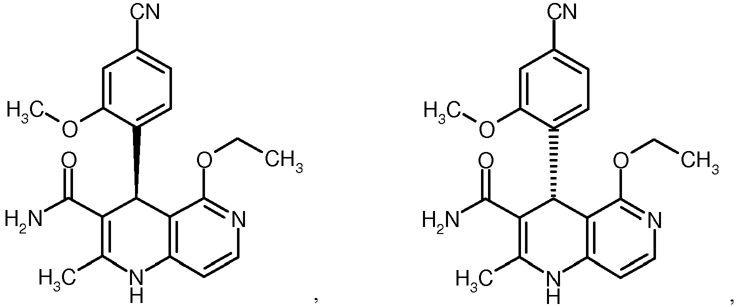 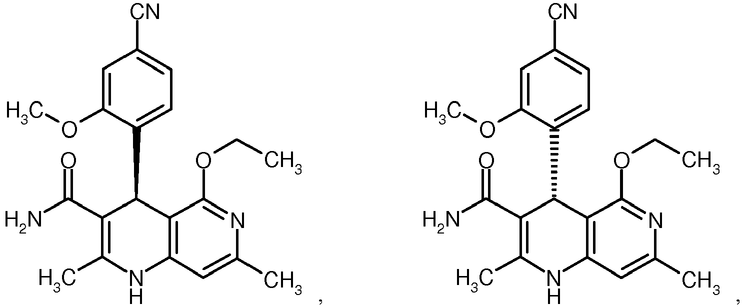 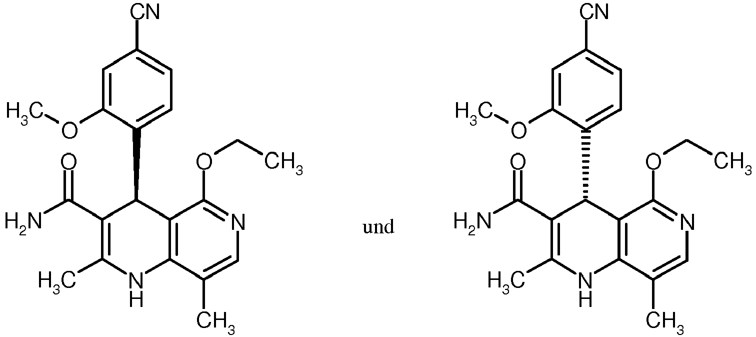 sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man eine Verbindung der Formel (II) 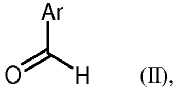 in welcher Ar die oben angegebene Bedeutung hat,
in einem inerten Lösungsmittel gegebenenfalls in Gegenwart einer Säure, einer Säure/Base-Kombination und/oder eines wasserentziehenden Mittels mit einer Verbindung der Formel (III) 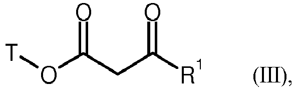 in welcher R¹ die oben angegebene Bedeutung hat und
- T: für Allyl oder 2-Cyanoethyl steht,
zu einer Verbindung der Formel (IV) 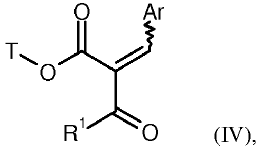 in welcher Ar, T und R¹ jeweils die oben angegebenen Bedeutungen haben,
umsetzt, diese dann in einem inerten Lösungsmittel mit einer Verbindung der Formel (V) 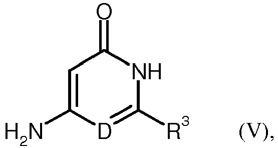 in welcher D und R³ die oben angegebenen Bedeutungen haben,
zu einer Verbindung der Formel (VI) 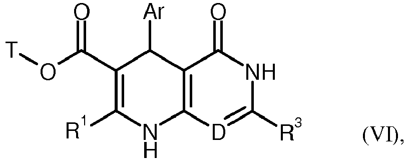 in welcher Ar, D, T, R¹ und R³ jeweils die oben angegebenen Bedeutungen haben,
kondensiert, anschließend die Verbindungen der Formel (VI) in einem inerten Lösungsmittel gegebenenfalls in Gegenwart einer Base mit einer Verbindung der Formel (VII) oder einem Trialkyloxonium-Salz der Formel (VIII) 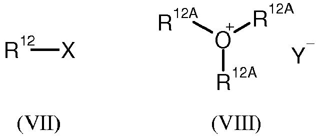 in welchen
- R¹²: für (C₁-C₆)-Alkyl, das mit (C₃-C₇)-Cycloalkyl oder bis zu dreifach mit Fluor substituiert sein kann, steht,
- R^{12A}: für Methyl oder Ethyl steht,
- X: für eine Abgangsgruppe, wie beispielsweise Halogen, Mesylat, Tosylat oder Triflat, steht
und
- Y⁻: für ein nicht-nukleophiles Anion, wie beispielsweise Tetrafluoroborat, steht,
oder in Gegenwart einer Säure mit einem Trialkylorthoformiat der Formel (IX) 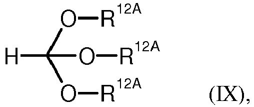 in welcher R^{12A} die oben angegebene Bedeutung hat,
zu Verbindungen der Formel (X-A) 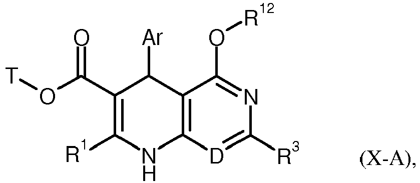 in welcher Ar, D, T, R¹, R³ und R¹² jeweils die oben angegebenen Bedeutungen haben,
alkyliert
oder die Verbindungen der Formel (VI) in einem inerten Lösungsmittel in Gegenwart einer Base mit einer Verbindung der Formel (XI) 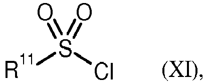 in welcher R¹¹ die oben angegebene Bedeutung hat,
zu Verbindungen der Formel (X-B) 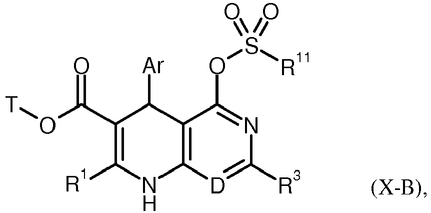 in welcher Ar, D, T, R¹, R³ und R¹¹ jeweils die oben angegebenen Bedeutungen haben,
umsetzt,
danach in den Verbindungen der Formel (X-A) bzw. (X-B) die Ester-Gruppe T nach an sich bekannten Methoden zu den Carbonsäuren der Formel (XII) 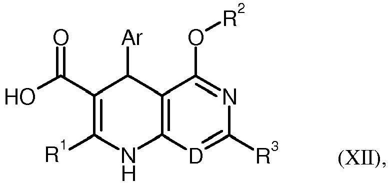 in welcher Ar, D, R¹, R² und R³ jeweils die oben angegebenen Bedeutungen haben,
abspaltet, anschließend mit 1,1'-Carbonyldiimidazol in die Imidazolide der Formel (XIII) 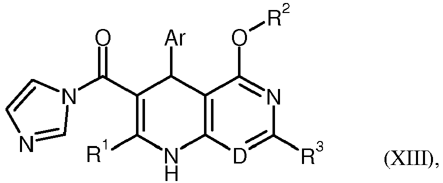 in welcher Ar, D, R¹, R² und R³ jeweils die oben angegebenen Bedeutungen haben,
überführt und diese dann in einem inerten Lösungsmittel gegebenenfalls in Gegenwart einer Hilfsbase mit Ammoniak zu den Amiden der Formel (I) umsetzt
und gegebenenfalls die Verbindungen der Formel (I) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

Die Verfahrenssequenz (II) + (III) → (IV) und (IV) + (V) → (VI) kann auch einstufig als 3-Komponenten-Reaktion (II) + (III) + (V) → (VI), ohne Isolierung der Zwischenstufe (IV), durchgeführt werden.

Die Verfahrensschritte (II) + (III) → (IV) und (IV) + (V) → (VI) bzw. (II) + (III) + (V) → (VI) werden im Allgemeinen in einem inerten Lösungsmittel in einem Temperaturbereich von +20°C bis zum Siedepunkt des Lösungsmittels bei Normaldruck durchgeführt.

Als inerte Lösungsmittel eignen sich hierfür beispielsweise Alkohole wie Methanol, Ethanol, n-Propanol, Isopropanol, n-Butanol oder *tert*.-Butanol, Halogenkohlenwasserstoffe wie Dichlormethan, Trichlormethan, Tetrachlormethan, Trichlorethan oder 1,2-Dichlorethan, oder andere Lösungsmittel wie Acetonitril, Tetrahydrofuran, Dioxan, 1,2-Dimethoxyethan, Hexan, Benzol, Toluol, Chlorbenzol, Pyridin oder Eisessig. Bevorzugt werden die Umsetzungen in Dichlormethan, Toluol, Ethanol oder Isopropanol bei der jeweiligen Rückfluss-Temperatur unter Normaldruck durchgeführt.

Die genannten Reaktionen können gegebenenfalls vorteilhaft in Gegenwart einer Säure, einer Säure/Base-Kombination und/oder eines wasserentziehenden Mittels, wie beispielsweise Molekularsieb, erfolgen. Als Säuren eignen sich beispielsweise Essigsäure, Trifluoressigsäure, Methansulfonsäure oder p-Toluolsulfonsäure; als Basen sind insbesondere Piperidin oder Pyridin geeignet [zur Synthese von 1,4-Dihydropyridinen vgl. auch D.M. Stout, A.I. Meyers, Chem. Rev. 1982, 82, 223-243; H. Meier et al., Liebigs Ann. Chem. 1977, 1888; H. Meier et al., ibid. 1977, 1895; H. Meier et al., ibid. 1976, 1762; F. Bossert et al., Angel. Chem. 1981, 93, 755].

Inerte Lösungsmittel für die Verfahrensschritte (VI) + (VII) → (X-A), (VI) + (VIII) → (X-A) und (VI) + (XI) → (X-B) sind beispielsweise Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethan, Tetrachlorethan, Trichlorethylen, Chlorbenzol oder Chlortoluol, oder andere Lösungsmittel wie *N,N*-Di-methylformanilid (DMF), Dimethylsulfoxid (DMSO), *N,N*'-Dimethylpropylenharnstoff (DMPU), *N*-Methylpyrrolidon (NMP), Pyridin oder Acetonitril. Ebenso ist es möglich, Gemischte der genannten Lösungsmittel zu verwenden. Bevorzugt werden im Verfahrensschritt (VI) + (VII) → (X-A) Tetrahydrofuran oder Dimethylformamid, im Verfahrensschritt (VI) + (VIII) → (X-A) Dichlormethan und im Verfahrensschritt (VI) + (XI) → (X-B) Pyridin eingesetzt.

Die Verfahrensvariante (VI) + (IX) → (X-A) wird vorzugsweise mit einem großen Überschuss an Orthoameisensäureester in Dimethylformamid oder ohne Zusatz eines weiteren Lösungsmittels durchgeführt; als Reaktionskatalysator sind beispielsweise starke anorganische Säuren wie Schwefelsäure von Vorteil [vgl. z.B. I.I. Barabanov et al., Russ. Chem. Bl. 47 (11), 2256-2261 (1998)].

Als Basen für den Verfahrensschritt (VI) + (VII) → (X-A) eignen sich insbesondere Alkali- oder Erdalkalicarbonate wie Lithium-, Natrium-, Kalium-, Calcium- oder Cäsiumcarbonat, Alkalihydride wie Natrium- oder Kaliumhydrid, Amide wie Lithium-, Natrium- oder Kalium-bis-(trimethylsilyl)amid oder Lithiumdiisopropylamid, metallorganische Verbindungen wie Butyllithium oder Phenyllithium, oder auch Phosphazen-Basen wie beispielsweise P2-t-Bu oder P4-t-Bu [so genannte "Schwesinger-Basen", vgl. R. Schwesinger, H. Schlemper, Angew. Chem. Int. Ed. Engl. 26, 1167 (1987); T. Pietzonka, D. Seebach, Chem. Ber. 124, 1837 (1991)]. Bevorzugt wird Natriumhydrid oder die Phosphazen-Base P4-t-Bu verwendet.

Als Basen für den Verfahrensschritt (VI) + (XI) → (X-B) eignen sich insbesondere Alkali- oder Erdalkalicarbonate wie Lithium-, Natrium-, Kalium-, Calcium- oder Cäsiumcarbonat, Alkalihydride wie Natrium- oder Kaliumhydrid, metallorganische Verbindungen wie Butyllithium oder Phenyllithium, oder organische Amine wie Trimethylamin, *N*-Methylmorpholin, *N*-Methylpiperidin, *N,N*-Diisopropylethylamin, Pyridin, 1,5-Diazabicyclo[4.3.0]non-5-en (DBN), 1,8-Diazabicyclo-[5.4.0]undec-7-en (DBU) oder 1,4-Diazabicyclo[2.2.2]octan (DABCO^{®}). Bevorzugt wird Pyridin verwendet, das gleichzeitig auch als Lösungsmittel dient.

Der Verfahrensschritt (VI) + (VIII) → (X-A) wird im Allgemeinen ohne Zusatz einer Base durchgeführt.

Die Umsetzungen (VI) + (VII) → (X-A), (VI) + (VIII) → (X-A) und (VI) + (XI) → (X-B) erfolgen im Allgemeinen in einem Temperaturbereich von -20°C bis +100°C, bevorzugt bei 0°C bis +60°C; die Verfahrensvariante (VI) + (IX) → (X-A) wird in der Regel in einem Temperaturbereich von +100°C bis +150°C durchgeführt. Die Reaktionen können bei normalem, bei erhöhtem oder bei vermindertem Druck erfolgen (z.B. von 0.5 bis 5 bar); im Allgemeinen arbeitet man bei Normaldruck.

Die Abspaltung des Allyl- bzw. 2-Cyanoethyl-Esters im Verfahrensschritt (X-A) bzw. (X-B) → (XII) geschieht nach bekannten, literaturüblichen Methoden. Im Falle des 2-Cyanoethylesters wird hierfür bevorzugt die wässrige Lösung eines Alkalihydroxids, wie beispielsweise Natron- oder Kalilauge, eingesetzt. Die Reaktion wird im Allgemeinen unter Verwendung eines wassermischbaren, inerten Ko-Solvens, wie beispielsweise Tetrahydrofuran, Dioxan oder 1,2-Dimethoxyethan, in einem Temperaturbereich von 0°C bis +40°C durchgeführt. Im Falle des Allylesters erfolgt die Abspaltung bevorzugt mit Hilfe des Wilkinson-Katalysators [Tris(triphenylphosphin)rhodium(I)-chlorid] in einem Wasser/Alkohol/Essigsäure-Gemisch bei Temperaturen von +50°C bis +100°C [vgl. z.B. Moseley, J.D., Tetrahedron Lett. 46, 3179-3181 (2005)].

Als inerte Lösungsmittel für den Verfahrensschritt (XII) → (XIII) eignen sich beispielsweise Ether wie Diethylether, Methyl-*tert*.-butylether, Dioxan, Tetrahydrofuran, Glykoldimethylether oder Diethylenglykoldimethylether, Halogenkohlenwasserstoffe wie Dichlormethan, Trichlormethan, 1,2-Dichlorethan, Trichlorethan, Tetrachlorethan, Chlorbenzol oder Chlortoluol, oder andere Lösungsmittel wie *N,N*-Dimthylformamid (DMF), Dimethylsulfoxid (DMSO), *N,N*'-Dimethylpropylenharnstoff (DMPU), *N*-Methylpyrrolidon (NMP), Aceton, Acetonitril oder Ethylacetat. Ebenso ist es möglich, Gemische der genannten Lösungsmittel zu verwenden. Bevorzugt wird Tetrahydrofuran, Dimethylformamid oder Ethylacetat eingesetzt. In der Regel wird die Reaktion in einem Temperaturbereich von 0° bis +40°C durchgeführt.

Für den Verfahrensschritt (XIII) → (I) sind als inerte Lösungsmittel beispielsweise Alkohole wie Methanol, Ethanol, n-Propanol, Isopropanol, n-Butanol oder *tert*.-Butanol, Ether wie Diethylether, Methyl-*tert*.-butylether, Dioxan, Tetrahydrofuran, Glykoldimethylether oder Diethylenglykoldimethylether, oder andere Lösungsmittel wie *N,N*-Diniethylformamid (DMF), Dimethylsulfoxid (DMSO), *N,N*'-Dimethylpropylenharnstoff (DMPU), *N*-Methylpyrrolidon (NMP), Acetonitril oder auch Wasser geeignet. Ebenso ist es möglich, Gemische dieser Lösungsmittel zu verwenden. Bevorzugt wird Tetrahydrofuran oder Dimethylformamid eingesetzt.

Als Anmioniak-Quelle für diese Umsetzung eignen sich Lösungen von gasförmigem Anmioniak in einem der oben genannten Lösungsmittel, insbesondere in Wasser. Die Reaktion kann gegebenenfalls vorteilhaft in Gegenwart eines tertiären Amins als Hilfsbase, wie beispielsweise Trimethylamin, *N*-Methylmorpholin, *N*-Methylpiperidin, *N,N*-Diisopropylethylamin oder 4-*N,N*-Dimethylaminopyridin, durchgeführt werden. Die Umsetzung erfolgt im Allgemeinen in einem Temperaturbereich von +20°C bis +120°C, bevorzugt bei +50°C bis +100°C.

Die Verbindungen der Formel (II) sind kommerziell erhältlich, literaturbekannt oder können in Analogie zu literaturbekannten Verfahren hergestellt werden (vgl. nachfolgende Reaktionsschemata 1-7). Die Verbindungen der Formeln (III), (VII), (VIII), (IX) und (XI) sind vielfach kommerziell erhältlich, literaturbekannt oder nach literaturbekannten Methoden herstellbar.

Die Verbindungen der Formel (V) sind literaturbeschrieben oder können in Analogie zu literaturbekannten Verfahren erhalten werden [vgl. z.B. T. Searls, L.W. McLaughlin, Tetrahedron 55, 11985-11996 (1999); D. McNamara, P.D. Cook, J. Med. Chem. 30, 340-347 (1987); S. Nesnow, C. Heidelberger, J. Heterocycl. Chem. 12, 941-944 (1975); N.C. Hung, E. Bisagni, Syyithesis 1984, 765-766; Z. Földi et al., Chem. Ber. 75 (7), 755-763 (1942); G.W. Kenner et al., J. Chem. Soc., 388 (1943)].

Gegebenenfalls kann eine Trennung der Enantiomere und/oder Diastereomere bereits auf der Stufe der Intermediate (VI), (X-A), (X-B) oder (XII) erfolgen, welche dann separat den nachfolgenden Umsetzungen unterworfen werden.

Die Herstellung der erfindungsgemäßen Verbindungen kann durch die folgenden Syntheseschemata veranschaulicht werden:

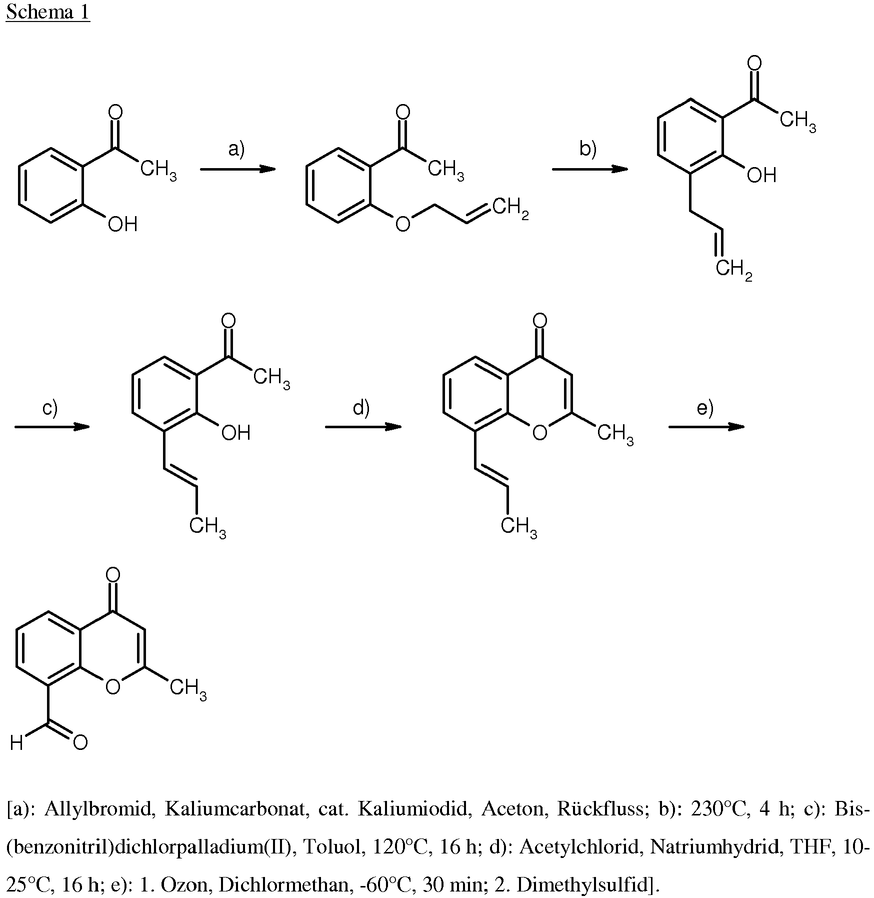

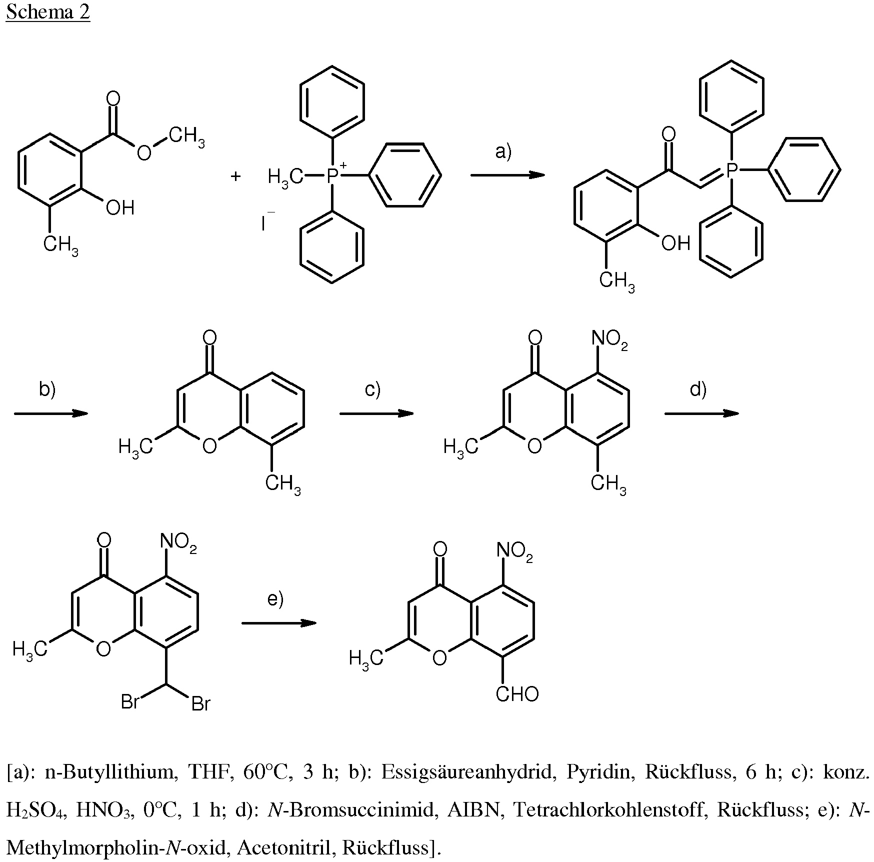

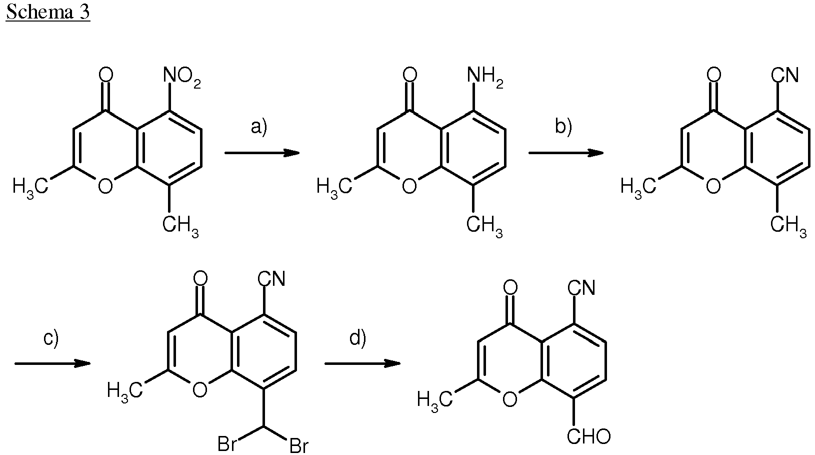

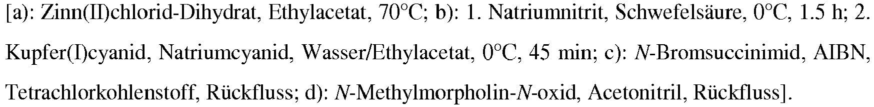

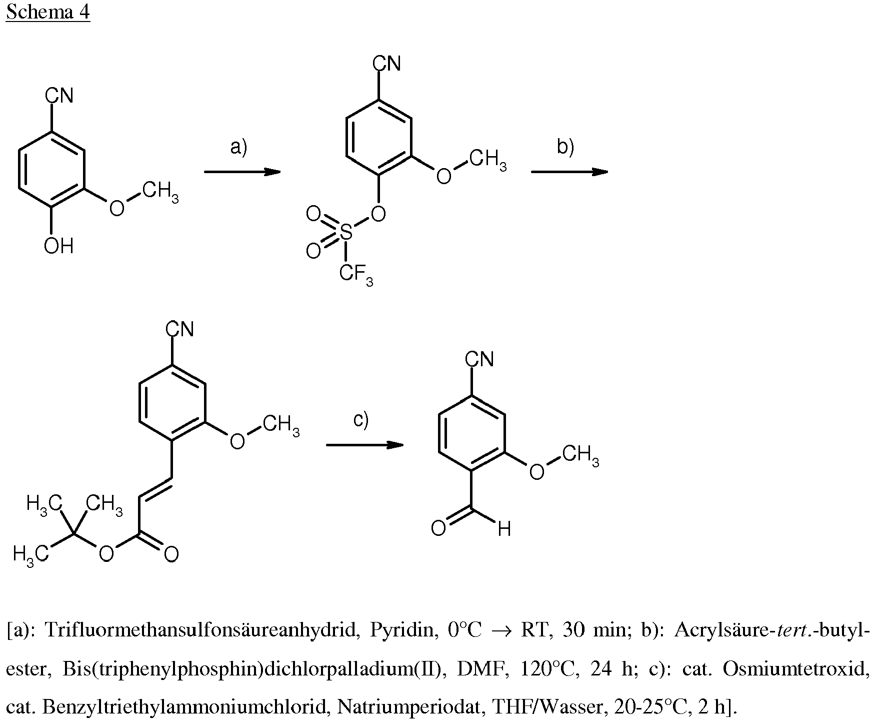

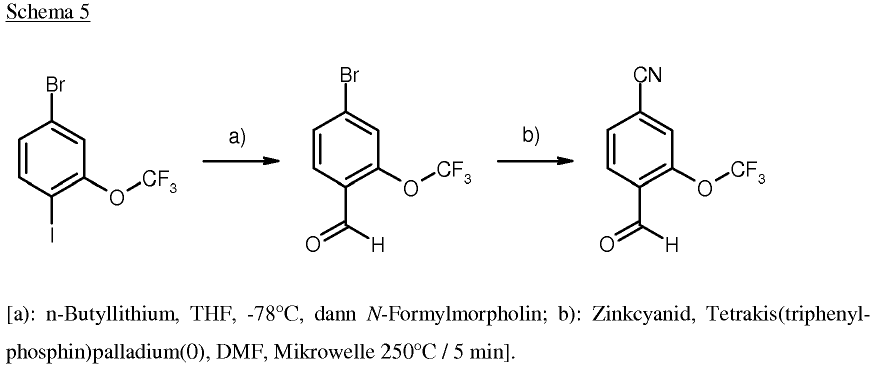

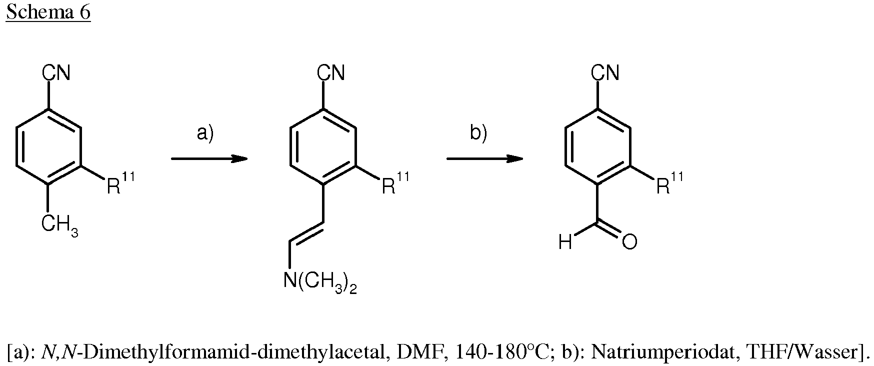

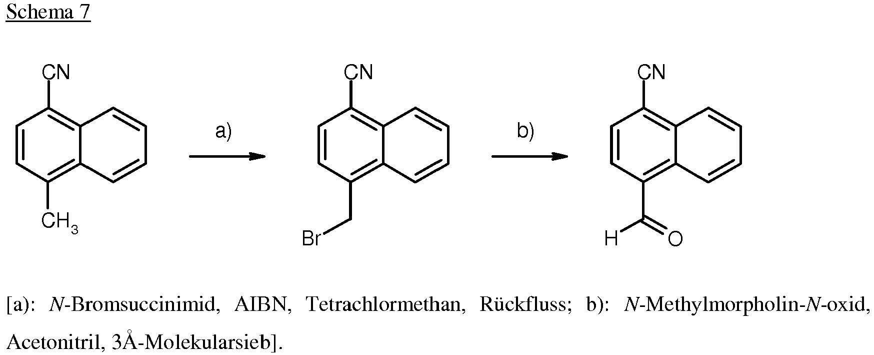

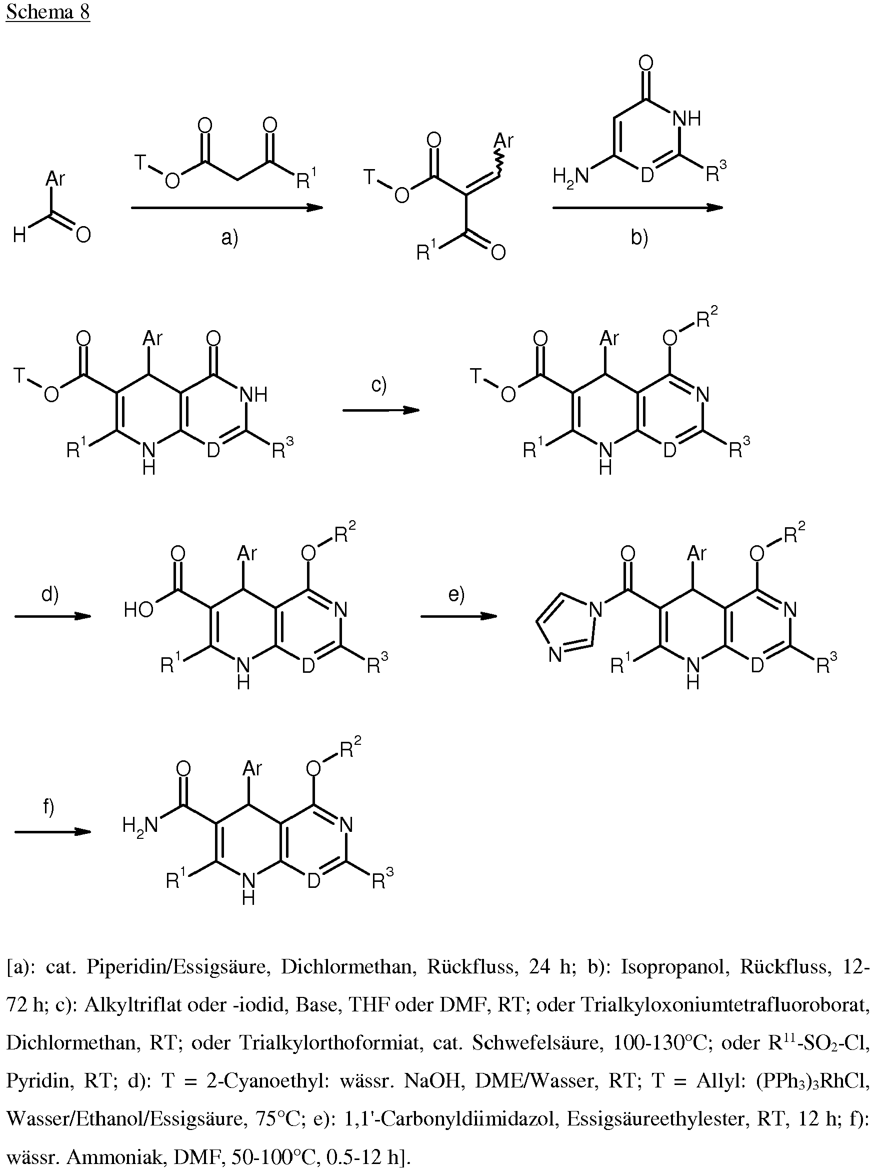

Die erfindungsgemäßen Verbindungen wirken als Antagonisten des Mineralokorticoid-Rezeptors und zeigen ein nicht vorhersehbares, wertvolles pharmakologisches Wirkspektrum. Sie eignen sich daher zur Verwendung als Arzneimittel zur Behandlung und/oder Prophylaxe von Krankheiten bei Menschen und Tieren.

Die erfindungsgemäßen Verbindungen sind geeignet für die Prophylaxe und/oder Behandlung von verschiedenen Erkrankungen und krankheitsbedingten Zuständen, insbesondere von Erkrankungen, die entweder durch eine Erhöhung der Aldosteron-Konzentration im Plasma oder durch eine Veränderung der Aldosteron-Plasmakonzentration relativ zur Renin-Plasmakonzentration gekennzeichnet sind oder mit diesen Veränderungen einhergehen. Beispielsweise seien genannt: idiopathischer primärer Hyperaldosteronismus, Hyperaldosteronismus bei Nebennierenhyperplasie, Nebennierenadenomen und/oder Nebennierencarzinomen, Hyperaldosteronismus bei Leberzirrhose, Hyperaldosteronismus bei Herzinsuffizienz sowie (relativer) Hyperaldosteronismus bei essentieller Hypertonie.

Die erfindungsgemäßen Verbindungen sind aufgrund ihres Wirkmechanismus ferner geeignet für die Prophylaxe des plötzlichen Herztodes bei Patienten, die unter einem erhöhten Risiko stehen, an einem plötzlichen Herztod zu versterben. Dies sind insbesondere Patienten, die z.B. an einer der folgenden Erkrankungen leiden: primäre und sekundäre Hypertonie, hypertensive Herzkrankheit mit oder ohne kongestive Herzinsuffizienz, therapieresistente Hypertonie, akute und chronische Herzinsuffizienz, koronare Herzerkrankung, stabile und instabile Angina pectoris, myokardiale Ischämie, Myokardinfarkt, dilatative Kardiomyopathien, angeborene primäre Kardiomyopathien wie z.B. Brugada-Syndrom, durch die Chagas-Erkrankung hervorgerufene Kardiomyopathien, Schock, Arteriosklerose, atriale und ventrikuläre Arrhythmie, transitorische und ischämische Attacken, Hirnschlag, entzündliche kardiovaskuläre Erkrankungen, periphere und kardiale Gefäßerkrankungen, periphere Durchblutungsstörungen, arterielle Verschlusskrankheiten wie Claudicatio intermittens, asymptomatische linksventrikuläre Dysfunktion, Myokarditis, hypertrophe Veränderungen des Herzens, pulmonale Hypertonie, Spasmen der Koronararterien und peripherer Arterien, Thrombosen, thromboembolische Erkrankungen sowie Vaskulitis.

Die erfindungsgemäßen Verbindungen können ferner verwendet werden für die Prophylaxe und/ oder Behandlung von Ödembildung, wie zum Beispiel pulmonales Ödem, renales Ödem oder Herzinsuffizienz-bedingtes Ödem, und von Restenosen, wie nach Thrombolysetherapien, percutan-transluminalen Angioplastien (PTA) und Koronarangioplastien (PTCA), Herztransplantationen sowie Bypass-Operationen.

Weiterhin eignen sich die erfindungsgemäßen Verbindungen zur Verwendung als kaliumsparendes Diuretikum und bei Elektrolytstörungen wie zum Beispiel Hyperkalzämie, Hypernatriämie oder Hypokaliämie.

Die erfindungsgemäßen Verbindungen eignen sich ebenso zur Behandlung von Nierenerkrankungen, wie akutem und chronischem Nierenversagen, hypertensiver Nierenkrankheit, arteriosklerotischer Nephritis (chronisch und interstitiell), Nephrosklerose, chronischer Niereninsuffizienz und zystischen Nierenerkrankungen, zur Verhinderung von Nierenschäden, die zum Beispiel durch Immunsuppressiva wie Cyclosporin A bei Organtransplantationen hervorgerufen werden können, sowie bei Nierenkrebs.

Außerdem können die erfindungsgemäßen Verbindungen eingesetzt werden für die Prophylaxe und/oder Behandlung von Diabetes mellitus und diabetischen Folgeerkrankungen wie z.B. Neuropathie und Nephropathie.

Die erfindungsgemäßen Verbindungen können ferner verwendet werden für die Prophylaxe und/ oder Behandlung von Mikroalbuminurie, zum Beispiel bedingt durch Diabetes mellitus oder Bluthochdruck, sowie der Proteinurie.

Die erfindungsgemäßen Verbindungen eignen sich auch für die Prophylaxe und/oder Behandlung von Erkrankungen, die entweder mit einer Erhöhung der Glukokortikoid-Konzentration im Plasma oder mit einer lokalen Konzentrationserhöhung von Glukokortikoiden im Gewebe (z.B. des Herzens) einhergehen. Beispielsweise seien genannt: Funktionsstörungen der Nebenniere, die zur Überproduktion von Glukokortikoiden führen (Cushing-Syndrom), Nebennierenrindentumore mit resultierender Überproduktion von Glukokortikoiden sowie Hypophysentumore, die autonom ACTH (adrenokortikotropes Hormon) produzieren und dadurch zu Nebennierenhyperplasien mit resultierendem Morbus Cushing führen.

Außerdem können die erfindungsgemäßen Verbindungen für die Prophylaxe und/oder Behandlung von Obesitas, des metabolischen Syndroms und der obstruktiven Schlaf-Apnoe eingesetzt werden.

Die erfindungsgemäßen Verbindungen können ferner verwendet werden für die Prophylaxe und/ oder Behandlung von entzündlichen Erkrankungen, die z.B. durch Viren, Spirochäten, Pilze, Bakterien oder Mykobakterien hervorgerufen werden, sowie von entzündlichen Erkrankungen unbekannter Ätiologie, wie der Polyarthritis, dem Lupus erythematodes, der Peri- oder Polyarteriitis, der Dermatomyositis, der Sklerodermie und der Sarkoidose.

Weiterhin können die erfindungsgemäßen Verbindungen eingesetzt werden für die Behandlung von zentralnervösen Erkrankungen wie Depressionen, Angstzuständen und chronischen Schmerzen, insbesondere Migräne, sowie bei neurodegenerativen Erkrankungen wie der Alzheimer'schen Krankheit und dem Parkinson-Syndrom.

Die erfindungsgemäßen Verbindungen sind auch geeignet für die Prophylaxe und/oder Behandlung von vaskulären Schäden, z.B. nach Interventionen wie percutan-transluminaler koronarer Angioplastie (PTCA), Implantationen von Stents, koronarer Angioskopie, Reokklusion oder Restenose nach Bypass-Operationen, sowie bei endothelialer Dysfunktion, bei Morbus Raynaud, bei der Thrombangiitis obliterans (Buerger-Syndrom) und beim Tinnitus-Syndrom.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker und Rho-Kinase-Inhibitoren;
- Diuretika, insbesondere Schleifendiuretika sowie Thiazide und Thiazid-ähnliche Diuretika;
- antithrombotisch wirkende Mittel, beispielhaft und vorzugsweise aus der Gruppe der Thrombozytenaggregationshemmer, der Antikoagulantien oder der profibrinolytischen Substanzen;
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten;
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- positiv-inotrop wirksame Verbindungen, wie beispielsweise Herzglycoside (Digoxin), betaadrenerge und dopaminerge Agonisten wie Isoproterenol, Adrenalin, Noradrenalin, Dopamin und Dobutamin;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, sowie PDE 3-Inhibitoren wie Amrinone und Milrinone;
- natriuretische Peptide, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Calcium-Sensitizer, wie beispielhaft und vorzugsweise Levosimendan;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- NO- und Häm-unabhängige Aktivatoren der Guanylatcyclase, wie insbesondere die in WO 01/19355, WO 01/19776, WO 01/19778, WO 01/19780, WO 02/070462 und WO 02/070510 beschriebenen Verbindungen;
- Inhibitoren der humanen neutrophilen Elastase (HNE), wie beispielsweise Sivelestat oder DX-890 (Reltran);
- die Signaltransduktionskaskade inhibierende Verbindungen, wie beispielsweise Tyrosinkinase-Inhibitoren, insbesondere Sorafenib, Imatinib, Gefitinib und Erlotinib; und/oder
- den Energiestoffwechsel des Herzens beeinflussende Verbindungen, wie beispielhaft und vorzugsweise Etomoxir, Dichloracetat, Ranolazine oder Trimetazidine.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600, SPP-635, SPP-676, SPP-800 oder SPP-1148, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Unter antithrombotisch wirkenden Mitteln (Antithrombotika) werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705, BAY 60-5521, BAY 78-7499 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- AIBN: 2,2'-Azobis-2-methylpropannitril
- cat.: katalytisch
- CI: chemische Ionisation (bei MS)
- d: Tag(e)
- DC: Dünnschichtchromatographie
- DME: 1,2-Dimethoxyethan
- DMF: Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- ent: Enantiomer / enantiomerenrein
- eq: Äquivalent(e)
- ESI: Elektrospray-Ionisation (bei MS)
- GC-MS: Gaschromatographie-gekoppelte Massenspektrometrie
- Gew.-%: Gewichtsprozent
- h: Stunde(n)
- HPLC: Hochdruck-, Hochleistungsflüssigchromatographie
- konz.: konzentriert
- LC-MS: Flüssigchromatographie-gekoppelte Massenspektrometrie
- min: Minute(n)
- MPLC: Mitteldruckflüssigchromatographie
- MS: Massenspektrometrie
- NMR: Kernresonanzspektrometrie
- Ph: Phenyl
- R_{f}: Retentionsindex (bei DC)
- Rₜ: Retentionszeit (bei HPLC)
- RT: Raumtemperatur
- THF: Tetrahydrofuran
- v/v: Volumen-zu-Volumen-Verhältnis (einer Lösung)
- wässr.: wässrig, wässrige Lösung

### LC-MS- und GC-MS-Methoden:

### Methode 1 (LC-MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 3 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 4 (LC-MS):

Instrument: Micromass Platform LCZ mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 5 (GC-MS):

Instrument: Micromass GCT, GC 6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

### Methode 6 (LC-MS):

Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### Methode 7 (LC-MS):

Instrument: Micromass Quattro LCZ mit HPLC Agilent Serie 1100; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Methode 8 (LC-MS):

Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 9 (LC-MS):

Instrument: Micromass Quattro Premier mit Waters UPLC Acquity; Säule: Thermo Hypersil GOLD 1.9µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

**Ausgangsverbindungen und Intermediate:**

### Beispiel 1A

### 1-[2-(Allyloxy)phenyl] ethanon

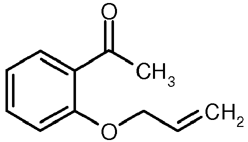

542 g (3.9 mol) 2-Hydroxyacetophenon werden mit 592 g (4.9 mol) Allylbromid, 1000 g (7.2 mol) Kaliumcarbonat und 13.2 g (79 mmol) Kaliumiodid in 2.4 Liter Aceton 24 h lang zum Rückfluss erhitzt. Nach Abkühlen auf Raumtemperatur wird filtriert und das Lösungsmittel im Vakuum entfernt. Der Rückstand wird in Toluol gelöst und mit 10%-iger Natronlauge und Wasser gewaschen. Nach Einengen werden 689 g (98% d. Th.) der Titelverbindung erhalten.

¹H-NMR (300 MHz, CDCl₃): δ = 2.68 (s, 3H), 4.68 (dd, 2H), 5.89 (dd, 2H), 6.09 (m, 1H), 6.99 (dd, 2H), 7.44 (m, 1H), 7.71 (d, 1H).

### Beispiel 2A

### 1-(3-Allyl-2-hydroxyphenyl)ethanon

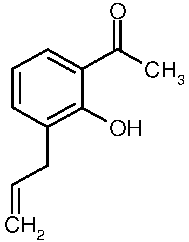

160 g (0.9 mol) 1-[2-(Allyloxy)phenyl]ethanon werden im Metallbad 4 h lang bei 230-240°C gerührt. Nach Abkühlen auf Raumtemperatur wird das Produkt über einen Dünnschichtverdampfer bei 140°C und 0.4 mbar destilliert. Es werden 155 g (97% d. Th.) der Titelverbindung erhalten.

¹H-NMR (300 MHz, CDCl₃): δ = 2.68 (s, 3H), 3.44 (d, 2H), 5.09 (m, 2H), 6.01 (m, 1H), 6.85 (t, 1H), 7.38 (dd, 1H), 7.62 (dd, 1H), 12.61 (s, 1H).

### Beispiel 3A

### 1-{2-Hydroxy-3-[(1E)-prop-1-en-1-yl]phenyl}ethanon

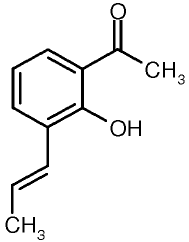

40 g (227 mmol) 1-(3-Allyl-2-hydroxyphenyl)ethanon werden in 120 ml Toluol gelöst und mit 2.17 g (5.6 mmol) Bis(benzonitril)dichlorpalladium(II) versetzt. Die Reaktionsmischung wird über Nacht auf 120°C erhitzt. Nach Abkühlen auf Raumtemperatur wird über Kieselgur filtriert und das Lösungsmittel im Vakuum entfernt. Es werden 20.9 g (95% d.Th.) der Titelverbindung erhalten, welche ohne weitere Reinigung in der nächsten Stufe umgesetzt wird.

LC-MS (Methode 1): Rₜ = 2.36 min; [M+H]⁺ = 177

¹H-NMR (300 MHz, CDCl₃): δ = 1.91 (dd, 3H), 2.63 (s, 3H), 6.32 (m, 1H), 6.73 (dd, 1H), 6.85 (t, 1H), 7.59 (m, 2H), 12.74 (s, 1H).

### Beispiel 4A

### 2-Methyl-8-[(1E)-prop-1-en-1-yl]-4H-chromen-4-on

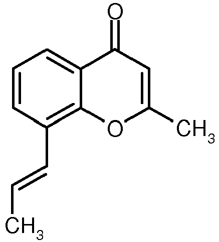

12.52 g (313.2 mmol) 60%-iges Natriumhydrid (Suspension in Mineralöl) werden unter Argon bei 10°C in 300 ml absolutem THF vorgelegt. Zu der Suspension werden 18.4 g (104.4 mmol) 1-{2-Hydroxy-3-[(1*E*)-prop-1-en-1-yl]phenyl}ethanon langsam zugetropft. Nach 15 min werden 9 g (114.9 mmol) Acetylchlorid zugegeben. Die Reaktionsmischung wird über Nacht bei Raumtemperatur gerührt. Man hydrolysiert mit 300 ml Wasser und extrahiert mehrfach mit Ethylacetat.

Nach Waschen der organischen Phase mit gesättigter Natriumchlorid-Lösung wird über Natriumsulfat getrocknet. Anschließend wird das Lösungsmittel im Vakuum entfernt. Der Rückstand wird in 200 ml Methanol aufgenommen und mit 50 ml 20%-iger Salzsäure 30 min auf 80°C erhitzt. Anschließend wird das Lösungsmittel im Vakuum entfernt und der Rückstand mit 400 ml Wasser versetzt. Es wird mehrfach mit Dichlormethan extrahiert. Nach Trocknen der organischen Phase über Magnesiumsulfat wird das Lösungsmittel im Vakuum entfernt und der Rückstand mittels Säulenchromatographie gereinigt (Laufmittel: Dichlormethan/Methanol 98:2). Es werden 10.5 g (50.2% d. Th.) der Titelverbindung als gelbes Öl erhalten.

LC-MS (Methode 2): Rₜ = 2.07 min; [M+H]⁺ = 201

¹H-NMR (300 MHz, CDCl₃): δ = 1.98 (dd, 3H), 2.43 (s, 3H), 6.18 (s, 1H), 6.40 (m, 1H), 6.85 (dd, 1H), 7.31 (t, 1H), 7.72 (dd, 1H), 8.05 (dd, 1H).

### Beispiel 5A

### 2-Methyl-4-oxo-4H-chromen-8-carbaldehyd

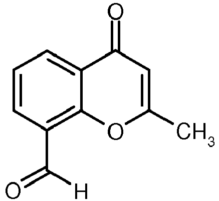

18.5 g (62.8 mmol) 2-Methyl-8-[(1*E*)-prop-1-en-1-yl]-4*H*-chromen-4-on werden in 400 ml Dichlormethan gelöst und auf -60°C abgekühlt. In die Reaktionslösung wird 30 min lang Ozon eingeleitet. Anschließend wird die Reaktionsmischung mit Dimethylsulfid versetzt. Nach Erwärmen auf Raumtemperatur wird das Lösungsmittel im Vakuum entfernt und der Rückstand mit wenig Methanol aufgeschlämmt. Nach Filtration wird der verbleibende Feststoff aus Diethylether umkristallisiert. Es werden 9.1 g (77.4% d. Th.) der Titelverbindung erhalten.

LC-MS (Methode 1): Rₜ = 1.31 min; [M+H]⁺ = 189

¹H-NMR (300 MHz, CDCl₃): δ = 2.48 (s, 3H), 6.27 (s, 1H), 7.51 (m, 1H), 8.21 (dd, 1H), 8.46 (dd, 1H), 10.67 (s, 1H).

### Beispiel 6A

### 4-Brom-2-(trifluormethoxy)benzaldehyd

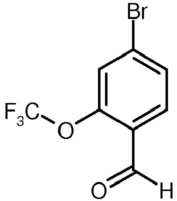

20.00 g (54.51 mmol) 4-Brom-2-(trifluormethoxy)iodbenzol werden in 200 ml THF gelöst und auf -78°C gekühlt. Anschließend werden 26.16 ml (65.41 mmol) einer 2.5 M Lösung von n-Butyllithium in Hexan zugetropft. Es wird 30 min nachgerührt und anschließend 14.43 g (125.37 mmol) *N*-Formylmorpholin zudosiert. Nachdem vollständiger Umsatz detektiert ist (DC-Kontrolle), wird bei -78°C mit Isopropanol solvolysiert. Nach dem Erwärmen auf Raumtemperatur wird mit Wasser versetzt und zweimal mit Dichlormethan extrahiert. Die vereinigten organischen Phasen werden mit gesättigter Natriumchlorid-Lösung gewaschen, mit Natriumsulfat getrocknet und das Lösungsmittel unter vermindertem Druck abdestilliert. Der Rückstand wird säulenchromatographisch gereinigt (Kieselgel, Laufmittel: Cyclohexan/Essigsäureethylester 5:1). Es werden 11.43 g (78% d. Th.) der Titelverbindung erhalten.

GC-MS (Methode 5): Rₜ = 4.24 min; MS (EIpos): m/z = 270 [M+H]⁺

¹H-NMR (300 MHz, DMSO-d₆): δ = 7.85-7.92 (m, 3H), 10.20 (s, 1H).

### Beispiel 7A

### 4-Formyl-3-(trifluormethoxy)benzonitril

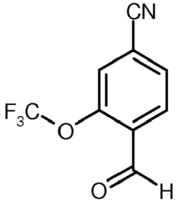

10.63 g (39.51 mmol) 4-Brom-2-(trifluormethoxy)benzaldehyd, 3.43 g (29.24 mmol) Zinkcyanid und 1.37 g (1.19 mmol) Tetrakis(triphenylphosphin)palladium(0) werden in 80 ml DMF gelöst. Anschließend wird die Reaktionsmischung in mehreren Portionen in einer Single Mode-Mikrowelle (Emrys Optimizer, 5 min bei 220°C) umgesetzt. Die vereinigten Ansätze werden mit Wasser versetzt und zweimal mit Toluol extrahiert. Die vereinigten organischen Phasen werden mit gesättigter Natriumchlorid-Lösung gewaschen, mit Natriumsulfat getrocknet und anschließend das Lösungsmittel am Rotationsverdampfer entfernt. Der Rückstand wird säulenchromatographisch aufgereinigt (Kieselgel, Laufmittel: Cyclohexan/Essigsäureethylester 10:1). Es werden 3.32 g (78% d. Th.) der Titelverbindung in 80%-iger Reinheit (nach LC-MS) erhalten.

MS (EIpos): m/z = 215 [M]⁺

¹H-NMR (300 MHz, DMSO-d₆): δ = 7.85-7.91 (m, 3H), 10.20 (s, 1H).

### Beispiel 8A

### 4-Cyano-2-methoxyphenyl-trifluormethansulfonat

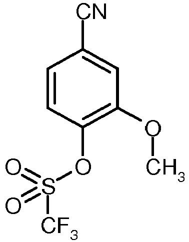

Zu einer Lösung von 20 g (134 mmol) 4-Hydroxy-3-methoxybenzonitril in Pyridin (80 ml) werden langsam 24 ml (141 mmol) Trifluormethansulfonsäureanhydrid getropft, wobei die Reaktionstemperatur mit Hilfe eines Eisbads unter 25°C gehalten wird. Die Suspension wird dann 1 h bei RT gerührt. Eiswasser (400 ml) wird zugegeben und die Suspension noch bis zum Erreichen der Raumtemperatur weiter gerührt. Dann wird filtriert, der Feststoff in Ethylacetat gelöst und diese Lösung mit gesättigter Natriumchlorid-Lösung gewaschen. Die organische Phase wird über Magnesiumsulfat getrocknet und eingeengt. Es werden 37.13 g (92% d. Th.) der Titelverbindung als weißer Feststoff erhalten.

LC-MS (Methode 3): Rt = 2.54 min; MS (EIpos): m/z = 282 [M+H]⁺

¹H-NMR (300 MHz, DMSO-d₆): δ = 3.97 (s, 3H), 7.60 (dd, 1H), 7.71 (d, 1H), 7.92 (d, 1H).

### Beispiel 9A

### tert.-Butyl (2E)-3-(4-cyano-2-methoxyphenyl)acrylat

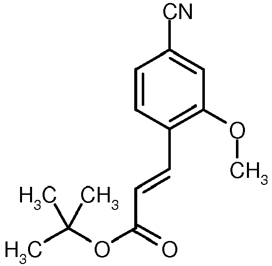

Zu einer entgasten Lösung von 37.13 g (132 mmol) 4-Cyano-2-methoxyphenyl-trifluormethansulfonat, 35 ml (245 mmol) *tert*.-Butylacrylat und 90 ml (645 mmol) Trimethylamin in DMF (250 ml) werden 4 g (5.7 mmol) Bis(triphenylphosphin)palladium(II)chlorid hinzugefügt. Die Lösung wird unter Schutzgasatmosphäre 24 h lang bei 100°C gerührt. Anschließend wird Eiswasser (1000 ml) zugegeben und die Suspension mit Ethylacetat (3 x 100 ml) extrahiert. Die organische Phase wird mit gesättigter Natriumchlorid-Lösung gewaschen, über Magnesiumsulfat getrocknet und eingeengt. Der Rückstand wird säulenchromatographisch gereinigt (Kieselgel, Laufmittel: Cyclohexan-Essigsäureethylester 10:1). Es werden 24.6 g (72% d. Th.) der Titelverbindung als weißer Feststoff erhalten.

LC-MS (Methode 1): Rₜ = 2.59 min; MS (EIpos): m/z = 260 [M+H]⁺

¹H-NMR (300 MHz, DMSO-d₆): δ = 1.48 (s, 9H), 3.93 (s, 3H), 6.65 (d, 1H), 7.42 (d, 1H), 7.58 (s, 1H), 7.74 (d, 1H), 7.89 (d, 1H).

### Beispiel 10A

### 4-Formyl-3-methoxybenzonitril

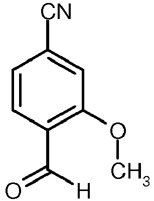

Zu einer kräftig gerührten Lösung von 48 g (185 mmol) *tert*.-Butyl (2*E*)-3-(4-cyano-2-methoxyphenyl)acrylat, 207 mg (0.81 mmol) Osmiumtetroxid und 1.4 g (6.14 mmol) Benzyltriethylammioniumchlorid in 750 ml Wasser/THF (2:1) werden 79 g (370 mmol) Natriummetaperiodat portionsweise zugegeben, wobei die Reaktionstemperatur unter 30°C gehalten wird. Die Lösung wird 1 h bei RT weitergerührt. Wasser (2000 ml) wird zugegeben und die Mischung anschließend filtriert. Der verbleibende Feststoff wird in Ethylacetat gelöst und die Lösung mit gesättigter Natriumchlorid-Lösung gewaschen. Die organische Phase wird über Magnesiumsulfat getrocknet und eingeengt. Der Rückstand wird mit Petrolether verrührt. Es werden 21.18 g (71% d. Th.) der Titelverbindung als weißer Feststoff erhalten.

LC-MS (Methode 3): Rt = 1.87 min; MS (EIpos): m/z = 162 [M+H]⁺

¹H-NMR (300 MHz, DMSO-d₆): δ = 3.98 (s, 3H), 7.53 (d, 1H), 7.80 (s, 1H), 7.81 (d, 1H), 10.37 (s, 1H).

### Beispiel 11A

### 4-Formyl-3-hydroxybenzonitril

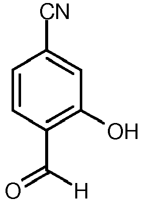

Zu einer Lösung von 8 g (49.64 mmol) 4-Formyl-3-methoxybenzonitril in 80 ml wasserfreiem Dichlormethan werden unter Argonatmosphäre bei -78°C 100 ml einer Bortribromid-Lösung in Dichlormethan (1 M, 100 mmol) getropft. Das Reaktionsgemisch wird bis zur vollen Umsetzung des Eduktes bei RT gerührt (ca. 5 Tage). Die Reaktionslösung wird dann bei 0°C mit gesättigter Natriumhydrogencarbonat-Lösung neutral gestellt. Die Phasen werden getrennt und die organische Phase wird mit gesättigter Natriumchlorid-Lösung gewaschen, über Magnesiumsulfat getrocknet und eingeengt. Der Rückstand wird mittels Säulenchromatographie an Kieselgel gereinigt (Laufmittels: Cyclohexan/Ethylacetat 3:1). Es werden 4.5 g (61% d. Th.) der Titelverbindung als gelber Feststoff erhalten.

LC-MS (Methode 1): Rₜ = 1.38 min; [M-H]- = 146

¹H-NMR (300 MHz, CDCl₃): δ = 7.38 (d, 1H), 7.38 (s, 1H), 7.77 (d, 1H), 10.33 (s, 1H), 11.38 (s, 1H).

### Beispiel 12A

### 5-Cyano-2-formylphenyl-trifluormethansulfonat

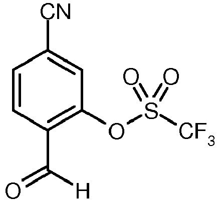

Zu einer Lösung von 2 g (13.59 mmol) 4-Formyl-3-hydroxybenzonitril und 2.5 ml (14.27 mmol) *N,N*-Diisopropylethylamin in 37 ml wasserfreiem Dichlormethan werden unter Argonatmosphäre bei 0°C 2.4 ml (14.27 mmol) Trifluormethansulfonsäureanhydrid getropft. Die Reaktionsmischung wird 1 h bei RT nachgerührt, dann mit 70 ml Dichlormethan verdünnt und nacheinander mit 1 M Salzsäure, gesättigter Natriumhydrogencarbonat-Lösung und gesättigter Natriumchlorid-Lösung gewaschen. Die organische Lösung wird über Magnesiumsulfat getrocknet und eingeengt. Der Rückstand wird mittels Säulenchromatographie an Kieselgel gereinigt (laufmittel: Cyclohexan/ Ethylacetat 7:1). Es werden 2.36 g (62% d. Th.) der Titelverbindung als weißer Feststoff erhalten.

LC-MS (Methode 3): Rₜ = 2.34 min; [M+H]⁺ = 280

¹H-NMR (300 MHz, CDCl₃): δ = 8.27 (m, 2H), 8.33 (s, 1H), 10.13 (s, 1H).

### Beispiel 13A

### 4-Formyl-3-vinylbenzonitril

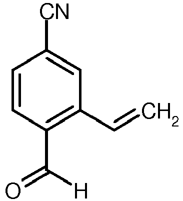

Zu einer Lösung von 1 g (3.58 mmol) 5-Cyano-2-formylphenyl-trifluormethansulfonat und 1.15 ml (3.94 mmol) Tri-n-butylvinylstannan in 6 ml wasserfreiem und entgastem DMF werden unter Argonatmosphäre 125 mg (0.18 mmol) Bis(triphenylphosphin)palladium(II)chlorid gegeben. Die Reaktionsmischung wird anschließend 90 min bei 80°C gerührt. Danach werden 100 ml 10%-ige Kaliumfluorid-Lösung zugesetzt und das Gemisch 1 h bei RT nachgerührt. Die Suspension wird dreimal mit jeweils 20 ml Ethylacetat extrahiert und die vereinigten organischen Phasen nacheinander mit gesättigter Natriumhydrogencarbonat-Lösung und gesättigter Natriumchlorid-Lösung gewaschen. Die organische Lösung wird über Magnesiumsulfat getrocknet und eingeengt. Der Rückstand (0.6 g) wird ohne weitere Reinigung in der nächsten Stufe eingesetzt.

GC-MS (Methode 5): Rt = 5.02 min; [M]⁺= 157

¹H-NMR (300 MHz, CDCl₃): δ = 5.62 (d, 1H), 6.05 (d, 1H), 7.58 (dd, 1H), 7.95 (d, 1H), 8.00 (d, 1H), 8.24 (s, 1H), 10.32 (s, 1H).

### Beispiel 14A

### 3-Ethyl-4-formylbenzonitril

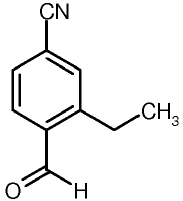

Eine Lösung von 1.3 g (8.27 mmol) 4-Formyl-3-vinylbenzonitril in 35 ml Ethanol wird mit 880 mg 10%-igem Palladium auf Kohle versetzt und 2 h lang unter einer Wasserstoff-Atmosphäre kräftig gerührt. Die Suspension wird durch eine Kieselgurschicht filtriert, der Rückstand mit Ethanol gewaschen und das Filtrat eingeengt. Der Rückstand (890 mg) wird ohne weitere Reinigung in der Folgestufe eingesetzt.

¹H-NMR (300 MHz, CDCl₃): δ = 1.2 (t, 1H), 3.07 (q, 2H), 7.88 (d, 1H), 7.90 (s, 1H), 7.97 (d, 1H), 10.32 (s, 1H).

### Beispiel 15A

### Methyl 4-cyano-2-fluorbenzoat

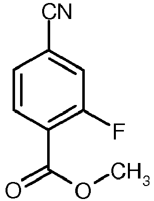

13.20 g (79.9 mmol) 4-Cyano-2-fluorbenzoesäure werden in 300 ml Aceton gelöst. Anschließend werden nacheinander 22.10 g (159.9 mmol) Kaliumcarbonat und 9.08 ml (95.9 mmol) Dimethylsulfat zugesetzt. Es wird 20 h bei Rückflusstemperatur gerührt. Das Reaktionsgemisch wird danach mit 300 ml Wasser versetzt und das Aceton am Rotationsverdampfer entfernt. Es wird mehrfach mit Dichlormethan extrahiert. Die vereinigten organischen Phasen werden mit gesättigter Natriumchlorid-Lösung gewaschen und über Natriumsulfat getrocknet. Dann wird das Lösungsmittel im Vakuum entfernt. Der verbleibende Feststoff wird ohne weitere Aufreinigung weiterverwendet. Es werden 16.1 g (84% d. Th.) der Titelverbindung als farbloser Feststoff erhalten.

GC-MS (Methode 5): Rt = 6.23 min; [M]⁺ (EIpos): m/z = 179

¹H-NMR (300 MHz, DMSO-d₆): δ = 3.90 (s, 3H), 7.83 (dd, 1H), 8.01-8.08 (m, 2H).

### Beispiel 16A

### 3-Fluor-4-(hydroxymethyl)benzonitril

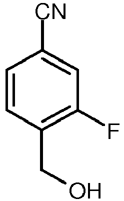

16.10 g (89.9 mmol) Methyl 4-cyano-2-fluorbenzoat werden in 150 ml Methanol gelöst. Anschließend werden portionsweise 3.40 g (89.9 mmol) Natriumborhydrid zugesetzt. Nach erfolgter Umsetzung (DC-Kontrolle) wird mit verdünnter Salzsäure auf pH 3 eingestellt und mehrfach mit Dichlormethan extrahiert. Die vereinigten organischen Phasen werden mit gesättigter Natriumchlorid-Lösung gewaschen und mit Magnesiumsulfat getrocknet. Dann wird das Lösungsmittel im Vakuum entfernt und der Rückstand per Säulenchromatographie aufgereinigt (Kieselgel, Laufmittel: Cyclohexan/Ethylacetat 15:1 → 3:7). Es werden 3.70 g (27.2% d. Th.) der Titelverbindung erhalten.

GC-MS (Methode 5): Rt = 6.51 min; [M]⁺ (EIpos): m/z = 151

¹H-NMR (300 MHz, DMSO-d₆): δ = 4.61 (s, 2H), 5.53 (s, 1H), 7.61-7.74 (m, 2H), 7.79 (dd, 1H).

### Beispiel 17A

### 3-Fluor-4-formylbenzonitril

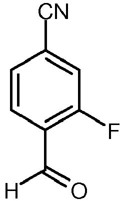

1.00 g (6.62 mmol) 3-Fluor-4-(hydroxymethyl)benzonitril werden in 50 ml Dichlormethan gelöst und mit 9.20 g (105.9 mmol) Mangan(IV)oxid versetzt. Es wird über Nacht bei Raumtemperatur gerührt und dann über eine kurze Kieselgur-Säule filtriert. Das Lösungsmittel wird unter vermindertem Druck abdestilliert und der Rückstand per Säulenchromatographie aufgereinigt (Kieselgel, Laufmittel: Dichlormethan). Es werden 120 mg (12.1% d. Th.) der Titelverbindung erhalten.

GC-MS (Methode 5): Rt = 5.11 min; [M]⁺ (EIpos): m/z = 149

¹H-NMR (300 MHz, DMSO-d₆): δ = 7.89 (d, 1H), 8.00 (t, 1H), 8.11 (d, 1H), 10.24 (d, 1H).

### Beispiel 18A

### 3-Chlor-4-formylbenzonitril

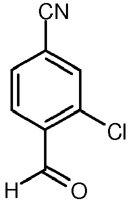

25.0 g (164.91 mmol) 3-Chlor-4-methylbenzonitril werden in 150 ml DMF gelöst und mit 25.55 g (214.39 mmol) *N,N*-Dimethylformamid-dimethylacetal versetzt. Es wird 20 h bei 140°C und dann 4 h bei 180°C Ölbadtemperatur gerührt. Die flüchtigen Komponenten werden am Rotationsverdampfer abgetrennt und der verbleibende Rückstand direkt weiter umgesetzt.

Das so erhaltene, rohe 3-Chlor-4-[2-(dimethylamino)vinyl]benzonitril wird in 500 ml THF/Wasser (1:1) aufgenommen und mit 77.6 g (362.9 mmol) Natriumperiodat versetzt. Es wird 18 h bei Raumtemperatur gerührt und anschließend der ausgefallene Niederschlag per Filtration abgetrennt. Das Filtrat wird mit gesättigter Natriumhydrogencarbonat-Lösung versetzt und dreimal mit Ethylacetat extrahiert. Die vereinigten organischen Phasen werden mit Natriumsulfat getrocknet und das Lösungsmittel am Rotationsverdampfer entfernt. Das Rohprodukt wird säulenchromatographisch aufgereinigt (Kieselgel, Laufmittel: Cyclohexan/Ethylacetat 7:3). Es werden 3.0 g (15% d. Th.) der Titelverbindung erhalten.

GC-MS (Methode 5): Rt = 6.64 min; [M]⁺ (EIpos): m/z = 165

¹H-NMR (300 MHz, DMSO-d₆): δ = 7.97-8.03 (m, 2H), 8.27 (s, 1H), 10.34 (d, 1H).

### Beispiel 19A

### 4-Formyl-1-naphthonitril

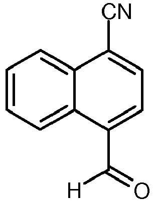

2.50 g (14.95 mmol) 4-Methyl-1-naphthonitril werden in 40 ml Tetrachlormethan gelöst und mit 3.19 g (17.94 mmol) *N*-Bromsuccinimid sowie 245 mg (1.50 mmol) 2,2'-Azobis-2-methylpropannitril versetzt. Es wird über Nacht bei Rückflusstemperatur gerührt. Nach dem Abkühlen wird das Produkt abfiltriert. Es werden 2.75 g (74.7% d. Th.) 4-(Brommethyl)-1-naphthonitril in 90%-iger Reinheit erhalten, welches ohne weitere Aufreinigung umgesetzt wird.

2.75 g (11.17 mmol) des so erhaltenen Bromide werden in 60 ml Acetonitril gelöst und mit 2 g Molekularsieb (3Å) versetzt. Anschließend werden 1.44 g (12.29 mmol) *N*-Methylmorpholin-*N-*oxid hinzugefügt und das Gemisch über Nacht bei Raumtemperatur gerührt. Der Ansatz wird danach über Kieselgel filtriert und das Filtrat eingeengt. Der Rückstand wird über eine Biotage-Kartusche (40 M) gereinigt (Eluent: Isohexan/Ethylacetat 3:1). Die Produktfraktionen werden vereinigt, das Lösungsmittel am Rotationsverdampfer entfernt und der Rückstand anschließend mit Diethylether verrührt, wobei Kristallisation eintritt. Es wird mit wenig Diethylether gewaschen und im Hochvakuum getrocknet. Es werden 254 mg (12.6% d. Th.) der Titelverbindung erhalten.

LC-MS (Methode 3): Rt = 2.27 min; [M+H]⁺ (EIpos): m/z = 182

¹H-NMR (300 MHz, CDCl₃): δ = 7.79-7.87 (m, 2H), 8.05 (d, 1H), 8.09 (d, 1H), 8.37 (m, 1H), 9.27 (m, 1H), 10.51 (s, 1H).

### Beispiel 20A

### 2-Cyanoethyl 4-(4-cyano-2-methoxyphenyl)-2-methyl-5-oxo-1,4,5,6-tetrahydro-1,6-naphthyridin-3-carboxylat

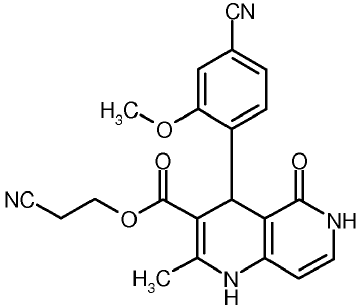

14.63 g (90.81 mmol) der Verbindung aus Beispiel 10A, 10.00 g (90.81 mmol) 4-aminopyridin-2(1*H*)-on [Searls, T., McLaughlin, L.W., Tetrahedron 55, 11985-11996 (1999)] und 15.65 g (90.81 mmol) 2-Cyanoethyl-3-oxobutanoat [Yamamoto, T., et al., Bioorg. Med. Chem. Lett. 16, 798-802 (2006)] werden in 300 ml Isopropanol gelöst und 3 Tage lang unter Argon bei Rückflusstemperatur gerührt. Das Gemisch wird danach eingeengt und anschließend säulenchromatographisch aufgereinigt (Kieselgel; Laufmittel: zunächst Essigsäureethylester, dann Dichlormethan/ Methanol 10:1). Die erhaltenen Produktfraktionen werden eingeengt und anschließend in wenig Essigsäureethylester aufgenommen. Das ausgefallene Produkt wird abfiltriert und über Nacht im Vakuum bei 40°C getrocknet. Man erhält 10.11 g (27% d. Th.) der Titelverbindung.

LC-MS (Methode 6): Rt = 1.83 min; MS (EIpos): m/z = 391 [M+H]⁺

¹H-NMR (300 MHz, DMSO-d₆): δ = 2.27 (s, 3H), 2.79 (m, 2H), 3.75 (s, 3H), 3.96-4.14 (m, 2H), 5.19 (s, 1H), 5.87 (d, 1H), 7.10 (d, 1H), 7.23 (dd, 1H), 7.30-7.35 (m, 2H), 9.30 (s, 1H), 10.83 (s, 1H).

### Beispiel 21A

### 2-Cyanoethyl 4-(4-cyano-2-methoxyphenyl)-5-ethoxy-2-methyl-1,4-dihydro-1,6-naphthyridin-3-carboxylat

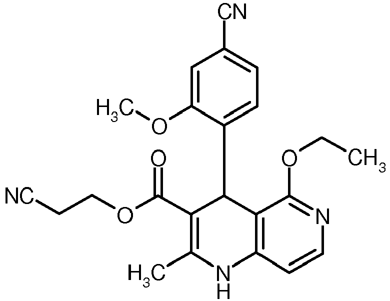

10.00 g (25.62 mmol) der Verbindung aus Beispiel 20A werden in 250 ml Orthoameisensäuretriethylester suspendiert und auf 130°C erhitzt. Danach wird das Reaktionsgemisch über einen Zeitraum von insgesamt 8 Stunden stündlich mit 15 Tropfen konzentrierter Schwefelsäure versetzt. Anschließend wird über Nacht bei gleicher Temperatur nachgerührt. Nach dem Abkühlen wird überschüssiger Orthoester am Rotationsverdampfer entfernt und das Rohprodukt mittels Säulenchromatographie aufgereinigt (Kieselgel; Laufmittel: Cyclohexan/Essigsäureethylester 1:1). Man erhält 7.20 g (65% d. Th.) der Titelverbindung.

LC-MS (Methode 6): Rt = 2.82 min; MS (EIpos): m/z = 419 [M+H]⁺

¹H-NMR (300 MHz, DMSO-d₆): δ = 1.12 (t, 3H), 2.33 (s, 3H), 2.77 (m, 2H), 3.78 (s, 3H), 3.99-4.13 (m, 4H), 5.37 (s, 1H), 6.48 (d, 1H), 7.25 (dd, 1H), 7.29-7.35 (m, 2H), 7.73 (d, 1H), 9.53 (s, 1H).

### Beispiel 22A

### 4-(4-Cyano-2-methoxyphenyl)-5-ethoxy-2-methyl-1,4-dihydro-1,6-naphthyridin-3-carbonsäure

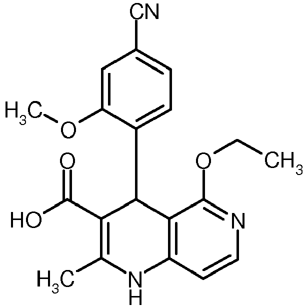

7.20 g (17.21 mmol) der Verbindung aus Beispiel 21A werden in 200 ml 1,2-Dimethoxyethan/ Wasser (3:1 v/v) gelöst, mit 34.42 ml (34.42 mmol) 1 N Natronlauge versetzt und über Nacht bei Raumtemperatur gerührt. Der Ansatz wird danach mit 100 ml Diethylether und 100 ml Wasser versetzt, die organische Phase abgetrennt und die wässrige Phase mit 1 N Salzsäure auf pH 4-5 eingestellt. Die entstandene Suspension wird 1 h gerührt und der ausgefallene Feststoff anschließend per Filtration abgetrennt. Das Präzipitat wird mit Wasser und etwas Diethylether gewaschen. Nach dem Trocknen bei 40°C im Vakuum erhält man 3.57 g (57% d. Th.) der Titelverbindung.

LC-MS (Methode 7): Rₜ = 2.32 min; MS (EIpos): m/z = 366 [M+H]⁺

¹H-NMR (300 MHz, DMSO-d₆): δ = 1.12 (t, 3H), 2.28 (s, 3H), 3.74 (s, 3H), 4.07 (m, 2H), 5.33 (s, 1H), 6.44 (d, 1H), 7.23-7.29 (m, 2H), 7.32 (s, 1H), 7.70 (d, 1H), 9.25 (s, 1H), 11.34 (s, 1H).

### Beispiel 23A

### 4-[5-Ethoxy-3-(1H-imidazol-1-ylcarbonyl)-2-methyl-1,4-dihydro-1,6-naphthyridin-4-yl]-3-methoxybenzonitril

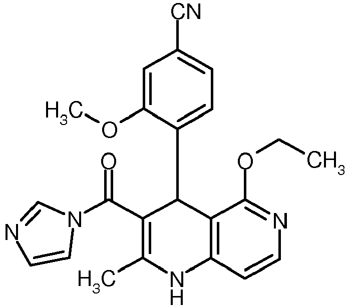

1.20 g (3.28 mmol) der Verbindung aus Beispiel 22A werden in 25 ml Essigsäureethylester vorgelegt, mit 0.666 g (4.11 mmol) 1,1'-Carbonyldiimidazol versetzt und anschließend über Nacht bei Raumtemperatur gerührt. Das Reaktionsgemisch wird am Rotationsverdampfer eingeengt und das so erhaltene Rohprodukt ohne Aufreinigung für weitere Umsetzungen eingesetzt.

MS (EIpos): m/z = 416 [M+H]⁺.

### Beispiel 24A

### 2-Cyanoethyl 2-(4-cyano-2-methoxybenzyliden)-3-oxobutanoat

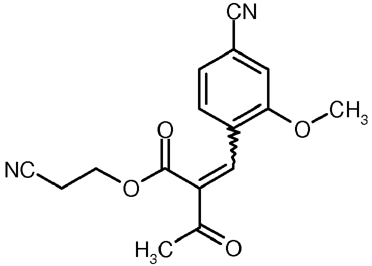

3.00 g (18.62 mmol) der Verbindung aus Beispiel 10A, 3.18 g (20.48 mmol) 2-Cyanoethyl-3-oxobutanoat [Yamamoto, T., et al., Bioorg. Med. Chem. Lett. 16, 798-802 (2006)], 213 µl (3.72 mmol) Essigsäure und 368 µl (3.72 mmol) Piperidin werden in 50 ml wasserfreiem Dichlormethan gelöst und über Nacht unter Rückfluss am Wasserabscheider gerührt. Die flüchtigen Komponenten werden danach am Rotationsverdampfer abgetrennt und der Rückstand mittels Säulenchromatographie aufgereinigt (Kieselgel; Laufmittel: Gradient Cyclohexan/Essigsäureethylester 7:3 → 1:1). Es werden 2.77 g (48% d. Th.) der Titelverbindung als Gemisch der E/Z-Isomere erhalten.

LC-MS (Methode 7): Rₜ = 2.89 und 3.00 min; MS (EIpos): m/z = 299 [M+H]⁺.

### Beispiel 25A

### 2-Cyanoethyl 4-(4-cyano-2-methoxyphenyl)-2,7-dimethyl-5-oxo-1,4,5,6-tetrahydro-1,6-naphthyridin-3-carboxylat

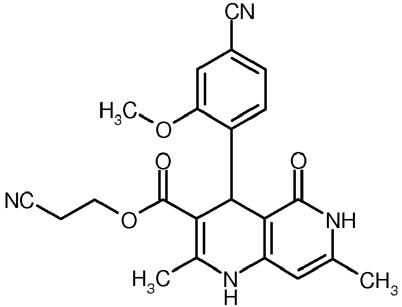

1.49 g (5.00 mmol) der Verbindung aus Beispiel 24A werden in 30 ml 2-Propanol vorgelegt, mit 620 mg (5.00 mmol) 4-Amino-6-methylpyridin-2(1*H*)-on [Bisagni, E., Hung, N.C., Synthesis, 765-766 (1984)] versetzt und anschließend über Nacht bei Rückflusstemperatur gerührt. Nach dem Abkühlen wird das Präzipitat abfiltriert, mit Diethylether gewaschen und im Hochvakuum getrocknet. Man erhält 1.53 g (76% d. Th.) der Titelverbindung.

LC-MS (Methode 3): Rₜ = 1.73 min; MS (EIpos): m/z = 405 [M+H]⁺

¹H-NMR (300 MHz, DMSO-d₆): δ = 2.05 (s, 3H), 2.26 (s, 3H), 2.78 (m, 2H), 3.74 (s, 3H), 3.96-4.13 (m, 2H), 5.14 (s, 1H), 5.64 (d, 1H), 7.23 (dd, 1H), 7.28-7.33 (m, 2H), 9.22 (s, 1H), 10.82 (s, 1H).

### Beispiel 26A

### 2-Cyanoethyl 4-(4-cyano-2-methoxyphenyl)-5-ethoxy-2,7-dimethyl-1,4-dihydro-1,6-naphthyridin-3-carboxylat

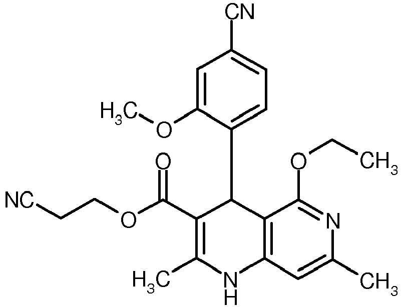

1.52 g (3.76 mmol) der Verbindung aus Beispiel 25A werden in 40 ml Orthoameisensäuretriethylester suspendiert und auf 130°C erhitzt. Danach wird das Reaktionsgemisch über einen Zeitraum von insgesamt 8 Stunden stündlich mit 10 Tropfen konzentrierter Schwefelsäure versetzt. Anschließend wird über Nacht bei gleicher Temperatur nachgerührt. Nach dem Abkühlen wird überschüssiger Orthoester am Rotationsverdampfer entfernt und das Rohprodukt mittels Säulenchromatographie aufgereinigt (Kieselgel; Laufmittel: Cyclohexan/Essigsäureethylester 1:1). Die Produktfraktionen werden vereinigt, das Lösungsmittel entfernt und der Rückstand in wenig Methanol aufgenommen. Das kristallisierende Produkt wird abfiltriert. Nach dem Trocknen im Hochvakuum erhält man 1.09 g (67% d. Th.) der Titelverbindung.

LC-MS (Methode 3): Rt = 2.23 min; MS (EIpos): m/z = 433 [M+H]⁺

¹H-NMR (300 MHz, DMSO-d₆): δ = 1.11 (t, 3H), 2.20 (s, 3H), 2.32 (s, 3H), 2.76 (m, 2H), 3.78 (s, 3H), 3.97-4.12 (m, 4H), 5.32 (s, 1H), 6.30 (s, 1H), 7.24 (d, 1H), 7.27-7.32 (m, 2H), 9.43 (s, 1H).

### Beispiel 27A

### 4-(4-Cyano-2-methoxyphenyl)-5-ethoxy-2,7-dimethyl-1,4-dihydro-1,6-naphthyridin-3-carbonsäure

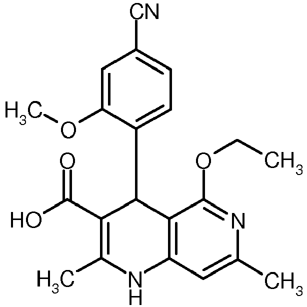

642 mg (2.52 mmol) der Verbindung aus Beispiel 26A werden in 40 ml 1,2-Dimethoxyethan/ Wasser (3:1 v/v) gelöst, mit 5.04 ml (5.04 mmol) 1 N Natronlauge versetzt und über Nacht bei Raumtemperatur gerührt. Der Ansatz wird danach mit 30 ml Diethylether und 30 ml Wasser versetzt, die organische Phase abgetrennt und die wässrige Phase mit 1 N Salzsäure auf pH 4-5 eingestellt. Die entstandene Suspension wird 1 h gerührt und der ausgefallene Feststoff anschließend per Filtration abgetrennt. Das Präzipitat wird mit Wasser und etwas Diethylether gewaschen. Nach dem Trocknen im Vakuum bei 40°C erhält man 642 mg (67% d. Th.) der Titelverbindung.

LC-MS (Methode 3): Rt = 1.87 min; MS (EIpos): m/z = 380 [M+H]⁺

¹H-NMR (300 MHz, DMSO-d₆): δ = 1.11 (t, 3H), 2.19 (s, 3H), 2.28 (s, 3H), 3.74 (s, 3H), 4.05 (m, 2H), 5.28 (s, 1H), 6.27 (s, 1H), 7.20-7.28 (m, 2H), 7.31 (s, 1H), 9.17 (s, 1H), 11.31 (s, 1H).

### Beispiel 28A

### 2-Cyanoethyl 4-(4-cyano-2-methoxyphenyl)-2,8-dimethyl-5-oxo-1,4,5,6-tetrahydro-1,6-naphthyridin-3-carboxylat

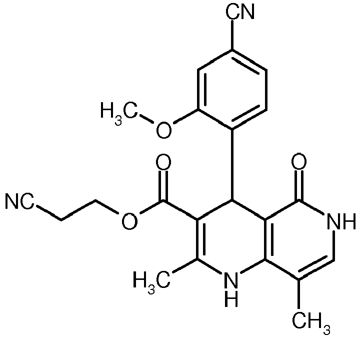

2.69 g (9.00 mmol) der Verbindung aus Beispiel 24A werden in 45 ml 2-Propanol vorgelegt, mit 1.17 g (9.00 mmol) 4-Amino-5-methylpyridin-2(1*H*)-on [Bisagni, E., Hung, N.C., *Synthesis,* 765-766 (1984)] versetzt und anschließend über Nacht bei Rückflusstemperatur gerührt. Nach dem Abkühlen wird das Präzipitat abfiltriert, mit Diethylether gewaschen und im Hochvakuum getrocknet. Man erhält 2.22 g (61% d. Th.) der Titelverbindung.

LC-MS (Methode 3): Rₜ = 1.75 min; MS (EIpos): m/z = 405 [M+H]⁺

¹H-NMR (300 MHz, DMSO-d₆): δ = 2.03 (s, 3H), 2.35 (s, 3H), 2.80 (m, 2H), 3.74 (s, 3H), 4.04 (m, 1H), 4.11 (m, 1H), 5.20 (s, 1H), 6.95 (s, 1H), 7.23 (dd, 1H), 7.28-7.33 (m, 2H), 8.18 (s, 1H), 10.76 (s, 1H).

### Beispiel 29A

### 2-Cyanoethyl 4-(4-cyano-2-methoxyphenyl)-5-ethoxy-2,8-dimethyl-1,4-dihydro-1,6-naphthyridin-3-carboxylat

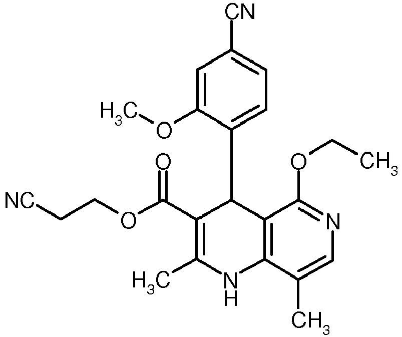

2.22 g (5.44 mmol) der Verbindung aus Beispiel 28A werden in 100 ml Orthoameisensäuretriethylester suspendiert und auf 130°C erhitzt. Danach wird das Reaktionsgemisch über einen Zeitraum von insgesamt 8 Stunden stündlich mit 10 Tropfen konzentrierter Schwefelsäure versetzt. Anschließend wird über Nacht bei gleicher Temperatur nachgerührt. Nach dem Abkühlen wird überschüssiger Orthoester am Rotationsverdampfer entfernt und das Rohprodukt mittels Säulenchromatographie aufgereinigt (Kieselgel; Laufmittel: zunächst Dichlormethan, dann Isohexan/ Essigsäureethylester 1:1). Die Produktfraktionen werden vereinigt, das Lösungsmittel entfernt und der Rückstand aus Essigsäureethylester/Diethylether kristallisiert. Das Präzipitat wird abfiltriert und im Hochvakuum getrocknet. Man erhält 1.80 g (77% d. Th.) der Titelverbindung.

LC-MS (Methode 6): Rt = 3.02 min; MS (EIpos): m/z = 433 [M+H]^{+ 1}H-NMR (300 MHz, DMSO-d₆): δ = 1.11 (t, 3H), 2.16 (s, 3H), 2.42 (s, 3H), 2.78 (m, 2H), 3.77 (s, 3H), 4.01-4.13 (m, 4H), 5.37 (s, 1H), 7.25 (d, 1H), 7.28-7.33 (m, 2H), 7.60 (s, 1H), 8.35 (s, 1H).

### Beispiel 30A

### 4-(4-Cyano-2-methoxyphenyl)-5-ethoxy-2,8-dimethyl-1,4-dihydro-1,6-naphthyridin-3-carbonsäure

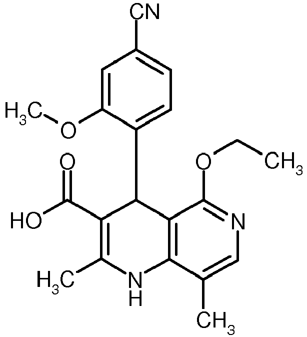

1.75 g (4.05 mmol) der Verbindung aus Beispiel 29A werden in 60 ml 1,2-Dimethoxyethan/ Wasser (2:1 v/v) gelöst, mit 8.09 ml (8.09 mmol) 1 N Natronlauge versetzt und eine Stunde bei Raumtemperatur gerührt. Der Ansatz wird dann mit 30 ml Diethylether versetzt und die wässrige Phase mit 6 N Salzsäure angesäuert. Das entstandene Präzipitat wird abfiltriert und mit Wasser sowie etwas Diethylether gewaschen. Nach dem Trocknen bei 40°C im Vakuumtrockenschrank erhält man 1.47 g (96% d. Th.) der Titelverbindung.

LC-MS (Methode 7): Rₜ = 2.50 min; MS (EIpos): m/z = 380 [M+H]⁺

¹H-NMR (300 MHz, DMSO-d₆): δ = 1.14 (t, 3H), 2.14 (s, 3H), 2.37 (s, 3H), 3.73 (s, 3H), 4.04 (m, 2H), 5.33 (s, 1H), 7.26 (m, 2H), 7.32 (s, 1H), 7.57 (s, 1H), 8.16 (s, 1H), 11.43 (br. s, 1H).

### Beispiel 31A

### 2-Cyanoethyl 2-methyl-4-(2-methyl-4-oxo-4H-chromen-8-yl)-5-oxo-1,4,5,6-tetrahydro-1,6-naphthyridin-3-carboxylat

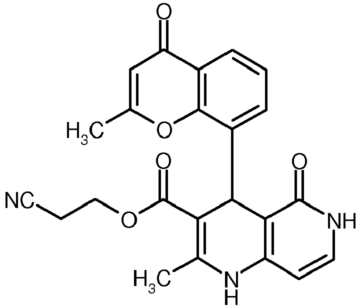

3.00 g (15.94 mmol) der Verbindung aus Beispiel 5A, 1.75 g (15.94 mmol) 4-aminopyridin-2(1*H*)-on [Searls, T., McLaughlin, L.W., Tetrahedron 55, 11985-11996 (1999)] und 2.47 g (15.94 mmol) 2-Cyanoethyl-3-oxobutanoat [Yamamoto, T., et al., Bioorg. Med. Chem. Lett. 16, 798-802 (2006)] werden in 60 ml Ethanol gelöst und über Nacht unter Argon bei Rückflusstemperatur gerührt. Das ausgefallene Produkt wird danach abfiltriert, mit Ethanol und Diethylether gewaschen und im Hochvakuum getrocknet. Man erhält 2.30 g (35% d. Th.) der Titelverbindung in Form beigefarbener Kristalle.

LC-MS (Methode 7): Rt = 1.59 min; MS (EIpos): m/z = 418 [M+H]⁺.

### Beispiel 32A

### 2-Cyanoethyl 5-ethoxy-2-methyl-4-(2-methyl-4-oxo-4H-chromen-8-yl)-1,4-dihydro-1,6-naphthyridin-3-carboxylat

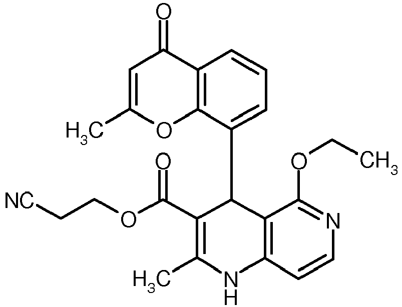

2.20 g (5.27 mmol) der Verbindung aus Beispiel 31A werden in 80 ml Orthoameisensäuretriethylester suspendiert und auf 130°C erhitzt. Danach wird das Reaktionsgemisch über einen Zeitraum von insgesamt 8 Stunden stündlich mit 5 Tropfen konzentrierter Schwefelsäure versetzt. Anschließend wird über Nacht bei gleicher Temperatur nachgerührt. Nach dem Abkühlen wird überschüssiger Orthoester am Rotationsverdampfer entfernt und das Rohprodukt mittels Säulenchromatographie aufgereinigt (Kieselgel; Laufmittel: zunächst Dichlormethan, dann Essigsäureethylester, zuletzt Essigsäureethylester/Methanol 20:1). Die Produktfraktionen werden bis auf ein Volumen von ca. 5 ml eingeengt. Das ausgefallene Produkt wird abfiltriert und nach dem Waschen mit Essigsäureethylester und Diethylether im Hochvakuum getrocknet. Man erhält 282 mg (12% d. Th.) der Titelverbindung in Form brauner Kristalle.

LC-MS (Methode 3): Rt = 1.96 min; MS (EIpos): m/z = 446 [M+H]⁺

¹H-NMR (300 MHz, DMSO-d₆): δ = 1.01 (t, 3H), 2.38 (s, 6H), 2.74 (m, 2H), 3.96-4.13 (m, 4H), 5.54 (s, 1H), 6.19 (s, 1H), 6.53 (d, 1H), 7.31 (t, 1H), 7.67 (dd, 1H), 7.76 (d, 1H), 7.80 (dd, 1H), 9.69 (s, 1H).

### Beispiel 33A

### 5-Ethoxy-2-methyl-4-(2-methyl-4-oxo-4H-chromen-8-yl)-1,4-dihydro-1,6-naphthyridin-3-carbonsäure

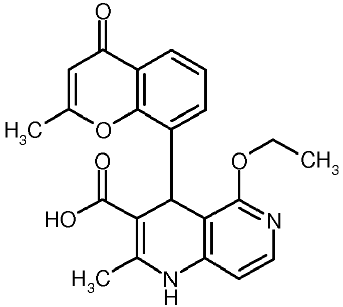

270 mg (0.61 mmol) der Verbindung aus Beispiel 32A werden in 15 ml 1,2-Dimethoxyethan/ Wasser (2:1 v/v) gelöst, mit 1.21 ml (1.21 mmol) 1 N Natronlauge versetzt und 1 h bei Raumtemperatur gerührt. Das Reaktionsgemisch wird danach mit 30 ml Diethylether versetzt. Die wässrige Phase wird abgetrennt und mit 1 N Salzsäure angesäuert. Das anfallende Präzipitat wird abfiltriert und mit Wasser und etwas Diethylether gewaschen. Nach dem Trocknen bei 40°C im Vakuum erhält man 167 mg (70% d. Th.) der Titelverbindung.

LC-MS (Methode 7): Rₜ = 2.01 min; MS (EIpos): m/z = 393 [M+H]⁺

¹H-NMR (300 MHz, DMSO-d₆): δ = 1.02 (t, 3H), 2.33 (s, 3H), 2.35 (s, 3H), 3.97 (m, 1H), 4.08 (m, 1H), 5.52 (s, 1H), 6.18 (s, 1H), 6.50 (d, 1H), 7.31 (t, 1H), 7.60 (dd, 1H), 7.74 (d, 1H), 7.79 (dd, 1H), 9.42 (s, 1H), 11.45 (br. s, 1H).

### Beispiel 34A

### 2-Cyanoethyl 4-[4-brom-2-(trifluormethoxy)phenyl]-2-methyl-5-oxo-1,4,5,6-tetrahydro-1,6-naphthyridin-3-carboxylat

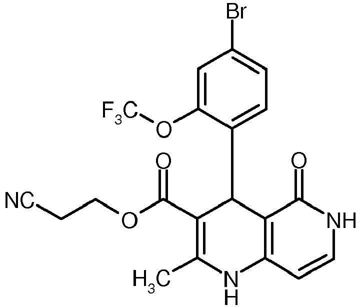

10.00 g (31.17 mmol) der Verbindung aus Beispiel 6A und 6.41 g (31.17 mmol) 2-Cyanoethyl-3-oxobutanoat [Yamamoto, T., et al., Bioorg. Med. Chem. Lett. 16, 798-802 (2006)] werden in 100 ml 2-Propanol vorgelegt, mit 4.09 g (37.17 mmol) 4-Aminopyridin-2(1*H*)-on [Searls, T., McLaughlin, L.W., Tetrahedron 55, 11985-11996 (1999)] versetzt und anschließend drei Tage bei Rückflusstemperatur gerührt. Nach dem Abkühlen wird das Lösungsmittel unter vermindertem Druck entfernt und das Rohprodukt mittels Säulenchromatographie aufgereinigt (Kieselgel; Laufmittels: Dichlormethan/Methanol 10:1). Man erhält 7.38 g (36% d. Th.) der Titelverbindung.

LC-MS (Methode 6): Rₜ = 2.84 min; MS (EIpos): m/z = 499 [M+H]⁺.

### Beispiel 35A

### 2-Cyanoethyl 4-[4-brom-2-(trifluormethoxy)phenyl]-5-ethoxy-2-methyl-1,4-dihydro-1,6-naphthyridin-3-carboxylat

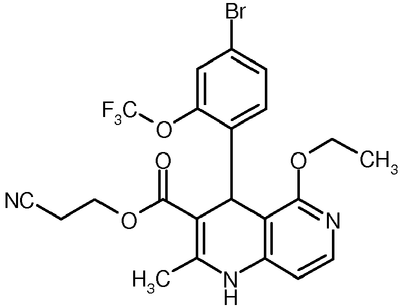

7.30 g (13.19 mmol) der Verbindung aus Beispiel 34A werden in 150 ml Orthoameisensäuretriethylester suspendiert und auf 130°C erhitzt. Danach wird das Reaktionsgemisch über einen Zeitraum von insgesamt 7 Stunden stündlich mit 15 Tropfen konzentrierter Schwefelsäure versetzt. Anschließend wird über Nacht bei gleicher Temperatur nachgerührt. Nach dem Abkühlen wird überschüssiger Orthoester am Rotationsverdampfer entfernt und das Rohprodukt mittels Säulenchromatographie aufgereinigt (Kieselgel; Laufmittel: Cyclohexan/Essigsäureethylester 1:1). Man erhält 4.59 g (64% d. Th.) der Titelverbindung.

LC-MS (Methode 3): Rt = 2.74 min; MS (EIpos): m/z = 527 [M+H]⁺

¹H-NMR (300 MHz, DMSO-d₆): δ = 1.17 (t, 3H), 2.33 (s, 3H), 2.81 (m, 2H), 3.99-4.21 (m, 4H), 5.29 (s, 1H), 6.50 (d, 1H), 7.31 (t, 1H), 7.37 (d, 1H), 7.44 (dd, 1H), 7.78 (d, 1H), 9.63 (s, 1H).

### Beispiel 36A

### 2-Cyanoethyl 4-[4-cyano-2-(trifluormethoxy)phenyl]-5-ethoxy-2-methyl-1,4-dihydro-1,6-naphthyridin-3-carboxylat

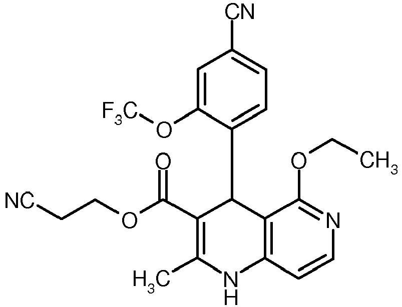

4.59 g (8.72 mmol) der Verbindung aus Beispiel 35A, 758 mg (6.45 mmol) Zinkcyanid und 504 mg (0.436 mmol) Tetrakis(triphenylphosphin)palladium(0) werden in 40 ml DMF gelöst und anschließend, verteilt auf drei Ansätze, in einer single mode-Mikrowelle (Emrys Opzimizer) für 5 min auf 220°C erhitzt. Die einzelnen Ansätze werden danach wieder vereinigt und das Lösungsmittel am Rotationsverdampfer entfernt. Das Rohprodukt wird in Essigsäureethylester aufgenommen und über Kieselgur filtriert. Die organische Phase wird mit Wasser (2 x) und mit gesättigter Natriumchlorid-Lösung gewaschen. Nach dem Abdestillieren des Lösungsmittels wird das Rohprodukt durch Säulenchromatographie aufgereinigt (Kieselgel; Laufmittel: Cyclohexan/Essigsäureethylester 7:3 → 1:1). Man erhält 1.40 g (31% d. Th.) der Titelverbindung.

LC-MS (Methode 3): Rt = 2.48 min; MS (EIpos): m/z = 473 [M+H]⁺

¹H-NMR (300 MHz, DMSO-d₆): δ = 1.10 (t, 3H), 2.34 (s, 3H), 2.80 (m, 2H), 4.00-4.21 (m, 4H), 5.36 (s, 1H), 6.51 (d, 1H), 7.60 (d, 1H), 7.66-7.74 (2H), 7.79 (d, 1H), 9.70 (s, 1H).

### Beispiel 37A

### 4-[4-Cyano-2-(trifluormethoxy)phenyl]-5-ethoxy-2-methyl-1,4-dihydro-1,6-naphthyridin-3-carbonsäure

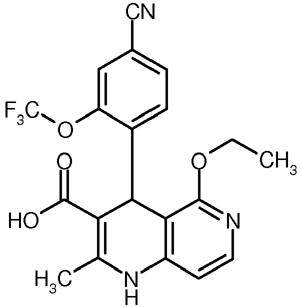

1400 mg (2.96 mmol) der Verbindung aus Beispiel 36A werden in 35 ml 1,2-Dimethoxyethan/ Wasser (2.5:1 v/v) gelöst, mit 5.93 ml (5.93 mmol) 1 N Natronlauge versetzt und 2 h bei Raumtemperatur gerührt. Das Reaktionsgemisch wird dann mit 50 ml Diethylether und 50 ml Wasser versetzt. Die wässrige Phase wird abgetrennt und mit 1 N Salzsäure auf einen pH-Wert von 4-5 eingestellt. Die entstandene Suspension wird anschließend 1 h gerührt. Das entstandene Präzipitat wird abfiltriert und mit Wasser und etwas Diethylether gewaschen. Nach dem Trocknen im Vakuum erhält man 850 mg (68% d. Th.) der Titelverbindung.

LC-MS (Methode 3): Rt = 2.19 min; MS (EIpos): m/z = 420 [M+H]⁺

¹H-NMR (300 MHz, DMSO-d₆): δ = 1.11 (t, 3H), 2.31 (s, 3H), 4.06 (m, 1H), 4.13 (m, 1H), 5.37 (s, 1H), 6.49 (d, 1H), 7.51 (d, 1H), 7.65-7.72 (m, 2H), 7.76 (d, 1H), 9.42 (s, 1H), 11.62 (s, 1H).

### Beispiel 38A

### 4-(4-Cyano-2-methoxyphenyl)-5-ethoxy-2-(trifluormethyl)-1,4-dihydro-1,6-naphthyridin-3-carbonsäure

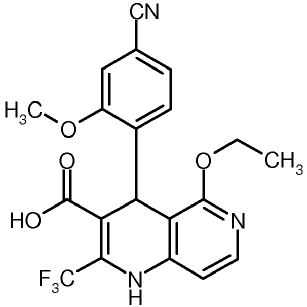

Die Titelverbindung kann ausgehend von stöchiometrischen Mengen 4-Formyl-3-methoxybenzonitril (Beispiel 10A), 4-Aminopyridin-2(1*H*)-on [Searls, T., McLaughlin, L.W., Tetrahedron 55, 11985-11996 (1999)] und Allyl 4,4,4-trifluor-3-oxobutanoat [Moseley, J.D., Tetrahedron Lett. 46, 3179-3181 (2005)] erhalten werden. Dabei wird zunächst die Dihydropyridinsynthese in Ethanol ohne Zusatz von Additiven über Nacht bei Rückflusstemperatur durchgeführt. Das initial anfallende Zwischenprodukt Allyl 4-(4-cyano-2-methoxyphenyl)-2-hydroxy-5-oxo-2-(trifluormethyl)-1,2,3,4,5,6-hexahydro-l,6-naphthyridin-3-carboxylat kann dann in Anlehnung an ein Literaturverfahren mit Essigsäure dehydratisiert werden [vgl. Moseley, J.D., Tetrahedron Lett. 46, 3179-3181 (2005)]. Anschließend ist analog zur Synthese von Beispiel 29A Allyl4-(4-cyano-2-methoxyphenyl)-5-ethoxy-2-(trifluormethyl)-1,4-dihydro-1,6-naphthyridin-3-carboxylat durch Umsetzung mit Triethylorthoformiat erhältlich. Abschließende Allylester-Spaltung mittels Wilkinson's Katalysator [Tris(triphenylphosphin)rhodium(I)chlorid] in Essigsäure liefert die Titelverbindung [vgl. Moseley, J.D., Tetrahedron Lett. 46, 3179-3181 (2005)].

LC-MS (Methode 7): Rt = 2.98 min; MS (EIpos): m/z = 420 [M+H]⁺

¹H-NMR (300 MHz, DMSO-d₆): δ = 1.09 (t, 3H), 3.77 (s, 3H), 3.98-4.16 (m, 2H), 5.37 (s, 1H), 6.73 (d, 1H), 7.19 (d, 1H), 7.34 (dd, 1H), 7.42 (d, 1H), 7.78 (d, 1H), 9.62 (s, 1H).

### Beispiel 39A

### 2-Cyanoethyl 2,8-dimethyl-4-(2-methyl-4-oxo-4H-chromen-8-yl)-5-oxo-1,4,5,6-tetrahydro-1,6-naphthyridin-3-carboxylat

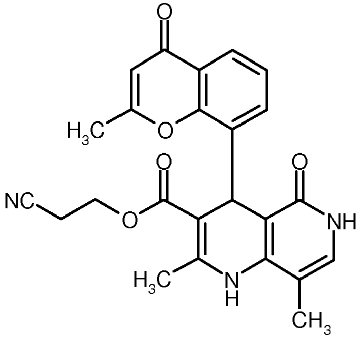

1.50 g (7.97 mmol) der Verbindung aus Beispiel 5A, 1.86 g (9.57 mmol) 2-Cyanoethyl-3-oxobutanoat [Yamamoto, T., et al., Bioorg. Med. Chem. Lett. 16, 798-802 (2006)], 91 µl (1.59 mmol) Essigsäure sowie 158 µl (1.59 mmol) Piperidin werden in 30 ml wasserfreiem Dichlormethan gelöst und über Nacht unter Rückfluss am Wasserabscheider gerührt. Danach wird mit Wasser gewaschen, die organische Phase mit Magnesiumsulfat getrocknet und die flüchtigen Komponenten am Rotationsverdampfer entfernt. 2.91 g (ca. 7.33 mmol) des so gewonnenen Rohprodukts 2-Cyanoethyl-(2*Z*)-2-[(2-methyl-4-oxo-4*H*-chromen-8-yl)methyliden]-3-oxobutanoat werden mit 0.990 g (9.00 mmol) 4-Amino-5-methylpyridin-2(1*H*)-on [Bisagni, E., Hung, N.C., *Synthesis,* 765-766 (1984)] versetzt, in 40 ml 2-Propanol aufgenommen und über Nacht bei Rückflusstemperatur gerührt. Nach dem Abkühlen wird das angefallene Präzipitat abfiltriert und mit wenig Diethylether gewaschen. Nach dem Trocknen im Hochvakuum werden 1.20 g (38% d. Th.) der Titelverbindung erhalten.

LC-MS (Methode 8): Rt = 1.00 min; MS (EIpos): m/z = 432 [M+H]⁺

¹H-NMR (300 MHz, DMSO-d₆): δ = 2.06 (s, 3H), 2.34 (s, 3H), 2.44 (s, 3H), 2.77 (m, 2H), 3.98-4.14 (m, 2H), 5.76 (s, 1H), 6.15 (s, 1H), 6.98 (s, 1H), 7.28 (t, 1H), 7.71 (dd, 1H), 7.78 (dd, 1H), 8.29 (s, 1H), 10.78 (br. s, 1H).

### Beispiel 40A

### 2-Cyanoethyl 5-ethoxy-2,8-dimethyl-4-(2-methyl-4-oxo-4H-chromen-8-yl)-1,4-dihydro-1,6-naphthyridin-3-carboxylat

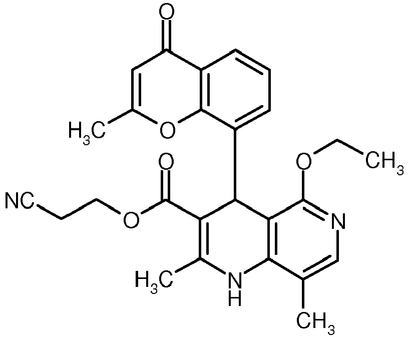

1.20 g (2.78 mmol) der Verbindung aus Beispiel 39A und 9.25 ml (55.6 mmol) Orthoameisensäuretriethylester werden in 30 ml trockenem DMF aufgenommen, auf 130°C erhitzt und mit 5 Tropfen konzentrierter Schwefelsäure versetzt. Nach 2 h zeigt eine HPLC-Kontrolle kompletten Umsatz. Nach dem Abkühlen werden die flüchtigen Komponenten am Rotationsverdampfer entfernt und das Rohprodukt mittels MPLC aufgereinigt (Biotage-Kartusche 40 M, Eluent: Isohexan/ Ethylacetat 1:2). Man erhält 640 mg (50% d. Th.) der Titelverbindung.

LC-MS (Methode 9): Rt = 0.99 min; MS (EIpos): m/z = 460 [M+H]⁺

¹H-NMR (300 MHz, DMSO-d₆): δ = 1.01 (t, 3H), 2.19 (s, 3H), 2.38 (s, 3H), 2.48 (s, 3H), 2.75 (m, 2H), 3.93-4.14 (m, 4H), 5.55 (s, 1H), 6.18 (s, 1H), 7.30 (t, 1H), 7.63 (s, 1H), 7.67 (dd, 1H), 7.79 (dd, 1H), 8.49 (s, 1H).

### Beispiel 41A

### 5-Ethoxy-2,8-dimethyl-4-(2-methyl-4-oxo-4H-chromen-8-yl)-1,4-dihydro-1,6-naphthyridin-3-carbonsäure

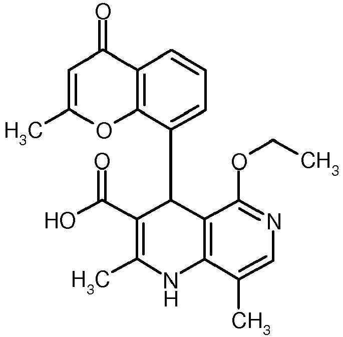

640 mg (1.39 mmol) der Verbindung aus Beispiel 40A werden in 30 ml 1,2-Dimethoxyethan/ Wasser (2:1 v/v) gelöst, mit 2.76 ml (2.76 mmol) 1 N Natronlauge versetzt und 30 min bei Raumtemperatur gerührt. Das Reaktionsgemisch wird anschließend mit 20 ml Diethylether versetzt. Die wässrige Phase wird abgetrennt, mit 1 N Salzsäure auf einen pH-Wert von 4-5 eingestellt und dreimal mit Essigsäureethylester extrahiert. Die organischen Phasen werden vereinigt und mit Magnesiumsulfat getrocknet. Die flüchtigen Komponenten werden am Rotationsverdampfer abgetrennt. Nach dem Trocknen im Vakuum erhält man 335 mg (56% d. Th.) der Titelverbindung in 94%-iger Reinheit (LC-MS).

LC-MS (Methode 8): Rt = 1.21 min; MS (EIpos): m/z = 407 [M+H]⁺

¹H-NMR (300 MHz, DMSO-d₆): δ = 1.01 (t, 3H), 2.18 (s, 3H), 2.35 (s, 3H), 2.42 (s, 3H), 3.90-4.10 (m, 2H), 5.54 (s, 1H), 6.18 (s, 1H), 7.31 (t, 1H), 7.58 (dd, 1H), 7.60 (s, 1H), 7.78 (dd, 1H), 8.25 (s, 1H), 11.52 (br. s, 1H).

### Ausführungsbeispiele:

### Beispiel 1

### 4-(4-Cyano-2-methoxyphenyl)-5-ethoxy-2-methyl-1,4-dihydro-1,6-naphthyridin-3-carboxamid

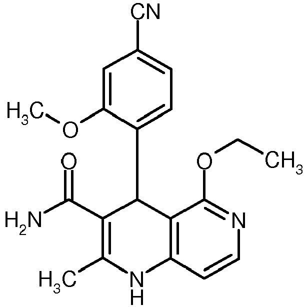

100 mg (ca. 0.24 mmol) der Verbindung aus Beispiel 23A werden in 3 ml DMF vorgelegt. Anschließend wird mit 2.94 mg (0.024 mmol) 4-*N,N*-Dimethylaminopyridin und 340 µl Ammoniak (28 Gew.-%-ige Lösung in Wasser, 2.41 mmol) versetzt und 3 h bei 100°C temperiert. Nach dem Abkühlen wird das Rohprodukt direkt mittels präparativer HPLC aufgereinigt (Eluent: Acetonitril/ Wasser mit 0.1% Ameisensäure, Gradient 20:80 → 95:5). Es werden 32 mg (37% d. Th.) der Titelverbindung erhalten.

LC-MS (Methode 3): Rt = 1.57 min; MS (EIpos): m/z = 365 [M+H]⁺

¹H-NMR (300 MHz, DMSO-d₆): δ = 1.07 (t, 3H), 2.13 (s, 3H), 3.83 (s, 3H), 4.04 (m, 2H), 5.36 (s, 1H), 6.42 (d, 1H), 6.66 (br. s, 2H), 7.18 (d, 1H), 7.29 (dd, 1H), 7.38 (d, 1H), 7.67 (d, 1H), 8.80 (s, 1H).

### Beispiel 2

### 4-(4-Cyano-2-methoxyphenyl)-5-ethoxy-2,7-dimethyl-1,4-dihydro-1,6-naphthyridin-3-carboxamid

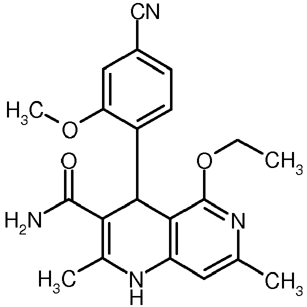

640 mg (1.69 mmol) der Verbindung aus Beispiel 27A werden in 30 ml Essigsäureethylester vorgelegt, mit 342 mg (2.11 mmol) 1,1'-Carbonyldiimidazol versetzt und anschließend über Nacht bei Raumtemperatur gerührt. Eine DC-Kontrolle (Kieselgel; Laufmittel: Cyclohexan/Essigsäureethylester 1:1 oder Dichlormethan/Methanol 9:1) zeigt vollständigen Umsatz. Die flüchtigen Komponenten werden am Rotationsverdampfer entfernt und der Rückstand in 20 ml DMF aufgenommen. Anschließend werden 2.36 ml Ammoniak (28 Gew.-%-ige Lösung in Wasser, 16.87 mmol) hinzugefügt und das Reaktionsgemisch 8 h bei 50°C temperiert. Das Lösungsmittel wird unter vermindertem Druck abdestilliert und der Rückstand mittels präparativer HPLC aufgereinigt (Eluent: Acetonitril/Wasser mit 0.1% Ameisensäure, Gradient 20:80 → 95:5). Man erhält 368 mg (58% d. Th.) der Titelverbindung.

LC-MS (Methode 7): Rt = 1.91 min; MS (EIpos): m/z = 379 [M+H]⁺

¹H-NMR (300 MHz, DMSO-d₆): δ = 1.05 (t, 3H), 2.13 (s, 3H), 2.19 (s, 3H), 3.84 (s, 3H), 4.02 (q, 2H), 5.32 (s, 1H), 6.25 (s, 1H), 6.62 (br. s, 2H), 7.16 (d, 1H), 7.28 (dd, 1H), 7.37 (d, 1H), 8.71 (s, 1H).

### Beispiel 3

### ent-4-(4-Cyano-2-methoxyphenyl)-5-ethoxy-2,7-dimethyl-1,4-dihydro-1,6-naphthyridin-3-carboxamid [(-)-Enantiomer und (+)-Enantiomer]

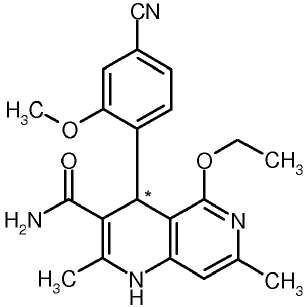

Das Racemat aus Beispiel 2 kann in präparativem Maßstab durch HPLC an chiraler Phase in seine Enantiomere aufgetrennt werden [Säule: Chiralpak IA, 250 mm x 20 mm; Elutionsmittel: Methyl-*tert*.-butylether/Methanol 85:15 (v/v); Fluss: 15 ml/min; Temperatur: 30°C; UV-Detektion: 220 nm].

### (-)-Enantiomer:

HPLC: Rt = 5.28 min, ee >98% [Säule: Chiralpak IA, 250 mm x 4.6 mm; Elutionsmittel: Methyl-*tert*.-butylether/Methanol 80:20 (v/v); Fluss: 1 ml/min; Temperatur: 25°C; UV-Detektion: 220 nm] ;

spezifischer Drehwert (Chloroform, 589 nm, 19.8°C, c = 0.50500 g /100 ml): -239.3°.

Eine Einkristall-Röntgenstrukturanalyse ergab für dieses Enantiomer eine *S*-Konfiguration am C*-Atom.

### (+)-Enantiomer:

HPLC: Rt = 4.50 min, ee >99 % [Säule: Chiralpak IA, 250 mm x 4.6 mm; Elutionsmittel: Methyl-*tert*.-butylether/Methanol 80:20 (v/v); Fluss: 1 ml/min; Temperatur: 25°C; UV-Detektion: 220 nm] ;
spezifischer Drehwert (Chloroform, 589 nm, 20°C, c = 0.51000 g / 100 ml): +222.7°.

### Beispiel 4

### 4-(4-Cyano-2-methoxyphenyl)-5-ethoxy-2,8-dimethyl-1,4-dihydro-1,6-naphthyridin-3-carboxamid

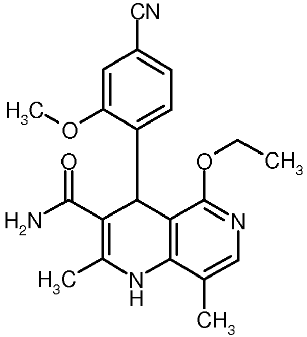

1.46 g (3.84 mmol) der Verbindung aus Beispiel 30A werden in 50 ml Essigsäureethylester vorgelegt, mit 777 mg (4.79 mmol) 1,1'-Carbonyldiimidazol versetzt und anschließend über Nacht bei Raumtemperatur gerührt. Eine DC-Kontrolle (Kieselgel; Laufmittel: Essigsäureethylester) zeigt vollständigen Umsatz. Die flüchtigen Komponenten werden am Rotationsverdampfer entfernt und der Rückstand in 20 ml DMF aufgenommen. Anschließend werden 10.74 ml Ammoniak (28 Gew.-%-ige Lösung in Wasser, 76.8 mmol) hinzugefügt und das Reaktionsgemisch 30 min bei 100°C temperiert. Das Lösungsmittel wird unter vermindertem Druck abdestilliert und der Rückstand mittels präparativer HPLC aufgereinigt (Eluent: Acetonitril/Wasser mit 0.1% Ameisensäure, Gradient 20:80 → 95:5). Nach Einengen der Produktfraktionen wird der Rückstand in 40 ml Dichlormethan/Methanol (1:1 v/v) gelöst und mit 100 ml Essigsäureethylester versetzt. Das Lösungsmittel wird auf ein Volumen von ca. 20 ml eingeengt, wobei das Produkt kristallisiert. Das Präzipitat wird abfiltriert und mit wenig Diethylether gewaschen. Nach dem Trocknen bei 40°C im Vakuumtrockenschrank erhält man 1.40 g (96% d. Th.) der Titelverbindung.

LC-MS (Methode 3): Rₜ = 1.64 min; MS (EIpos): m/z = 379 [M+H]⁺

¹H-NMR (300 MHz, DMSO-d₆): δ = 1.05 (t, 3H), 2.12 (s, 3H), 2.18 (s, 3H), 3.82 (s, 3H), 3.99-4.07 (m, 2H), 5.37 (s, 1H), 6.60-6.84 (m, 2H), 7.14 (d, 1H), 7.28 (dd, 1H), 7.37 (d, 1H), 7.55 (s, 1H), 7.69 (s, 1H).

### Beispiel 5

### ent-4-(4-Cyano-2-methoxyphenyl)-5-ethoxy-2,8-dimethyl-1,4-dihydro-1,6-naphthyridin-3-carboxamid [(-)-Enantiomer und (+)-Enantiomer]

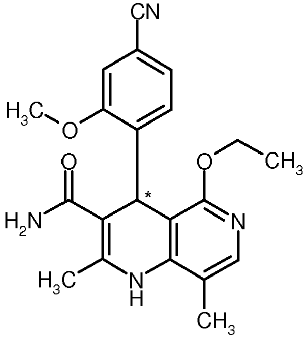

Das Racemat aus Beispiel 4 kann in präparativem Maßstab durch HPLC an chiraler Phase in seine Enantiomere aufgetrennt werden [Säule: 680 mm x 40 mm; Kieselgel-Phase basierend auf dem chiralen Selektor Poly(*N*-methacryloyl-D-leucin-dicyclopropylmethylamid; Eluent: Essigsäureethylester; Temperatur: 24°C; Fluss: 80 ml/min; UV-Detektion: 260 nm].

### (-)-Enantiomer:

HPLC: Rt = 2.48 min, ee = 99.6% [Säule: 250 mm x 4.6 mm; Kieselgel-Phase basierend auf dem chiralen Selektor Poly(*N*-methacryloyl-D-leucin-dicyclopropyleethylamid; Eluent: Essigsäureethylester; Temperatur: 24°C; Fluss: 2 ml/min; UV-Detektion: 260 nm];
spezifischer Drehwert (Chloroform, 589 nm, 19.7°C, c = 0.38600 g / 100 ml): -148.8°.

Eine Einkristall-Röntgenstrukturanalyse ergab für dieses Enantiomer eine *S*-Konfiguration am C*-Atom.

### (+)-Enantiomer:

HPLC: Rt = 4.04 min, ee = 99.3% [Säule: 250 mm x 4.6 mm; Kieselgel-Phase basierend auf dem chiralen Selektor Poly(*N*-methacryloyl-D-leucin-dicyclopropylmethylamid; Eluent: Essigsäureethylester; Temperatur: 24°C; Fluss: 2 ml/min; UV-Detektion: 260 nm];
spezifischer Drehwert (Chloroform, 589 nm, 19.8°C, c = 0.36300 g / 100 ml): +153.0°.

### Beispiel 6

### 5-Ethoxy-2-methyl-4-(2-methyl-4-oxo-4H-chromen-8-yl)-1,4-dihydro-1,6-naphthyridin-3-carboxamid

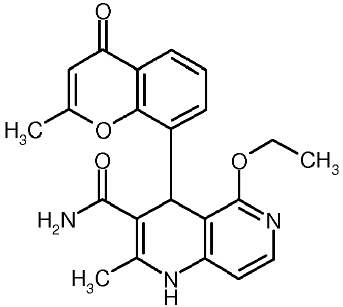

155 mg (0.395 mmol) der Verbindung aus Beispiel 31A werden in 10 ml THF vorgelegt, mit 80.1 mg (0.494 mmol) 1,1'-Carbonyldiimidazol versetzt und anschließend über Nacht bei Raumtemperatur gerührt. Eine DC-Kontrolle (Kieselgel; Laufmittel: Essigsäureethylester oder Dichlormethan/ Methanol 9:1) zeigt vollständigen Umsatz. Die flüchtigen Komponenten werden am Rotationsverdampfer entfernt und der Rückstand in 3 ml DMF aufgenommen. Anschließend werden 553 mg Ammoniak (28 Gew.-%-ige Lösung in Wasser, 3.95 mmol) hinzugefügt und das Reaktionsgemisch 10 min bei 100°C temperiert. Das Lösungsmittel wird unter vermindertem Druck entfernt und der Rückstand mittels präparativer HPLC aufgereinigt (Eluent: Acetonitril/Wasser mit 0.1% Ameisensäure, Gradient 20:80 → 95:5). Man erhält 30 mg (19% d. Th.) der Titelverbindung.

LC-MS (Methode 6): Rt = 1.17 min; MS (EIpos): m/z = 392 [M+H]⁺

¹H-NMR (300 MHz, DMSO-d₆): δ = 0.96 (t, 3H), 2.09 (s, 3H), 2.36 (s, 3H), 3.94 (m, 1H), 4.03 (m, 1H), 5.59 (s, 1H), 6.19 (s, 1H), 6.42 (d, 1H), 6.66 (br. s, 1H), 7.00 (br. s, 1H), 7.31 (t, 1H), 7.53 (dd, 1H), 7.68 (d, 1H), 7.79 (dd, 1H), 8.83 (s, 1H).

### Beispiel 7

### 4-[4-Cyano-2-(trifluormethoxy)phenyl]-5-ethoxy-2-methyl-1,4-dihydro-1,6-naphthyridin-3-carboxamid

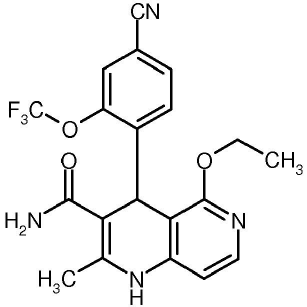

200 mg (0.477 mmol) der Verbindung aus Beispiel 37A werden in 5 ml Essigsäureethylester vorgelegt, mit 96.7 mg (0.596 mmol) 1,1'-Carbonyldiimidazol versetzt und anschließend über Nacht bei Raumtemperatur gerührt (DC-Kontrolle: unzureichende Umsetzung). Danach wird 1 ml DMF zugesetzt und eine weitere Nacht bei Raumtemperatur gerührt (DC-Kontrolle: vollständiger Umsatz). Die flüchtigen Komponenten werden am Rotationsverdampfer entfernt und der Rückstand in 4 ml DMF aufgenommen. Anschließend werden 663 µl Ammoniak (28 Gew.-%-ige Lösung in Wasser, 4.77 mmol) hinzugefügt und das Reaktionsgemisch über Nacht in einem geschlossenen Gefäß bei 100°C temperiert. Nach dem Abkühlen wird das Lösungsmittel unter vermindertem Druck entfernt und der Rückstand mittels präparativer HPLC aufgereinigt (Eluent: Acetonitril/ Wasser mit 0.1% Ameisensäure, Gradient 20:80 → 95:5). Man erhält 140 mg (70% d. Th.) der Titelverbindung.

LC-MS (Methode 6): Rt = 2.26 min; MS (EIpos): m/z = 419 [M+H]⁺

¹H-NMR (300 MHz, DMSO-d₆): δ = 1.04 (t, 3H), 2.04 (s, 3H), 4.06 (m, 2H), 5.42 (s, 1H), 6.41 (d, 1H), 6.80 (br. s, 1H), 6.97 (br. s, 1H), 7.45 (d, 1H), 7.68-7.74 (m, 3H), 8.82 (d, 1H).

### Beispiel 8

### 4-(4-Cyano-2-methoxyphenyl)-5-ethoxy-2-(trifluormethyl)-1,4-dihydro-1,6-naphthyridin-3-carboxamid

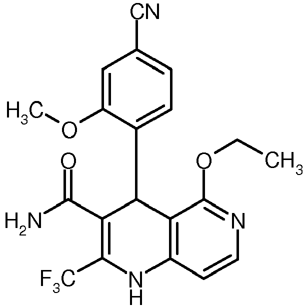

180 mg (0.429 mmol) der Verbindung aus Beispiel 38A werden in 5 ml Essigsäureethylester vorgelegt, mit 87.0 mg (0.537 mmol) 1,1'-Carbonyldiimidazol versetzt und anschließend zwei Stunden bei Raumtemperatur gerührt. Per DC-Kontrolle wird kompletter Umsatz festgestellt. Die flüchtigen Komponenten werden am Rotationsverdampfer entfernt und der Rückstand in 4 ml DMF aufgenommen. Anschließend werden 597 µl Ammoniak (28 Gew.-%-ige Lösung in Wasser, 4.29 mmol) hinzugefügt und das Reaktionsgemisch drei Stunden in einem geschlossenen Gefäß bei 100°C temperiert. Nach dem Abkühlen wird das Lösungsmittel unter vermindertem Druck entfernt und der Rückstand mittels präparativer HPLC aufgereinigt (Eluent: Acetonitril/Wasser mit 0.1 % Ameisensäure, Gradient 20:80 → 95:5). Man erhält 10 mg (5% d. Th.) der Titelverbindung.

LC-MS (Methode 3): Rt = 1.85 min; MS (EIpos): m/z = 419 [M+H]⁺

¹H-NMR (300 MHz, DMSO-d₆): δ = 1.03 (t, 3H), 3.79 (s, 3H), 3.96-4.11 (m, 2H), 5.37 (s, 1H), 6.62 (d, 1H), 7.08-7.14 (m, 2H), 7.32 (dd, 1H), 7.37-7.46 (m, 2H), 7.73 (d, 1H), 9.18 (s, 1H).

### Beispiel 9

### 5-Ethoxy-2,8-dimethyl-4-(2-methyl-4-oxo-4H-chromen-8-yl)-1,4-dihydro-1,6-naphthyridin-3-carboxamid

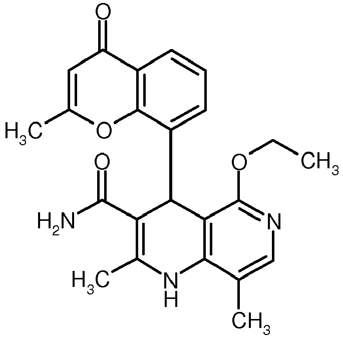

335.0 mg (0.824 mmol) der Verbindung aus Beispiel 41A werden in 10 ml Essigsäureethylester vorgelegt und mit 167.1 mg (0.537 mmol) 1,1'-Carbonyldiimidazol versetzt. Anschließend wird die Suspension über Nacht bei Raumtemperatur gerührt. Da keine klare Lösung entsteht, werden 2 ml DMF zugesetzt und die Mischung zwei weitere Stunden bei Raumtemperatur gerührt. Per DC-Kontrolle wird dann kompletter Umsatz festgestellt. Die flüchtigen Komponenten werden am Rotationsverdampfer entfernt und der Rückstand in 4 ml DMF aufgenommen. Anschließend werden 2.293 ml Ammoniak (28 Gew.-%-ige Lösung in Wasser, 16.5 mmol) und 10.1 mg 4-*N*,*N*-Di-methylaminopyridin hinzugefügt. Das Reaktionsgemisch wird dreißig Minuten in einem geschlossenen Gefäß bei 100°C temperiert. Nach dem Abkühlen wird das Lösungsmittel unter vermindertem Druck entfernt und der Rückstand mittels präparativer HPLC aufgereinigt (Eluent: Acetonitril/ Wasser mit 0.1% Ameisensäure, Gradient 20:80 → 95:5). Die Produktfraktionen werden eingeengt und der Rückstand in wenig Dichlormethan aufgenommen und mit Diisopropylether bis zur Trübung versetzt. Der ausfallende Feststoff wird isoliert und im Vakuum getrocknet. Man erhält 207 mg (59% d. Th.) der Titelverbindung.

LC-MS (Methode 9): Rt = 0.67 min; MS (EIpos): m/z = 406 [M+H]⁺

¹H-NMR (300 MHz, DMSO-d₆): δ = 0.94 (t, 3H), 2.13 (s, 3H), 2.14 (s, 3H), 2.34 (s, 3H), 3.90 (m, 1H), 4.00 (m, 1H), 5.58 (s, 1H), 6.18 (s, 1H), 6.70 (br. s, 1H), 7.06 (br. s, 1H), 7.30 (t, 1H), 7.50 (dd, 1H), 7.56 (s, 1H), 7.71 (s, 1H), 7.78 (dd, 1H).

### B. Bewertung der pharmakologischen Wirksamkeit

### Abkürzungen:

- DMEM: Dulbecco's Modified Eagle Medium
- DNA: Desoxyribo Nucleic Acid
- FCS: Fetal Calf Serum
- HEPES: 4-(2-Hydroxyethyl)-1-piperazin-ethansulfonsäure
- PCR: Polymerase Chain Reaction
- Tris: Tris-(hydroxymethyl)-methylamin

Die vorteilhaften pharmakologischen Eigenschaften der erfindungsgemäßen Verbindungen können in folgenden Assays gezeigt werden:

### 1. Zellulärer in vitro-Test zur Bestimmung der inhibitorischen MR-Aktivität und MR-Selektivität gegenüber anderen Steroidhormon-Rezentoren

Die Identifizierung von Antagonisten des humanen Mineralokorticoid-Rezeptors (MR) sowie die Quantifizierung der Wirksamkeit der hier beschriebenen Verbindungen erfolgt mit Hilfe einer rekombinanten Zelllinie. Die Zelle leitet sich ursprünglich von einer Ovarepithelzelle des Hamsters ab (Chinese Hamster Ovary, CHO K1, ATCC: American Type Culture Collection, VA 20108, USA).

In dieser CHO K1-Zelllinie wird ein etabliertes Chimärensystem verwendet, in dem die Liganden-Bindungsdomänen humaner Steroidhormon-Rezeptoren an die DNA-Bindungsdomäne des Hefe-Transkriptionsfaktors GAL4 fusioniert werden. Die so entstehenden GAL4-Steroidhormonrezeptor-Chimären werden in den CHO-Zellen mit einem Reporterkonstrukt co-transfiziert und stabil exprimiert.

### Klonierungen:

Zur Generierung der GAL4-Steroidhormonrezeptor-Chimären wird die GAL4-DNA-Bindungsdomäne (Aminosäuren 1-147) aus dem Vektor pFC2-dbd (Fa. Stratagene) mit den PCR-amplifizierten Liganden-Bindungsdomänen des Mineralokorticoid-Rezeptors (MR, Aminosäuren 734-985), des Glucokorticoid-Rezeptors (GR, Aminosäuren 443-777), des Progesteron-Rezeptors (PR, Aminosäuren 680-933) und des Androgen-Rezeptors (AR, Aminosäuren 667-919) in den Vektor pIRES2 (Fa. Clontech) kloniert. Das Reporterkonstrukt, welches fünf Kopien der GAL4-Bindestelle, vorgeschaltet vor einem Thymidinkinase-Promotor enthält, führt zur Expression der Firefly-Luciferase (*Photinus pyralis*) nach Aktivierung und Bindung der GAL4-Steroidhormonrezeptor-Chimären durch die jeweiligen spezifischen Agonisten Aldosteron (MR), Dexamethason (GR), Progesteron (PR) und Dihydrotestosteron (AR).

### Testablauf:

Die MR-, GR-, PR- und AR-Zellen werden am Tag vor dem Test in Medium (Optimem, 2.5% FCS, 2 mM Glutamin, 10 mM HEPES) in 96- (oder 384- bzw. 1536-) Loch-Mikrotiterplatten ausplattiert und in einem Zellinkubator (96% Luftfeuchtigkeit, 5% v/v CO₂, 37°C) gehalten. Am Testtag werden die zu prüfenden Substanzen in oben genanntem Medium aufgenommen und zu den Zellen hinzugegeben. Etwa 10 bis 30 Minuten nach Zugabe der Testsubstanzen werden die jeweiligen spezifischen Agonisten der Steroidhormon-Rezeptoren hinzugesetzt. Nach einer weiteren Inkubationszeit von 5 bis 6 Stunden wird die Luciferaseaktivität mit Hilfe einer Videokamera gemessen. Die gemessenen relativen Lichteinheiten ergeben in Abhängigkeit von der Substanzkonzentration eine sigmoide Stimulationskurve. Die Berechnung der IC₅₀-Werte erfolgt mit Hilfe des Computerprogramms GraphPad PRISM (Version 3.02).

Tabelle A zeigt die IC₅₀-Werte (MR) repräsentativer Beispielverbindungen:

**Tabelle A**

| **Beispiel**-**Nr**. | **MR IC₅₀ [nM]** |
|---|---|
| 1 | 35 |
| 4 | 23 |
| 5 [(-)-Enantiomer] | 16 |

### 2. In vitro-Test zur Bestimmung möglicher Bindungsaktivität am L-Typ Calcium-Kanal

Membranpräparationen des zerebralen Cortex von Wistar-Ratten dienen als Ausgangsmaterial für einen radioaktiven Bindungstest, der als Standardassay in der Literatur ausführlich beschrieben ist [Ehlert, F.J., Roeske, W.R., Itoga E., Yamamura, H.I., Life Sci. 30, 2191-2202 (1982); Gould, R.J., Murphy, K.M.M., Snyder, S.H., Proc. Natl. Acad. Sci. U.S.A. 79, 3656-3660] und von kommerziellen Anbietern (z.B. Fa. MDS Pharma Services) im Rahmen von Auftragsuntersuchungen verwendet wird. In diesem Bindungsassay werden Verdünnungsreihen der Testverbindungen in DMSO typischerweise für 90 Minuten bei 25°C in einem 50 mM TrisHCl-Puffer, pH 7.7, mit den Membranpräparationen und dem Tritium-markierten Liganden Nitrendipin (0.1 nM) inkubiert und die spezifische Bindung der Testverbindungen über Quantifizierung des spezifisch verdrängten, radioaktiv markierten Liganden bestimmt. IC₅₀-Werte werden über eine nicht-lineare Regressionsanalyse ermittelt.

In diesem L-Typ Calcium-Kanal-Bindungsassay wird für einen klassischen Calcium-Antagonisten vom Dihydropyridin-Typ, wie z.B. Nitrendipin, ein IC₅₀-Wert von 0.3 nM bestimmt, während untersuchte Beispiele der hier beschriebenen erfindungsgemäßen Verbindungen IC₅₀-Werte von > 1 µM und somit eine mindestens um den Faktor 3000 verminderte Affinität zum L-Typ Calcium-Kanal aufweisen. Verbindungen mit einer solchen geringen Residualbindungsaffinität zum L-Typ Calcium-Kanal zeigen *ein vivo* keine ausgeprägten hämodynamischen Effekte mehr, die über den L-Typ Calcium-Kanal vermittelt sind.

### 3. In vivo-Test zum Nachweis der kardiovaskulären Wirkung: Diurese-Untersuchungen an wachen Ratten in Stoffwechselkäfigen

Wistar-Ratten (250-350 g Körpergewicht) werden mit freiem Zugang zu Futter (Altromin) und Trinkwasser gehalten. Ab ca. 72 Stunden vor Versuchsbeginn erhalten die Tiere anstelle des normalen Futters ausschließlich Kochsalz-reduziertes Futter mit einem Gehalt von 0.02% Natriumchlorid (ssniff R/M-H, 10 mm mit 0.02% Na, S0602-E081, Fa. ssniff Spezialdiäten GmbH, D-59494 Soest). Während des Versuches werden die Tiere für ca. 24 Stunden einzeln in für Ratten dieser Gewichtsklasse geeigneten Stoffwechselkäfigen (Fa. Tecniplast Deutschland GmbH, D-82383 Hohenpeißenberg) mit freiem Zugang zu Kochsalz-reduziertem Futter und Trinkwasser gehalten. Am Versuchsbeginn wird den Tieren die zu prüfende Substanz in einem Volumen von 0.5 ml/kg Körpergewicht eines geeigneten Lösemittels mittels einer Schlundsonde in den Magen verabreicht. Als Kontrolle dienende Tiere erhalten nur Lösemittel. Kontrollen und Substanztestungen werden am selben Tag parallel durchgeführt. Kontrollgruppen und Substanz-Dosisgruppen bestehen aus jeweils 3 bis 6 Tieren. Während des Versuchs wird der von den Tieren ausgeschiedene Urin kontinuierlich in einem Auffangbehälter am Käfigboden gesammelt. Für jedes Tier wird gesondert das Urinvolumen pro Zeiteinheit bestimmt und die Konzentration der im Urin ausgeschiedenen Natrium- bzw. Kalium-Ionen mittels flammenphotometrischer Standardmethoden gemessen. Aus den Messwerten wird der Natrium/Kalium-Quotient als ein Maß für die Substanzwirkung berechnet. Die Messintervalle betragen typischerweise den Zeitraum bis zu 8 Stunden nach Versuchsbeginn (Tagintervall) und den Zeitraum von 8 bis 24 Stunden nach Versuchsbeginn (Nachtintervall). In einer abgewandelten Versuchsanordnung wird der Urin während des Tagintervalls im Abstand von zwei Stunden gesammelt und gemessen. Um eine hierfür ausreichende Urinmenge zu erhalten, wird den Tieren zu Versuchsbeginn und dann im Abstand von zwei Stunden per Schlundsonde eine definierte Menge Wasser zugeführt.

### 4. DOCA/Salz-Modell

Die Verabreichung von Desoxycorticosteronacetat (DOCA) in Kombination mit einer Hochsalzdiät und einseitiger Nierenentfernung induziert bei der Ratte einen Hypertonus, der durch relativ niedrige Reninspiegel charakterisiert ist. Als Folge dieser endokrinen Hypertonie (DOCA ist eine direkte Vorstufe von Aldosteron) kommt es in Abhängigkeit von der gewählten DOCA-Konzentration zu einer Hypertrophie des Herzens und weiteren Endorgan-Schäden, z.B. der Niere, die u.a. durch Proteinurie und Glomerulosklerose charakterisiert sind. In diesem Rattenmodell lassen sich somit Testsubstanzen auf vorhandene antihypertrophe und Endorgan-schützende Wirkung hin untersuchen.

Etwa 8 Wochen alte (Körpergewicht zwischen 250 und 300 Gramm), männliche Sprague Dawley (SD)-Ratten werden linksseitig uninephrektomiert. Dazu werden die Ratten mit 1.5-2%-igem Isofluran in einer Mischung aus 66% N₂O und 33% O₂ anästhesiert und die Niere über einen Flankenschnitt entfernt. Als spätere Kontrolltiere dienen sogenannte sham-operierte Tiere, denen keine Niere entfernt wird.

Uninephrektomierte SD-Ratten erhalten 1% Natriumchlorid im Trinkwasser und einmal wöchentlich eine subkutane Injektion von Desoxycorticosteronacetat (gelöst in Sesamöl; Fa. Sigma) zwischen die Schulterblätter gespritzt (Hochdosis: 100 mg/kg/Woche s.c.; Normaldosis: 30 mg/ kg/Woche s.c.).

Die Substanzen, die auf ihre protektive Wirkung *in vivo* untersucht werden sollen, werden per Gavage oder über das Futter (Fa. Ssniff) verabreicht. Die Tiere werden einen Tag vor Versuchsbeginn randomisiert und Gruppen mit gleicher Tierzahl, in der Regel n = 10, zugeordnet. Während des gesamten Versuchs steht den Tieren Trinkwasser und Futter *ad libitum* zur Verfügung. Die Substanzen werden einmal täglich 4-8 Wochen lang per Gavage oder per Futter verabreicht. Als Plazebogruppe dienen Tiere, die genauso behandelt werden, aber entweder nur das Lösungsmittel oder das Futter ohne Testsubstanz erhalten.

Die Wirkung der Testsubstanzen wird durch Messung hämodynamischer Parameter [Blutdruck, Herzfrequenz, Inotropie (dp/dt), Relaxationszeit (tau), maximaler linksventrikulärer Druck, linksventrikulärer enddiastolischer Druck (LVEDP)], Gewichtsbestimmung von Herz, Niere und Lunge, Messung der Proteinausscheidung sowie durch Messung der Genexpression von Biomarkern (z.B. ANP, Atrial Natriuretic Peptide, und BNP, Brain Natriuretic Peptide) mittels RT/TaqMan-PCR nach RNA-Isolation aus kardialem Gewebe bestimmt.

Die statistische Auswertung erfolgt mit Student's t-Test nach vorheriger Überprüfung der Varianzen auf Homogenität.

### 5. In vivo-Test zum Nachweis von anti-mineralokortikoider Wirksamkeit an narkotisierten Hunden

Männliche oder weibliche Mischlingshunde (Mongrels, Marshall BioResources, USA) mit einem Gewicht zwischen 20 und 30 Kilogramm werden mit Pentobarbital (30 mg/kg intravenös; Narcoren^{®}, Merial, Deutschland) narkotisiert. Alcuroniumchlorid (3 mg/Tier intravenös; Alloferin^{®}, ICN Pharmaceuticals, Deutschland) dient dabei zusätzlich als Muskelrelaxans. Die Hunde werden intubiert und mit einem Sauerstoff-Raumluft-Gemisch (40/60 Vol.-%) beatmet (etwa 5-6 Liter/min). Die Beatmung erfolgt mit einem Beatmungsgerät der Fa. Draeger (Sulla 808) und wird überwacht mit einem CO₂-Analysator (Fa. Engström). Die Narkose wird aufrecht erhalten durch eine ständige Infusion von Pentobarbital (50 µg/kg/min) oder Isofluran (1-2 Vol.-%). Als Analgetikum dient Fentanyl (10 µg/kg/h).

Primäres Versuchsziel ist die Untersuchung des Einflusses von Testverbindungen mit antimineralokortikoider Rezeptorwirksamkeit auf die Aldosteron-induzierte Natrium-Retention. Hierbei wird analog einer publizierten Methode vorgegangen [H.P. Ramjoe, U.M. Bucher, J. Richter und M. De Gasparo, Anti-mineralocorticoid activity of three nobel aldosterone antagonistes in the conscious dog and in man, in: Diuretics II: Chemistry, Pharmacology, and Clinical Applications, J.B. Puschett und A. Greenberg (Ed.), Elsevier Science Publishing Co., Inc., 1987]. Eine kontinuierliche Infusion von Aldosteron (0.6 µg/kg/h) führt nach 3 Stunden zu einer Abnahme des Natrium-Kalium-Verhältnisses im Urin (die Natrium- und Kalium-Bestimmung erfolgt flammenphotometrisch). Unter weiterer Aldosteron-Infusion wird die Testsubstanz intravenös, intraduodenal oder oral appliziert. Als Positivkontrolle dient Spironolakton, das das Natrium/Kalium-Verhältnis im Urin dosisabhängig erhöht.

Zur Sicherstellung einer konstanten Hämodynamik sowie zur Erfassung von funktionellen Herz-Kreislaufparametern wird der Hund hämodynamisch überwacht und folgendermaßen instrumentiert:
- Einführung eines Blasenkatheters zur Messung des Urinflusses und der Urinzusammensetzung;
- Anbringung von EKG-Ableitungen an den Extremitäten zur EKG-Messung;
- Einführung eines mit Kochsalz-Lösung gefüllten Fluidmedic PE 300-Schlauches in die *A*. *femoralis,* der mit einem Drucksensor (Fa. Braun, Melsungen, Deutschland) zur Messung des systemischen Blutdrucks verbunden ist;
- Einführung eines Millar Tip-Katheters (Typ 350 PC, Millar Instruments, Houston, USA) über den linken Vorhof oder über eine in der *A*. *carotin* eingebundene Schleuse zur Messung der Herz-Hämodynamik;
- Einführung eines Swan-Ganz-Katheters (CCOmbo 7.5F, Edwards, Irvine, USA) über die *V*. *jugularis* in die *A*. *pulmonalis* zur Messung von Herzzeitvolumen, Sauerstoffsättigung, Pulmonalartiendrücken und zentralvenösem Druck;
- Anbringung einer Ultraschall-Flussmess-Sonde (Transsonic Systems, Ithaka, USA) an der *A*. *descendens* zur Messung des Aortenflusses;
- Anbringung einer Ultraschall-Flussmess-Sonde (Transsonic Systems, Ithaka, USA) an der linken *A*. *coronaria* zur Messung des Koronarflusses;
- Platzierung einer Braunüle in der *V*. *cephalicae* zur Infusion von Pentobarbital, der Flüssigkeitssubstitution und zur Blutentnahme (Bestimmung der Substanz-Plasmaspiegel oder sonstiger klinischer Blutwerte);
- Platzierung einer Braunüle in der *V*. *saphenae* zur Infusion von Fentanyl, Aldosteron und zur Substanzapplikation.

Die Primärsignale werden eventuell verstärkt (Gould Verstärker, Gould Instrument Systems, Valley View, USA bzw. Edwards Vigilance-Monitor, Edwards, Irvine, USA) und anschließend zur Auswertung in das Ponemah-System (DataSciences Inc., Minneapolis, USA) eingespeist. Die Signale werden kontinuierlich über den gesamten Versuchszeitraum aufgezeichnet, von dieser Software digital weiterverarbeitet und über 30 Sekunden gemittelt.

### 6. Chronisches Myokardinfarkt-Modell der wachen Ratte

Männliche Wistar-Ratten (280-300 g Körpergewicht; Harlan-Winkelmann) werden mit 5% Isofluran im Narkosekäfig narkotisiert, intubiert, an eine Beatmungspumpe (ugo basile 7025 rodent, 50 Hübe/min, 7 ml) angeschlossen und mit 2% Isofluran/N₂O/O₂ beatmet. Die Körpertemperatur wird durch eine Wärmematte auf 37-38°C gehalten. Als Schmerzmittel werden 0.05 mg/kg Temgesic subkutan gegeben. Der Brustkorb wird zwischen der dritten und vierten Rippe seitlich geöffnet und das Herz freigelegt. Die Herzkranzarterie des linken Ventrikels (LAD) wird mit einem Okklusionsfaden (Prolene 1 metric 5-0 Ethicon1H) kurz unterhalb ihres Ursprungs (unterhalb des linken Atriums) unterstochen und permanent abgebunden. Das Auftreten eines Myokardinfarkts wird durch eine EKG-Messung (Cardioline, Remco, Italien) überwacht. Der Thorax wird wieder geschlossen und die Muskelschichten mit Ethibond excel 1 metric 5/0 6951H und die Oberhaut mit Ethibond excel 3/0 6558H zugenäht. Die OP-Naht wird mit Sprühpflaster benetzt (z.B. Nebacetin^{®}N Sprühverband, Wirkstoff: Neomycinsulfat) und anschließend die Narkose beendet.

Eine Woche nach der Okklusion der LAD wird die Größe des Myokardinfarkts durch Echokardiographie (Sequoia 512, Acuson) abgeschätzt. Die Tiere werden randomisiert und in einzelne Behandlungsgruppen sowie eine Kontrollgruppe ohne Substanzbehandlung aufgeteilt. Als weitere Kontrolle wird eine "sham"-Gruppe, bei der nur der Operationsvorgang, nicht aber die LAD-Okklusion durchgeführt wurde, mitgeführt.

Die Substanzbehandlung erfolgt über 8 Wochen per Gavage oder durch Zusatz der Testverbindung zum Futter oder Trinkwasser. Die Tiere werden wöchentlich gewogen und der Wasser- bzw. Futterverbrauch alle 14 Tage bestimmt.

Nach 8 Wochen Behandlung werden die Tiere erneut narkotisiert (2% Isofluran/N₂O/Luft) und ein Druckkatheter (Millar SPR-320 2F) über die *A. carotis* in den linken Ventrikel eingeführt. Dort werden Herzfrequenz, linksventrikulärer Druck (LVP), linksventrikulärer enddiastolischer Druck (LVEDP), Kontraktilität (dp/dt) und Relaxationsgeschwindigkeit (τ) mit Hilfe des Powerlab-Systems (AD Instruments, ADI-PWLB-4SP) und der Chart 5-Software (SN 425-0586) erfasst und ausgewertet. Anschließend wird eine Blutprobe zur Bestimmung der Substanz-Plasmaspiegel und Plasmabiomarker entnommen und die Tiere abgetötet. Herz (Herzkammern, linker Ventrikel mit Septum, rechter Ventrikel), Leber, Lunge und Niere werden entnommen und gewogen.

### 7. Modell der stroke-prone spontan-hypertensiven Ratte

Die Verabreichung von Kochsalz bei der sogenannten stroke-prone spontan-hypertensiven Ratte (SP-SHR) führt in diesem Modell paradoxerweise nach wenigen Tagen zu einer Aufhebung der physiologischen salzinduzierten Repression der Renin- und Angiotensin-Freisetzung. Somit ist der Hypertonus in den SP-SHR-Tieren charakterisiert durch einen relativ hohen Reninspiegel. Als Folge des sich entwickelnden Hypertonus kommt es zu ausgeprägten Endorganschäden des Herzens und der Niere, die u.a. durch Proteinurie und Glomerulosklerose charakterisiert sind, sowie zu allgemeinen vaskulären Veränderungen. So können sich vor allem durch cerebrovaskuläre Läsionen im weiteren Verlauf Schlaganfälle bilden ("stroke-prone"), die zu einer hohen Mortalität der unbehandelten Tiere führen. In diesem Rattenmodell lassen sich somit Testsubstanzen auf Blutdruck-senkende und Endorgan-schützende Wirkung hin untersuchen.

Etwa 10 Wochen alte, männliche SP-SH-Ratten (Körpergewicht zwischen 190 und 220 g) werden einen Tag vor Versuchsbeginn randomisiert und Gruppen mit gleicher Tierzahl, in der Regel n = 12-14, zugeordnet. Während des gesamten Versuchs steht den Tieren kochsalzhaltiges Trinkwasser (2% NaCl) und Futter *ad libitum* zur Verfügung. Die Substanzen werden einmal täglich 6-8 Wochen lang per Gavage oder per Futter verabreicht (Ssniff, Germany). Als Plazebogruppe dienen Tiere, die genauso behandelt werden, aber entweder nur das Lösungsmittel oder das Futter ohne Testsubstanz erhalten. Im Rahmen einer Mortalitätsstudie wird der Versuch beendet, wenn ca. 50% der Plazebo-behandelten Tiere verstorben sind.

Die Wirkung der Testsubstanzen wird durch Verlaufsmessungen des systolischen Blutdrucks (über eine Schwanzmanschette) sowie der Proteinausscheidung im Urin verfolgt. *Post mortem* erfolgen Gewichtsbestimmungen von Herz, Niere und Lunge, sowie histopathologische Analysen von Herz, Niere und Gehirn mit semi-quantitativem Scoring der histologischen Veränderungen. Verschiedene Biomarker (z.B. ANP, Atrial Natriuretic Peptide, und BNP, Brain Natriuretic Peptide, KIM-1, kidney induced molecule 1, Osteopontin-1) werden mittels RT/TaqMan-PCR nach RNA-Isolation aus Herz- und Nierengewebe bzw. Serum oder Plasma bestimmt.

Die statistische Auswertung erfolgt mit Student's t-Test nach vorheriger Überprüfung der Varianzen auf Homogenität.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm. Wölbungsradius 12 mm

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 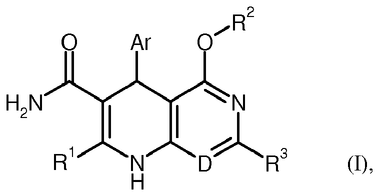 in welcher
D für C-R⁴ steht, worin
R⁴ Wasserstoff oder Methyl bedeutet,
Ar für eine Gruppe der Formel 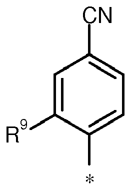 steht, worin
* die Verknüpfungsstelle
und
R⁹ Ethyl, Methoxy oder Trifluormethoxy bedeutet,
R¹ für Methyl oder Trifluormethyl steht,
R² für Methyl, Ethyl, n-Propyl oder Isopropyl steht
und
R³ für Wasserstoff oder Methyl steht,
sowie ihre Salze, Solvate und Solvate der Salze.

2. Verbindung nach Anspruch 1 mit einer der folgenden Strukturen: 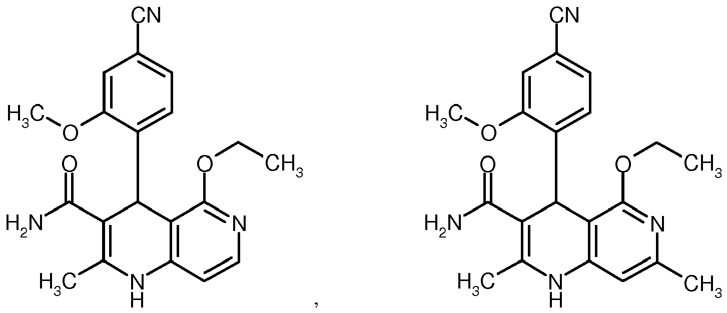 und 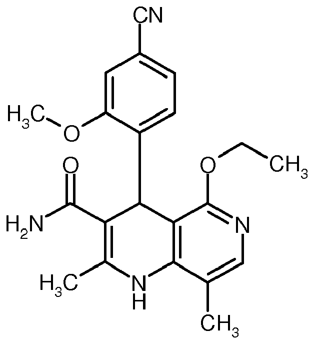 sowie ihre Salze, Solvate und Solvate der Salze.

3. Verbindung nach Anspruch 1 oder 2 mit einer der folgenden Strukturen: 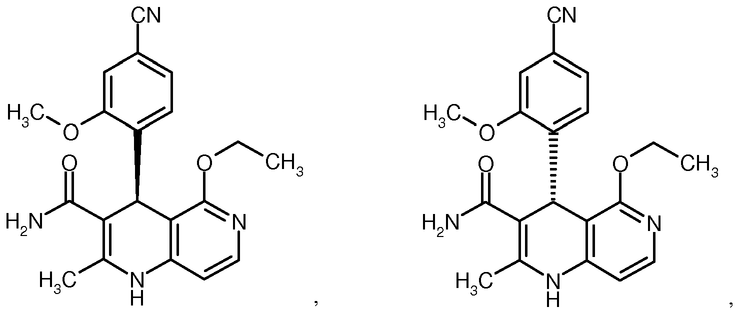 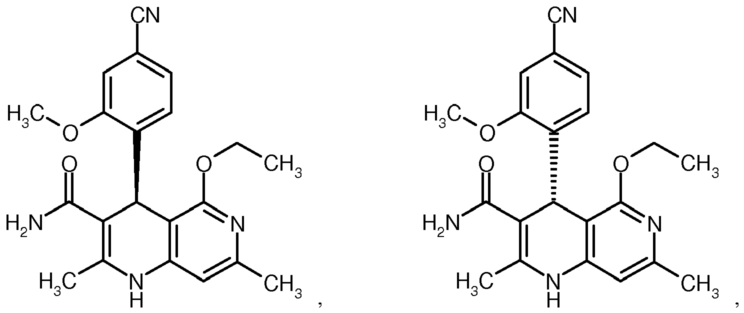 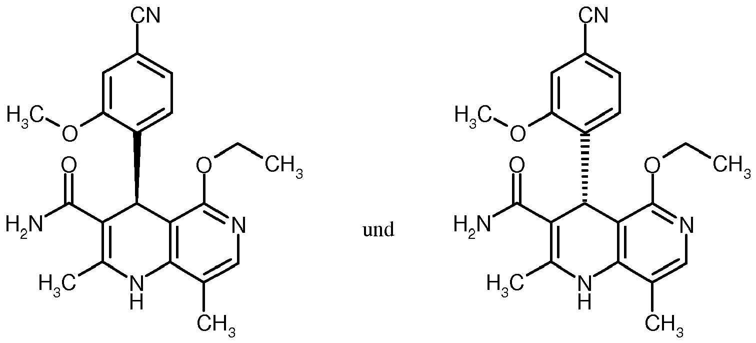 sowie ihre Salze, Solvate und Solvate der Salze.

4. Verbindung nach Anspruch 1, 2 oder 3 mit der folgenden Struktur: 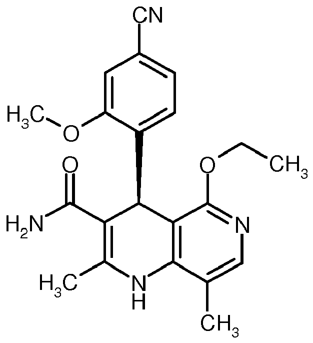 sowie ihre Salze, Solvate und Solvate der Salze.

5. Verfahren zur Herstellung von Verbindungen der Formel (I), wie in den Ansprüchen 1 bis 4 definiert, **dadurch gekennzeichnet, dass** man eine Verbindung der Formel (II) 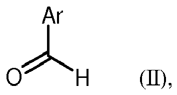 in welcher Ar die in den Ansprüchen 1 bis 4 angegebene Bedeutung hat,
in einem inerten Lösungsmittel gegebenenfalls in Gegenwart einer Säure, einer Säure/ Base-Kombination und/oder eines wasserentziehenden Mittels mit einer Verbindung der Formel (III) 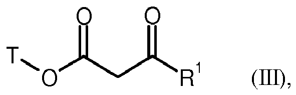 in welcher R¹ die in den Ansprüchen 1 bis 4 angegebene Bedeutung hat und
T für Allyl oder 2-Cyanoethyl steht,
zu einer Verbindung der Formel (IV) 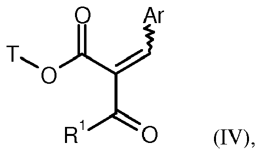 in welcher Ar, T und R¹ jeweils die oben angegebenen Bedeutungen haben,
umsetzt, diese dann in einem inerten Lösungsmittel mit einer Verbindung der Formel (V) 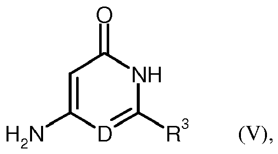 in welcher D und R³ die in den Ansprüchen 1 bis 4 angegebenen Bedeutungen haben, zu einer Verbindung der Formel (VI) 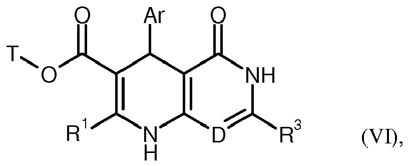 in welcher Ar, D, T, R¹ und R³ jeweils die oben angegebenen Bedeutungen haben,
kondensiert, anschließend die Verbindungen der Formel (VI) in einem inerten Lösungsmittel gegebenenfalls in Gegenwart einer Base mit einer Verbindung der Formel (VII) oder einem Trialkyloxonium-Salz der Formel (VIII) 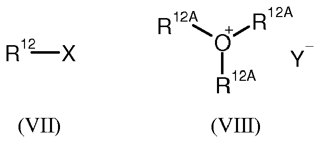 in welchen
R¹² für (C₁-C₆)-Alkyl, das mit (C₃-C₇)-Cycloalkyl oder bis zu dreifach mit Fluor substituiert sein kann, steht,
R^{12A} für Methyl oder Ethyl steht,
X für eine Abgangsgruppe, wie beispielsweise Halogen, Mesylat, Tosylat oder Triflat, steht
und
Y⁻ für ein nicht-nukleophiles Anion, wie beispielsweise Tetrafluoroborat, steht,
oder in Gegenwart einer Säure mit einem Trialkylorthoformiat der Formel (IX) 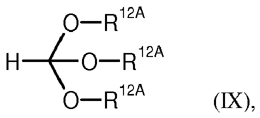 in welcher R^{12A} die oben angegebene Bedeutung hat,
zu Verbindungen der Formel (X-A) 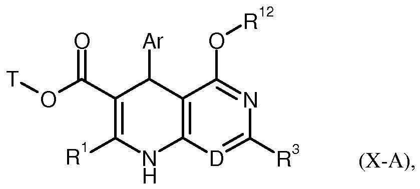 in welcher Ar, D, T, R¹, R³ und R¹² jeweils die oben angegebenen Bedeutungen haben, alkyliert
oder die Verbindungen der Formel (VI) in einem inerten Lösungsmittel in Gegenwart einer Base mit einer Verbindung der Formel (XI) 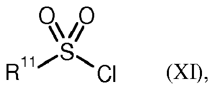 in welcher R¹¹ die in Anspruch 1 oder 2 angegebene Bedeutung hat,
zu Verbindungen der Formel (X-B) 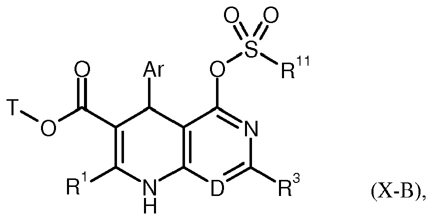 in welcher Ar, D, T, R¹, R³ und R¹¹ jeweils die oben angegebenen Bedeutungen haben, umsetzt,
danach in den Verbindungen der Formel (X-A) bzw. (X-B) die Ester-Gruppe T zu den Carbonsäuren der Formel (XII) 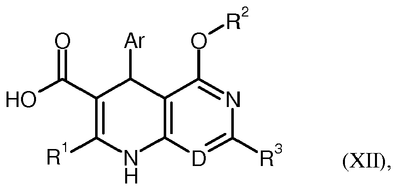 in welcher Ar, D, R¹, R² und R³ jeweils die in den Ansprüchen 1 bis 4 angegebenen Bedeutungen haben,
abspaltet, anschließend mit 1,1'-Carbonyldiimidazol in die Imidazolide der Formel (XIII) 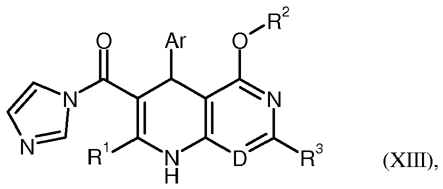 in welcher Ar, D, R¹, R² und R³ jeweils die oben angegebenen Bedeutungen haben,
überführt und diese dann in einem inerten Lösungsmittel gegebenenfalls in Gegenwart einer Hilfsbase mit Ammoniak zu den Amiden der Formel (I) umsetzt
und gegebenenfalls die Verbindungen der Formel (I) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/ oder Solvate der Salze überführt.

6. Verbindung der Formel (I), wie in einem der Ansprüche 1 bis 4 definiert, zur Verwendung in der Behandlung und/oder Prophylaxe von Krankheiten.

7. Verwendung einer Verbindung der Formel (I), wie in einem der Ansprüche 1 bis 4 definiert, zur Herstellung eines Arzneimittels zur Behandlung und/oder Prophylaxe von Aldosteronismus, Bluthochdruck, chronischer Herzinsuffizienz, den Folgen eines Myokardinfarkts, Leberzirrhose, Niereninsuffizienz und Hirnschlag.

8. Arzneimittel enthaltend eine Verbindung der Formel (I), wie in einem der Ansprüche 1 bis 4 definiert, in Kombination mit einem inerten, nicht-toxischen, pharmazeutisch geeigneten Hilfsstoff.

9. Arzneimittel enthaltend eine Verbindung der Formel (I), wie in einem der Ansprüche 1 bis 4 definiert, in Kombination mit einem oder mehreren weiteren Wirkstoffen ausgewählt aus der Gruppe bestehend aus ACE-Inhibitoren, Renin-Inhibitoren, Angiotensin II-Rezeptor-Antagonisten, Beta-Blocker, Acetylsalicylsäure, Diuretika, Calcium-Antagonisten, Statine, Digitalis (Digoxin)-Derivate, Calcium-Sensitizer, Nitrate sowie Antithrombotika.

10. Arzneimittel nach Anspruch 8 oder 9 zur Behandlung und/oder Prophylaxe von Aldosteronismus, Bluthochdruck, chronischer Herzinsuffizienz, den Folgen eines Myokardinfarkts, Leberzirrhose, Niereninsuffizienz und Hirnschlag.

## Claims

1. Compound of the formula (I) 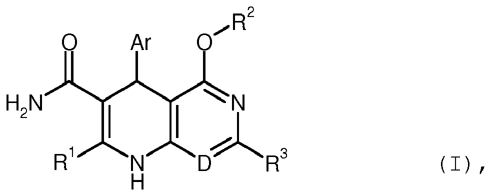 in which
D is C-R⁴ in which
R⁴ is hydrogen or methyl,
Ar is a group of the formula 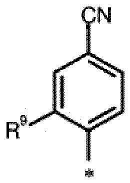 in which
* is the linkage point
and
R⁹ is ethyl, methoxy or trifluoromethoxy,
R¹ is methyl or trifluoromethyl,
R² is methyl, ethyl, n-propyl or isopropyl
and
R³ is hydrogen or methyl,
and the salts, solvates and solvates of the salts thereof.

2. Compound according to Claim 1 having one of the following structures: 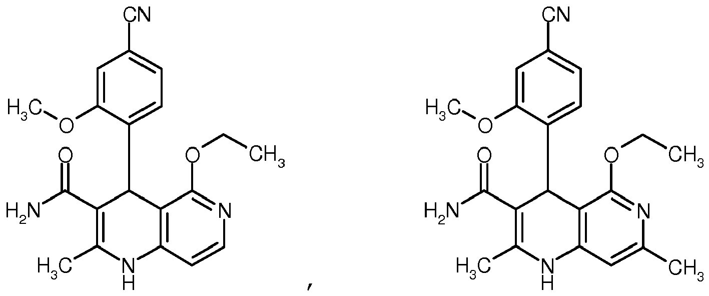 and 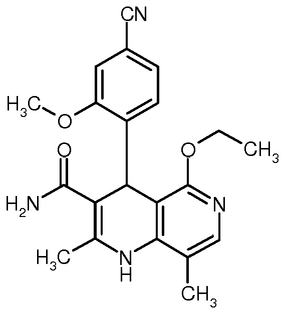 and the salts, solvates and solvates of the salts thereof.

3. Compound according to Claim 1 or 2 having one of the following structures: 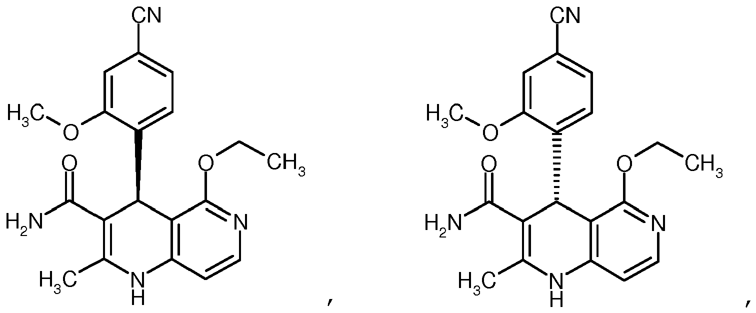 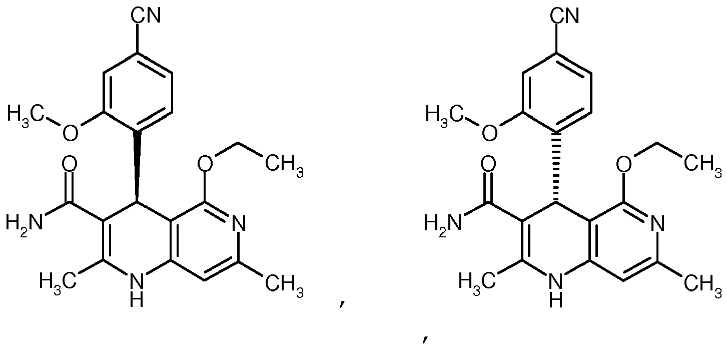 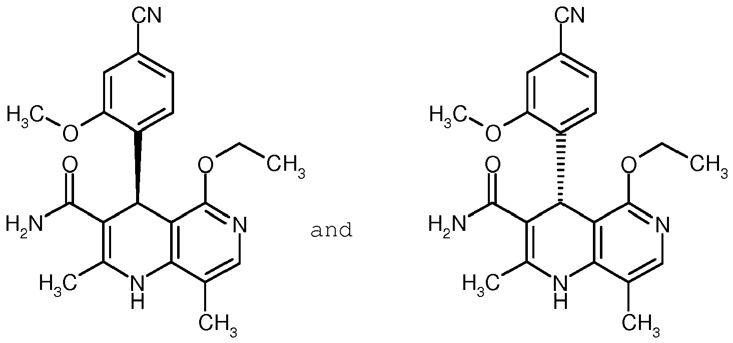 and the salts, solvates and solvates of the salts thereof.

4. Compound according to Claim 1, 2 or 3 having the following structure: 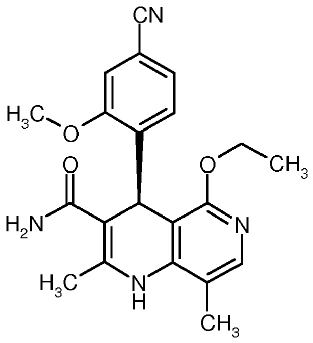 and the salts, solvates and solvates of the salts thereof.

5. Process for preparing compounds of the formula (I) as defined in Claims 1 to 4, **characterized in that** a compound of the formula (II) 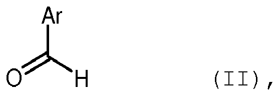 in which Ar has the meaning indicated in Claims 1 to 4,
is reacted in an inert solvent, where appropriate in the presence of an acid, an acid/base combination and/or a dehydrating agent, with a compound of the formula (III) 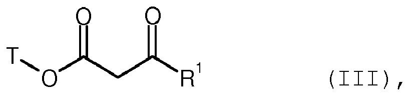 in which R¹ has the meaning indicated in Claims 1 to 4, and
T is allyl or 2-cyanoethyl,
to give a compound of the formula (IV) 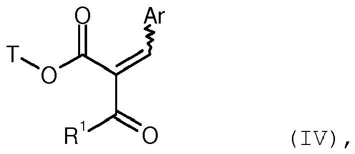 in which Ar, T and R¹ each have the meanings indicated above,
the latter is then condensed in an inert solvent with a compound of the formula (V) 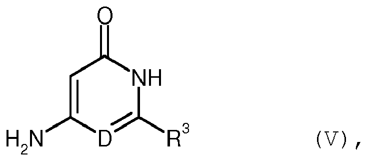 in which D and R³ have the meanings indicated in Claims 1 to 4,
to give a compound of the formula (VI) 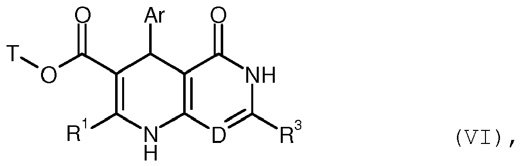 in which Ar, D, T, R¹ and R³ each have the meanings indicated above,
then the compounds of the formula (VI) are alkylated in an inert solvent, where appropriate in the presence of a base, with a compound of the formula (VII) or a trialkyloxonium salt of the formula (VIII) 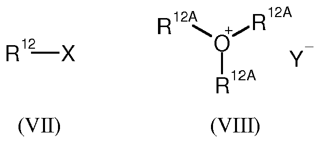 in which
R¹² is (C₁-C₆)-alkyl which may be substituted by (C₃-C₇)-cycloalkyl or up to three times by fluorine,
R^{12A} is methyl or ethyl,
X is a leaving group such as, for example, halogen, mesylate, tosylate or triflate,
and
Y- is a non-nucleophilic anion such as, for example, tetrafluoroborate,
or in the presence of an acid with a trialkyl orthoformate of the formula (IX) 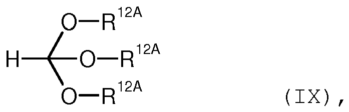 in which R^{12A} has the meaning indicated above,
to give compounds of the formula (X-A) 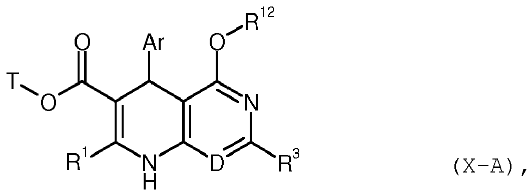 in which Ar, D, T, R¹, R³ and R¹² each have the meanings indicated above,
or the compounds of the formula (VI) are reacted in an inert solvent in the presence of a base with a compound of the formula (XI) 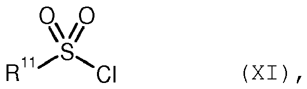 in which R¹¹ has the meaning indicated in Claim 1 or 2,
to give compounds of the formula (X-B) 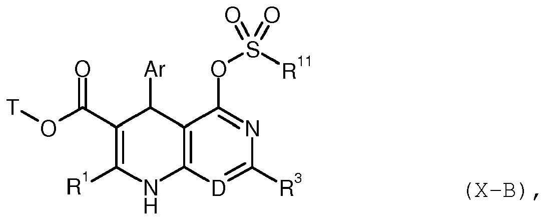 in which Ar, D, T, R¹, R³ and R¹¹ each have the meanings indicated above,
then the ester group T in the compounds of the formula (X-A) or (X-B) is eliminated by to give the carboxylic acids of the formula (XII) 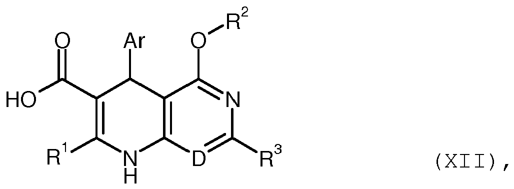 in which Ar, D, R¹, R² and R³ each have the meanings indicated in Claims 1 to 4,
then converted with 1,1'-carbonyldiimidazole into the imidazolides of the formula (XIII) 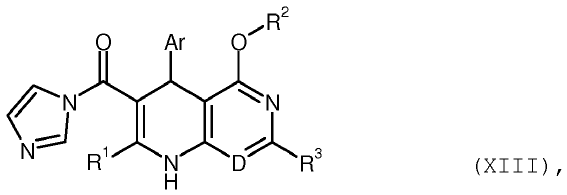 in which Ar, D, R¹, R² and R³ each have the meanings indicated above,
and the latter are then reacted in an inert solvent, where appropriate in the presence of an auxiliary base, with ammonia to give the amides of the formula (I),
and where appropriate the compounds of the formula (I) are separated by methods known to the skilled worker into their enantiomers and/or diastereomers, and/or converted with the appropriate (i) solvents and/or (ii) bases or acids into the solvates, salts and/or solvates of the salts thereof.

6. Compound of the formula (I) as defined in any of Claims 1 to 4 for use in the treatment and/or prophylaxis of diseases.

7. Use of a compound of the formula (I) as defined in any of Claims 1 to 4 for the manufacture of a medicament for the treatment and/or prophylaxis of aldosteronism, high blood pressure, chronic heart failure, the sequelae of a myocardial infarction, cirrhosis of the liver, renal failure and stroke.

8. Medicament comprising a compound of the formula (I) as defined in any of Claims 1 to 4 in combination with an inert, non-toxic, pharmaceutically suitable excipient.

9. Medicament comprising a compound of the formula (I) as defined in any of Claims 1 to 4 in combination with one or more further active ingredients selected from the group consisting of ACE inhibitors, renin inhibitors, angiotensin II receptor antagonists, beta-blockers, acetylsalicylic acid, diuretics, calcium antagonists, statins, digitalis (digoxin) derivatives, calcium sensitizers, nitrates and antithrombotics.

10. Medicament according to Claims 8 or 9 for the treatment and/or prophylaxis of aldosteronism, high blood pressure, chronic heart failure, the sequelae of a myocardial infarction, cirrhosis of the liver, renal failure and stroke.

## Revendications

1. Composé de formule (I) 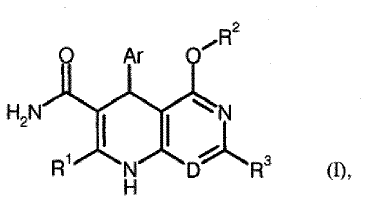 dans laquelle
D représente C-R⁴,
R⁴ signifiant hydrogène ou méthyle,
Ar représente un groupe de formule 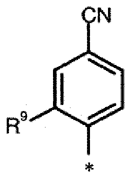
* signifiant l'emplacement de liaison
et
R⁹ signifiant éthyle, méthoxy ou trifluorométhoxy,
R¹ représente méthyle ou trifluorométhyle,
R² représente méthyle, éthyle, n-propyle ou isopropyle
et
R³ représente hydrogène ou méthyle,
ainsi que ses sels, solvates et solvates des sels.

2. Composé selon la revendication 1, présentant une des structures suivantes : 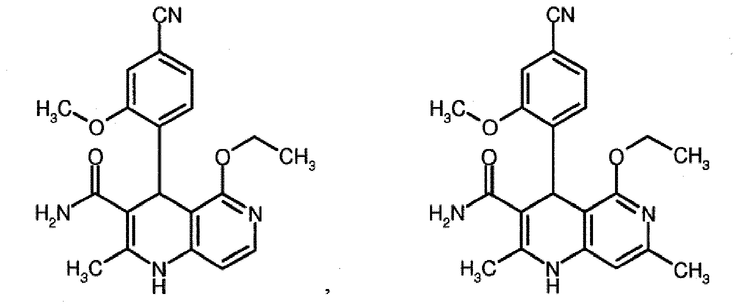 et 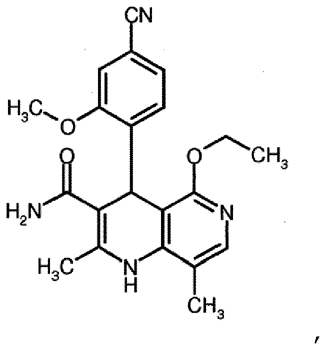 ainsi que ses sels, solvates et solvates des sels.

3. Composé selon la revendication 1 ou 2, présentant une des structures suivantes : 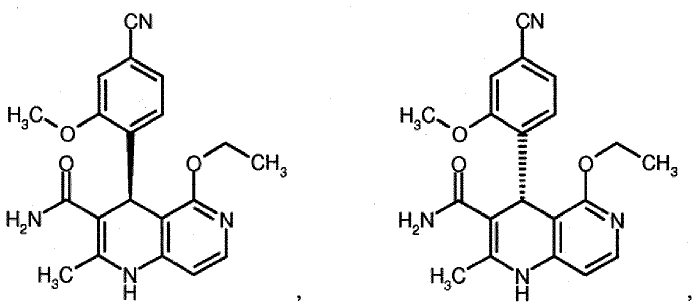 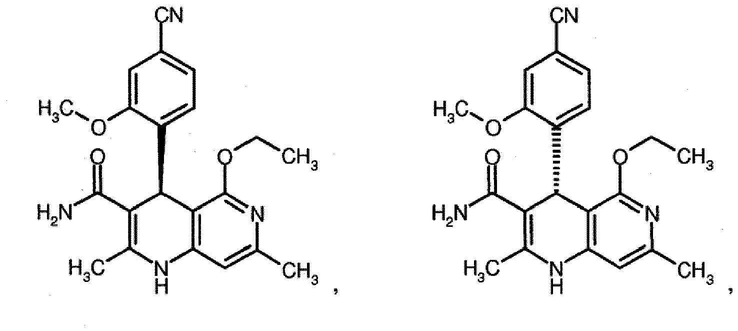 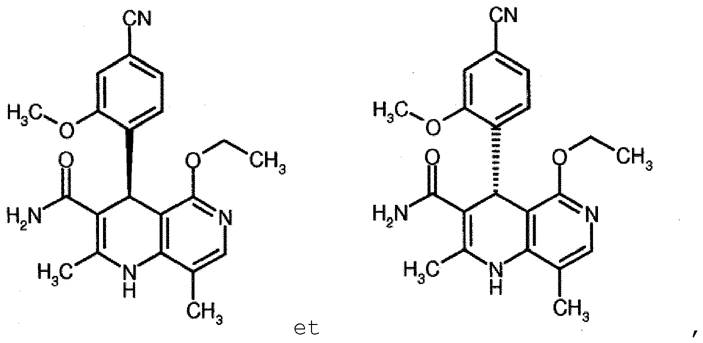 ainsi que ses sels, solvates et solvates des sels.

4. Composé selon la revendication 1, 2 ou 3, présentant la structure suivante : 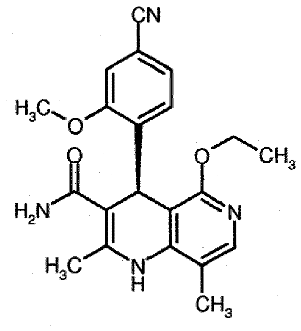 ainsi que ses sels, solvates et solvates des sels.

5. Procédé de fabrication de composés de formule (I), tels que définis dans les revendications 1 à 4, **caractérisé en ce qu'**un composé de formule (II) 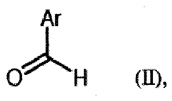 dans laquelle Ar a la signification indiquée dans les revendications 1 à 4,
est mis en réaction dans un solvant inerte, éventuellement en présence d'un acide, d'une combinaison acide/base et/ou d'un agent déshydratant, avec un composé de formule (III) 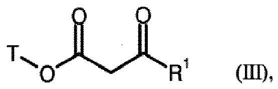 dans laquelle R¹ a la signification indiquée dans les revendications 1 à 4, et
T représente allyle ou 2-cyanoéthyle,
pour former un composé de formule (IV) 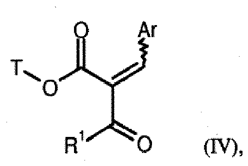 dans laquelle Ar, T et R¹ ont chacun les significations indiquées précédemment,
puis celui-ci est condensé dans un solvant inerte avec un composé de formule (V) 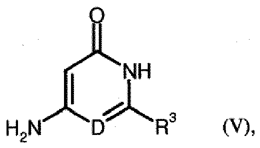 dans laquelle D et R³ ont les significations indiquées dans les revendications 1 à 4,
pour former un composé de formule (VI) 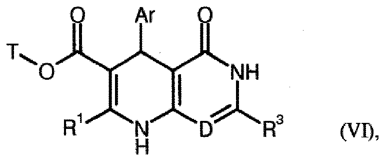 dans laquelle Ar, D, T, R¹ et R³ ont chacun les significations indiquées précédemment,
puis les composés de formule (VI) sont alkylés dans un solvant inerte, éventuellement en présence d'une base, avec un composé de formule (VII) ou un sel de trialkyloxomium de formule (VIII) 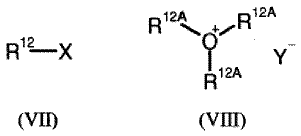 dans lesquelles
R¹² représente alkyle en (C₁-C₆), qui peut être substitué avec cycloalkyle en (C₃-C₇) ou jusqu'à trois fois avec fluor,
R^{12A} représente méthyle ou éthyle,
X représente un groupe partant, tel que par exemple halogène, mésylate, tosylate ou triflate,
et
Y⁻ représente un anion non nucléophile, tel que par exemple tétrafluoroborate,
ou en présence d'un acide avec un orthoformiate de trialkyle de formule (IX) 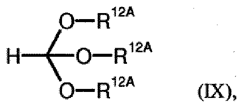 dans laquelle R^{12A} a la signification indiquée précédemment,
pour former des composés de formule (X-A) 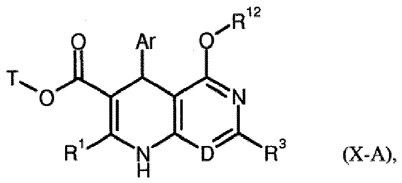 dans laquelle Ar, D, T, R¹, R³ et R¹² ont chacun les signification indiquées précédemment,
ou les composés de formule (VI) sont mis en réaction dans un solvant inerte en présence d'une base avec un composé de formule (XI) 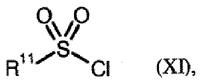 dans laquelle R¹¹ a la signification indiquée dans la revendication 1 ou 2,
pour former des composés de formule (X-B) 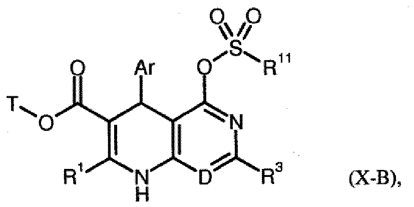 dans laquelle Ar, D, T, R¹, R³ et R¹¹ ont chacun les significations indiquées précédemment,
puis, dans les composés de formule (X-A) ou (X-B), le groupe ester T est clivé pour former les acides carboxyliques de formule (XII) 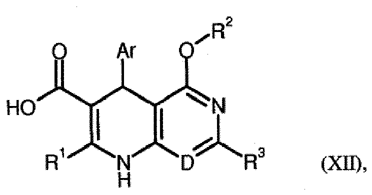 dans laquelle Ar, D, R¹, R² et R³ ont chacun les significations indiquées dans les revendications 1 à 4, puis transformés avec du 1,1'-carbonyldiimidazole en les imidazolides de formule (XIII) 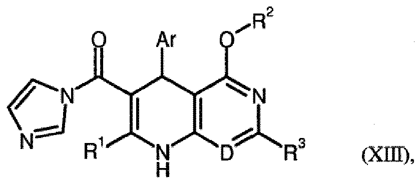 dans laquelle Ar, D, R¹, R² et R³ ont chacun les significations indiquées précédemment,
et ceux-ci sont ensuite mis en réaction dans un solvant inerte, éventuellement en présence d'une base auxiliaire, avec de l'ammoniac pour former les amides de formule (I)
et les composés de formule (I) sont éventuellement séparés par des méthodes connues de l'homme du métier en leurs énantiomères et/ou diastéréomères et/ou transformés avec les (i) solvants et/ou (ii) bases ou acides appropriés en leurs solvates, sels et/ou solvates des sels.

6. Composé de formule (I), tel que défini dans l'une quelconque des revendications 1 à 4, destiné à une utilisation pour le traitement et/ou la prophylaxie de maladies.

7. Utilisation d'un composé de formule (I), tel que défini dans l'une quelconque des revendications 1 à 4, pour la fabrication d'un médicament pour le traitement et/ou la prophylaxie d'un aldostéronisme, d'une tension artérielle élevée, d'une insuffisance cardiaque chronique, des conséquences d'un infarctus du myocarde, d'une cirrhose du foie, d'une insuffisance rénale et d'une attaque cérébrale.

8. Médicament contenant un composé de formule (I), tel que défini dans l'une quelconque des revendications 1 à 4, en combinaison avec un adjuvant inerte, non toxique, pharmaceutiquement approprié.

9. Médicament contenant un composé de formule (I), tel que défini dans l'une quelconque des revendications 1 à 4, en combinaison avec un ou plusieurs agents actifs supplémentaires choisis dans le groupe constitué par les inhibiteurs d'ACE, les inhibiteurs de rénine, les antagonistes des récepteurs d'angiotensine II, les bêtabloquants, l'acide acétylsalicylique, les diurétiques, les antagonistes de calcium, les statines, les dérivés de digitaline (digoxine), les sensibilisateurs calciques, les nitrates et les antithrombotiques.

10. Médicament selon la revendication 8 ou 9, pour le traitement et/ou la prophylaxie d'un aldostéronisme, d'une tension artérielle élevée, d'une insuffisance cardiaque chronique, des conséquences d'un infarctus du myocarde, d'une cirrhose du foie, d'une insuffisance rénale et d'une attaque cérébrale.
